(19) Europäisches Patentamt
European Patent Office
Office européen des brevets

(11) Publication number: 0 425 134 A1

(12) **EUROPEAN PATENT APPLICATION**

(21) Application number: 90311144.1

(22) Date of filing: 11.10.90

(51) Int. Cl.5: C07D 513/04, A61K 31/425, A61K 31/54, //(C07D513/04, 277:00,209:00),(C07D513/04, 279:00,209:00)

The title of the invention has been amended (Guidelines for Examination in the EPO, A-III, 7.3).

(30) Priority: 11.10.89 JP 264756/89
20.12.89 JP 330166/89
20.06.90 JP 162054/90

(43) Date of publication of application: 02.05.91 Bulletin 91/18

(84) Designated Contracting States: AT BE CH DE DK ES FR GB GR IT LI LU NL SE

(71) Applicant: YAMANOUCHI PHARMACEUTICAL CO. LTD.
No. 3-11 Nihonbashi-Honcho, 2-chome
Chuo-ku
Tokyo(JP)

(72) Inventor: Mase, Toshiyasu
81 Maruyama-cho, Nijusseikigaoka
Matsudo-shi, Chiba 271(JP)
Inventor: Nagaoka, Hitoshi
2-35-2-402 Kasuga
Tsukuba-shi, Ibaraki 305(JP)
Inventor: Kondoh, Yutaka
1-19-12-404 Ninomiya
Tsukuba-shi, Ibaraki 305(JP)
Inventor: Tomioka, Kenichi
1214-76 Sakata
Okegawa-shi, Saitama 363(JP)
Inventor: Yamada, Toshimitsu
6-36-12, Mizukino Moriyamachi
Kitasouma-gun, Ibaraki 302-01(JP)

(74) Representative: Geering, Keith Edwin et al
REDDIE & GROSE 16 Theobalds Road
London WC1X 8PL(GB)

(54) **Condensed pyrrolo derivatives, process for their preparation and pharmaceutical compositions containing them.**

(57) A heterocyclic compound of the formula (I):

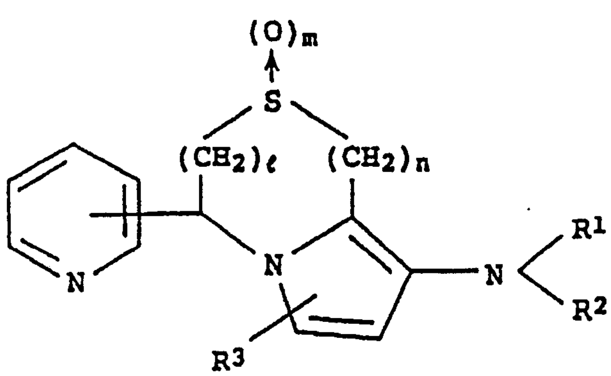

(I)

and a pharmacologically acceptable salt thereof which have platelet activating factor (PAF) antagonizing activity

EP 0 425 134 A1

are disclosed.

# NOVEL HETEROCYCLIC COMPOUNDS AND PRODUCTION THEREOF

The present invention relates to novel heterocyclic compounds and salts thereof which are useful as medicinal compounds, in particular as platelet activating factor (PAF) antagonizing agents.

PAF is a chemical substance released from human and other animal cells and is an acetylglyceryl ether of phosphorylcholine as represented by the following formula:

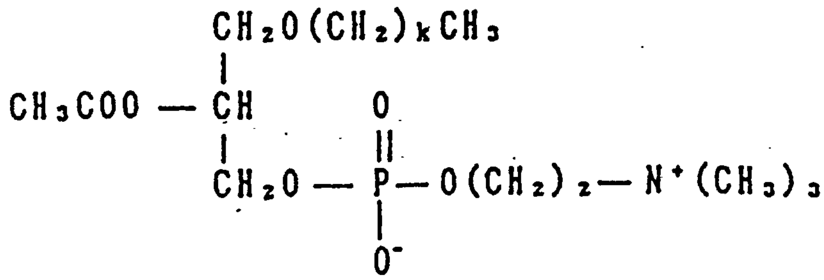

wherein k is the integer 15 or 17.

PAF is physiologically active and causes contraction of the airway smooth muscle, increased vascular permeation, platelet aggregation and blood pressure fall, among others. PAF is thought to be a factor inducing asthma, inflammation, thrombosis, shock, nephritis and other symptoms. Therefore, studies of substances capable of antagonizing the physiological activities of PAF are under way and several anti-PAF agents have been reported so far (e.g. U.S. Patent 4,734,280, EP-A-115979, EP-A-178261, EP-A-144804, EP-A-142801, EP-A-176927, EP-A-252823 and EP-A-253711).

We have found novel heterocyclic compounds, inclusive of salts thereof, which differ in chemical structure from the hitherto known anti-PAF agents and have potent anti-PAF activity.

The present invention provides heterocyclic compounds of the general formula (I) shown below and salts thereof.

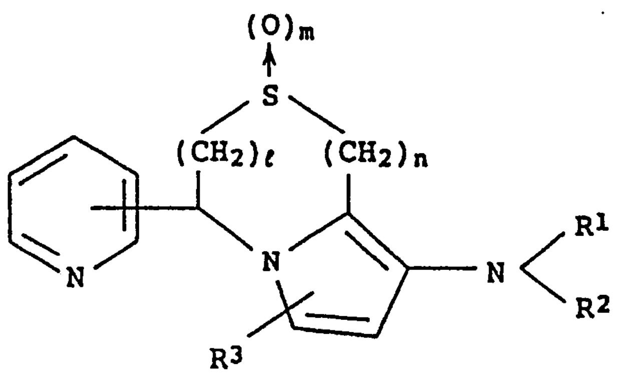

(I)

In the above formula (I), the substituents used in the above formula are respectively defined as follows:

$\ell$ represents the integer 0 or 1;

m represents the integer 0, 1 or 2;

n represents the integer 1 or 2, provided that the sum of $\ell$ and n is 1 or 2;

$R^1$ represents a hydrogen atom, a lower alkyl group, a group of the formula

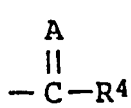

(A is an oxygen atom or a sulfur atom) or a group of the formula $-SO_2R^4$;

$R^2$ represents a hydrogen atom or a lower alkyl group;

$R^3$ represents a hydrogen atom or a lower alkyl group;

$R^4$ represents a substituted or unsubstituted aryl group, a lower alkyl group, an aralkyl group, a lower alkoxy group, an aryloxy group, an aralkyloxy group, a group of the formula

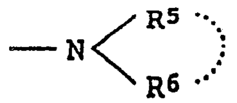 ,

a cycloalkyl group or a group of the formula

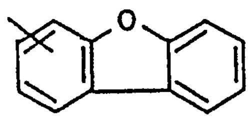 ;

the substituent or each of the substituents in the substituted aryl group being a group of the formula -O-$R^7$, a nitro group, a halogen atom, a cyano group, a trihalogeno-lower alkyl group, a group of the formula

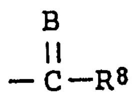

(B is O or N-OH), an amino group of the formula

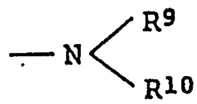 ,

a group of the formula

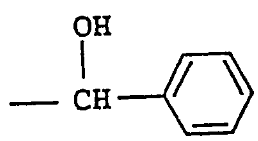 ,

a lower alkyl group or an aralkyl group;
$R^5$ and $R^6$, which may be the same or different, each represents a hydrogen atom, a lower alkyl group, an aralkyl group or an aryl group, or $R^5$ and $R^6$, together with the adjacent nitrogen atom, combinedly form a piperidine ring, a morpholine ring, or a piperazine ring which may optionally be substituted in position 4 by a lower alkyl or aralkyl group;
$R^7$ represents a hydrogen atom, a lower alkyl group, an aralkyl group, an aryl group which may have on or more substituents selected from a lower alkyl group and a halogen atom, or an acyl group;
$R^8$ represents an aralkyl group, an aryl group which may have one or more substituents selected from a lower alkoxy group, a lower alkyl group, and a halogen atom, a group of the formula

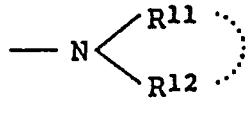 ,

a hydroxyl group or a lower alkoxy group;
$R^9$ and $R^{10}$ may be the same or different, and one of them is a hydrogen atom or a lower alkyl group and the other is a hydrogen atom, a lower alkyl group, a carboxyl group, a lower alkoxycarbonyl group, an arylcarbonyl group, an aralkylcarbonyl group, a group of the formula

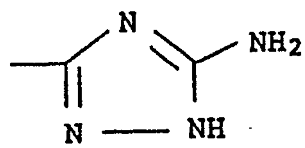

or a group of the formula

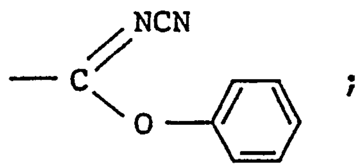

and

$R^{11}$ and $R^{12}$, which may be the same or different, each represents a hydrogen atom, a lower alkyl group, an aralkyl group, an aryl group or a cycloalkyl group or $R^{11}$ and $R^{12}$, together with the adjacent nitrogen atom, combinedly form a pyrrolidine ring, a piperidine ring, a morpholine ring, or a piperazine ring which may optionally be substituted in position 4 thereof by a lower alkyl or aralkyl group; and pharmacologically acceptable salts thereof.

## DETAILED DESCRIPTION OF THE INVENTION

The compounds of the present invention are described in more detail hereinbelow.

In the definitions of the substituents used herein in the formulas given above and the formulas appearing later herein, the term "lower" means, unless otherwise specified, that the relevant group includes a straight or branched carbon chain containing 1 to 6 carbon atoms.

Accordingly, the "lower alkyl group" includes, among others, methyl, ethyl, propyl, isopropyl, butyl, isobutyl, sec-butyl, tert-butyl, pentyl (amyl), isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl.

The "lower alkoxy group" includes methoxy, ethoxy, propoxy, isopropoxy, butoxy, isobutoxy, sec-butoxy, tert-butoxy, pentyloxy (amyloxy), isopentyloxy, tert-pentyloxy, neopentyloxy, 2-methylbutoxy, 1,2-dimethylpropoxy, 1-ethylpropoxy, hexyloxy and so on.

In the definitions of the substituents appearing herein, the term "aryl", unless otherwise specified, includes, within the meaning thereof, both carbocyclic and heterocyclic aryl groups, which may optionally be substituted by one to three substituents each independently selected from the group consisting of a halogen atom, a lower alkoxy group, an aralkyloxy group, an aryloxy group, a lower alkanoyl group, an aralkylcarbonyl group, an arylcarbonyl group, a cyano group, a nitro group, an amino group and a mono- or di-lower alkylamino group.

Typical examples of the carbocyclic aryl group are phenyl, naphthyl, anthryl and phenanthryl.

As the heterocyclic aryl group, there may be mentioned pyrrolyl, furyl, thienyl, pyridyl, pyrimidyl, quinolyl, isoquinolyl, imidazolyl and quinazolinyl, for instance.

The "aralkyl group" is a group derived from the above-mentioned "lower alkyl group" by substitution of any hydrogen atom by the above-mentioned "aryl group". Thus, when the aryl group is typified by phenyl or naphtyl, for instance, the aralkyl group includes, among others, benzyl, phenetyl, 1-phenylethyl, 3-phenylpropyl, 2-phenylpropyl, 1-phenylpropyl, 1-methyl-2-phenylethyl, 4-phenylbutyl, 3-phenylbutyl, 2-phenylbutyl, 1-phenylbutyl, 2-methyl-3-phenylpropyl, 2-methyl-2-phenylpropyl, 2-methyl-1-phenylpropyl, 1-methyl-3-phenylpropyl, 1-methyl-2-phenylpropyl, 1-methyl-1-phenylpropyl, 1-ethyl-2-phenylethyl, 1,1-dimethyl-2-phenylethyl, 5-phenylpentyl, 4-phenylpentyl, 3-phenylpentyl, 2-phenylpentyl, 1-phenylpentyl, 3-methyl-4-phenylbutyl, 3-methyl-3-phenylbutyl, 3-methyl-2-phenylbutyl, 3-methyl-1-phenylbutyl, 6-phenylhexyl, 5-phenylhexyl, 4-phenylhexyl, 3-phenylhexyl, 2-phenylhexyl, 1-phenylhexyl, 4-methyl-5-phenylpentyl, 4-methyl-4-phenylpentyl, 4-methyl-3-phenylpentyl, 4-methyl-2-phenylpentyl, 4-methyl-1-phenylpentyl, 1-naphthylmethyl, 2-naphthylmethyl, 2-(1-naphthyl)ethyl, 2-(2-naphthyl)ethyl, 1-(1-naphtyl)ethyl, 1-(2-(naphthyl)ethyl, 3-(1-naphthyl)propyl, 3-(2-naphthyl)propyl, 2-(1-naphthyl)propyl, 2-(2-naphthyl)propyl, 1-(1-naphthyl)propyl, 1-(2-naphthyl)propyl, 1-methyl-2-(1-naphthyl)ethyl, 1-ethyl-2-(2-naphthyl)ethyl, 4-(1-naphthyl)butyl, 4-(2-naphthyl)butyl, 3-(1-naphthyl)butyl, 3- (2-naphthyl)butyl, 2-(1-naphthyl)butyl, 2-(2-naphthyl)-butyl, 1-(1-naphthyl)butyl, 1-(2-naphthyl)butyl, 2-methyl-3-(1-naphthyl)propyl, 2-methyl-3-(2-naphthyl)propyl, 2-methyl-2-(1-naphthyl)propyl, 2-methyl-2-(2-naphthyl)propyl, 2-methyl-1-(1-naphthyl)propyl, 2-methyl-1-(2-naphthyl)propyl, 5-(1-naphthyl)pentyl, 5-(2-naphthyl)pentyl, 4-(1-naphthyl)pentyl, 4-(2-naphthyl)pentyl, 3-methyl-4-(1-naphthyl)butyl, 3-methyl-4-(2-naphthyl)butyl, 6-(1-naphthyl)hexyl, 6-(2-naphthyl)hexyl, 5-(1-naphthyl)hexyl, 5-(2-naphthyl)hexyl, 4-methyl-5-(1-naphthyl)pentyl, 4-methyl-5-(2-naphthyl)pentyl, diphenylmethyl (benzydryl) and trityl (triphenylmethyl).

The term "aryloxy group" as used herein includes, within the meaning thereof, both carbocyclic and heterocyclic aryloxy groups. Typical carbocyclic aryloxy groups are phenoxy, naphthyloxy, anthryloxy, phenanthryloxy and the like while typical heterocyclic aryloxy groups are pyrrolyloxy, furyloxy, thienyloxy, pyridyloxy, pyrimidyloxy, quinolyloxy, isoquinolyloxy, quinazolinyloxy and the like.

The "aralkyloxy group" means a group resulting from substitution of one optional hydrogen atom of the above-mentioned "lower alkoxy group" by the above-mentioned "aryl group" and, more specifically, includes the following examples in which the "aryl group" is typified by a phenyl group alone: benzyloxy, phenethyloxy, 1-phenylethoxy, 3-phenylpropoxy, 2-phenylpropoxy, 1-phenylpropoxy, 1-methyl-2-phenylethoxy, 4-phenylbutoxy, 3-phenylbutoxy, 2-phenylbutoxy, 1-phenylbutoxy, 2-methyl-3-phenylpropoxy, 2-methyl-2-phenylpropoxy, 2-methyl-1-phenylpropoxy, 1-methyl-3-phenylpropoxy, 1-methyl-2-phenylpropoxy, 1-methyl-1-phenylpropoxy, 1-ethyl-2-phenylethoxy, 1,1-dimethyl-2-phenylethoxy, 5-phenylpentyloxy and 6-phenylhexyloxy.

The "cycloalkyl group" includes 5- to 8-membered ones, which may optionally be condensed with a benzene ring. Typical examples are cyclopentyl, cyclohexyl, cycloheptyl and a group of the formula:

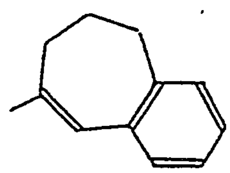

.

The "halogen atom", which is to serve as an optional substituent on the above-mentioned "aryl group", includes a fluorine, chlorine, bromine or iodine atom. The optional substituent -O-$R^7$ where $R^7$ is a lower alkyl, aralkyl or aryl group, namely the "lower alkoxy group", "aralkyloxy group" or "aryloxy group", may include those corresponding examples respectively mentioned hereinabove. The "acyloxy group" -O-$R^7$ - (where $R^7$ is an acyl group) includes, among others, formyloxy, acetyloxy, propionyloxy, butyryloxy, isobutyryloxy, valeryloxy, isovaleryloxy, pivaloyloxy and hexanoyloxy.

The group -CO-$R^8$ as an optional substituent on the above-mentioned "aryl group" where $R^8$ is an aralkyl group, namely in the "aralkylcarbonyl group", includes groups derived from the above-mentioned "lower alkanoyl group" by substitution of one optional hydrogen atom by the above-mentioned "aryl group". When the aryl group is typified by phenyl, the aralkylcarbonyl group may include, among others, phenylacetyl, 3-phenylpropionyl, 4-phenylbutyryl, 5-phenylvaleryl and 6-phenylhexanoyl. The optional substituent "arylcarbonyl group" typically includes such carbocyclic arylcarbonyl groups as benzoyl and naphthoyl and such heterocyclic arylcarbonyl groups as nicotinoyl and may optionally have at least one substituent selected from the group consisting of a lower alkoxy group, a lower alkyl group and a halogen atom.

The aminocarbonyl group

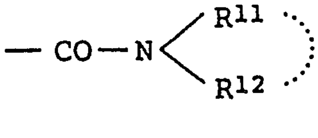

to serve as an optional substituent on the "aryl group" mentioned above includes a mono- or di-lower alkylaminocarbonyl group, an aralkylaminocarbonyl group, an arylaminocarbonyl group, a cycloalkylaminocarbonyl group, a lower alkylcycloalkylaminocarbonyl group, an aryl-lower alkylaminocarbonyl group, an aralkylarylaminocarbonyl group, a pyrrolidinylcarbonyl group, a piperidinylcarbonyl group, a morpholinylcarbonyl group, a piperazinylcarbonyl group (which may optionally be substituted, in position 4

of the piperazine ring, by a lower alkyl or aralkyl group) and the like.

The amino group

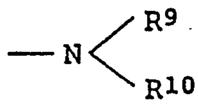

as an optional substituent on the aryl group is an unsubstituted amino group or a mono- or disubstituted amino group. The substituent or substituents on the amino group each includes a lower alkyl group, a carboxyl group, a lower alkoxycarbonyl group (e.g. t-butoxycarbonyl), an arylcarbonyl group (e.g. benzoyl), an aralkylcarbonyl group (e.g. benzylcarbonyl) and the like.

The "lower alkyl group" as an optional substituent on the aryl group is as described hereinabove and may further be substituted by a group of the formula:

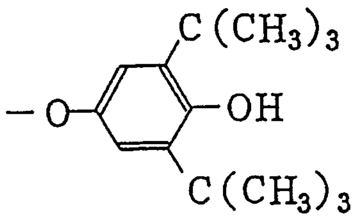

.

Typical examples of the "aralkyl group" as an optional substituent on the aryl group are benzyl, trimethoxyphenethyl, imidazolylmethyl and pyridylmethyl.

The compounds (I) according to the invention can form salts. The scope of the invention includes salts of the compounds (I). Such salts include acid addition salts with inorganic acids, such as hydrochloric acid, sulfuric acid, nitric acid, phosphoric acid, hydrobromic acid and hydriodic acid and with organic acids such as acetic acid, oxalic acid, succinic acid, citric acid, maleic acid, malic acid, fumaric acid, tartaric acid, picric acid, methanesulfonic acid and ethanesulfonic acid, salts with acidic amino acids such as glutamic acid and aspartic acid, quaternary ammonium salts resulting from quaternization with alkyl halides such as methyl chloride, methyl bromide and methyl iodide, and so forth.

For the compounds according to the invention, there can exist geometric isomers due to the presence of an asymmetric carbon atom. Such isomers all fall within the scope of the present invention either in each individual isolated form or in a mixture form.

The compounds (I) according to the invention can be produced by applying various synthetic methods taking advantage of the characteristics of the skeletal structure and various substituents. Typical examples of applicable production processes are given below.

Process 1

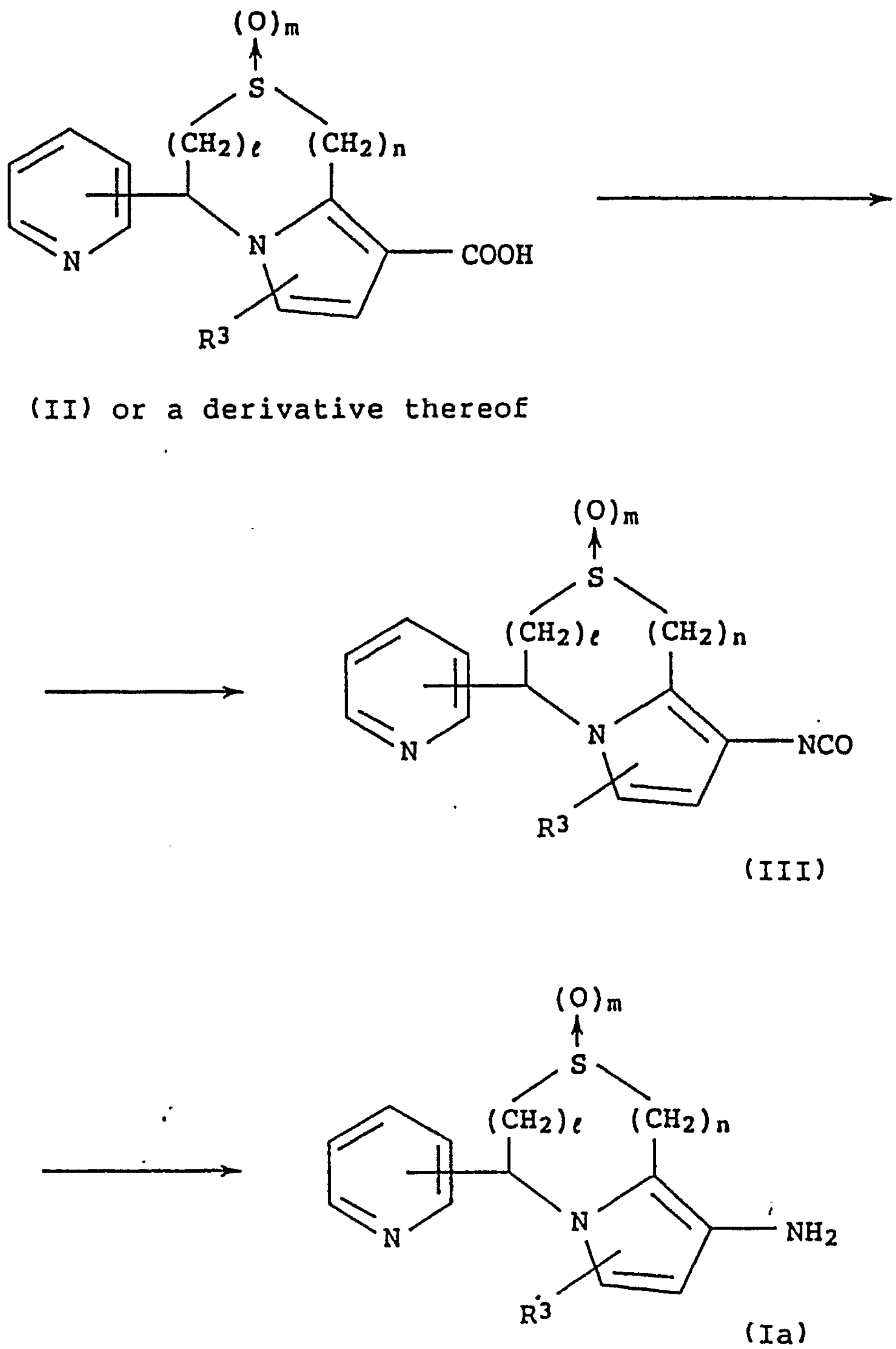

(II) or a derivative thereof

(III)

(Ia)

In the above formulas, $R^3$, ℓ, m and n are as defined in the above formula (I).

Among the compounds according to the invention, those amino-substituted pyrrolothiazole derivatives of the formula (Ia) can be produced by starting with the corresponding carboxylic acids (II) or derivatives thereof (e.g. acid halides, esters, acid anhydrides, amides) and applying thereto various rearrangement reactions for amine synthesis.

The halogen atom for forming the acid halides of compounds (II) is, for example, a chlorine atom or a bromine atom. Usable as the ester residue for forming the esters of compounds (II) are the above-mentioned straight or branched lower alkoxy group containing 1 to 6 carbon atoms and the above-mentioned aralkyloxy group, among others. As the acid anhydride-forming carboxylic acid residue, there may be mentioned the residue of the carboxylic acid of formula (II) for symmetric acid anhydride formation and the residues of alkylcarbonic acids, p-toluenesulfonic acid and the like for mixed acid anhydride formation.

The above-mentioned rearrangement reactions for amine synthesis are, for example, the Hofmann rearrangement, Schmidt rearrangement and Curtius rearrangement, and modifications of these.

For producing the amines (Ia) according to the invention, it is advantageous to employ the Curtius rearrangement which comprises converting the corresponding carboxylic acid to an acid azide by reaction of the carboxylic acid (if necessary after conversion to an acid halide or an acid anhydride) with sodium azide or by reaction of the carboxylic acid with hydrazine followed by reaction of the resulting hydrazone with nitrous acid, then subjecting the thus-obtained acid azide to thermal degradation to give an isocyanate of the formula (III) and hydrolyzing said isocyanate, preferably the modified Curtius rearrangement illustrated below where diphenylphosphoryl azide (DPPA) is used in lieu of sodium azide.

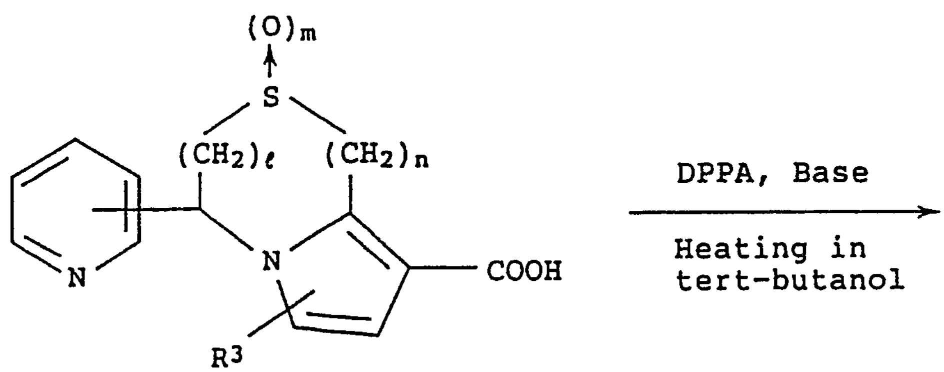


(II) or a derivative thereof

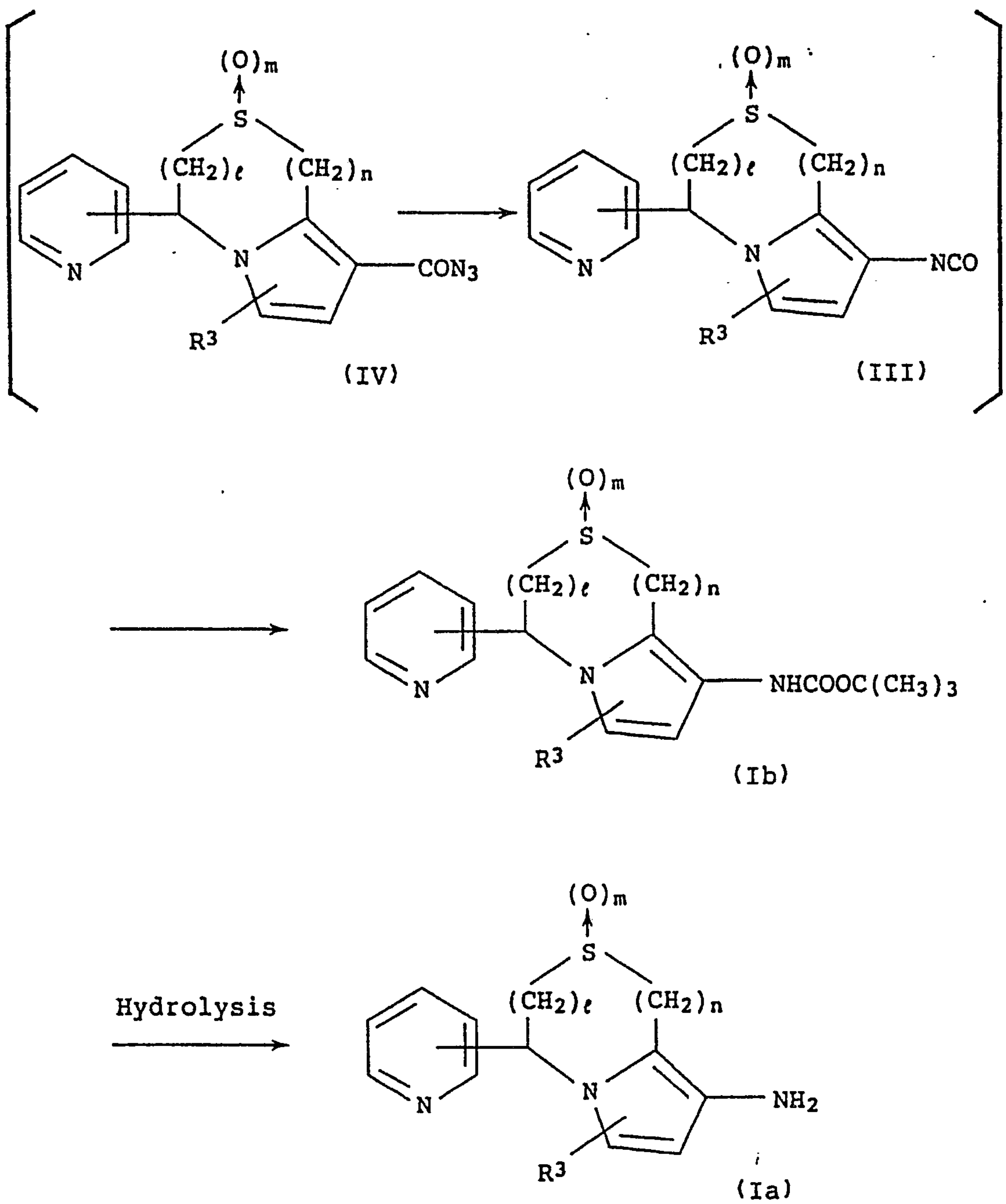


(IV) (III) (Ib) (Ia)

In the above formulas, $R^3$, $\ell$, m and n are as defined in the above formula (I).

In cases where the above modified Curtius rearrangement is employed, the compound (II) is first reacted with diphenylphosphoryl azide in tert-butanol in the presence of a base, such as trimethylamine, triethylamine, N,N-dimethylaniline, pyridine, picoline or lutidine, with heating or with heating under reflux. The resulting carbamic acid ester of formula (Ib) is then subjected to hydrolysis, preferably acid hydrolysis comprising treatment with an acid, for example trifluoroacetic acid, hydrochloric acid, acetic acid, hydrobromic acid-hydrochloric acid, hydrobromic acid-acetic acid or a mixture of such acid and anisole or dioxane.

The starting compounds (II) can be readily prepared by the method described in EP-A-252823 or later herein in the reference examples.

Process 2

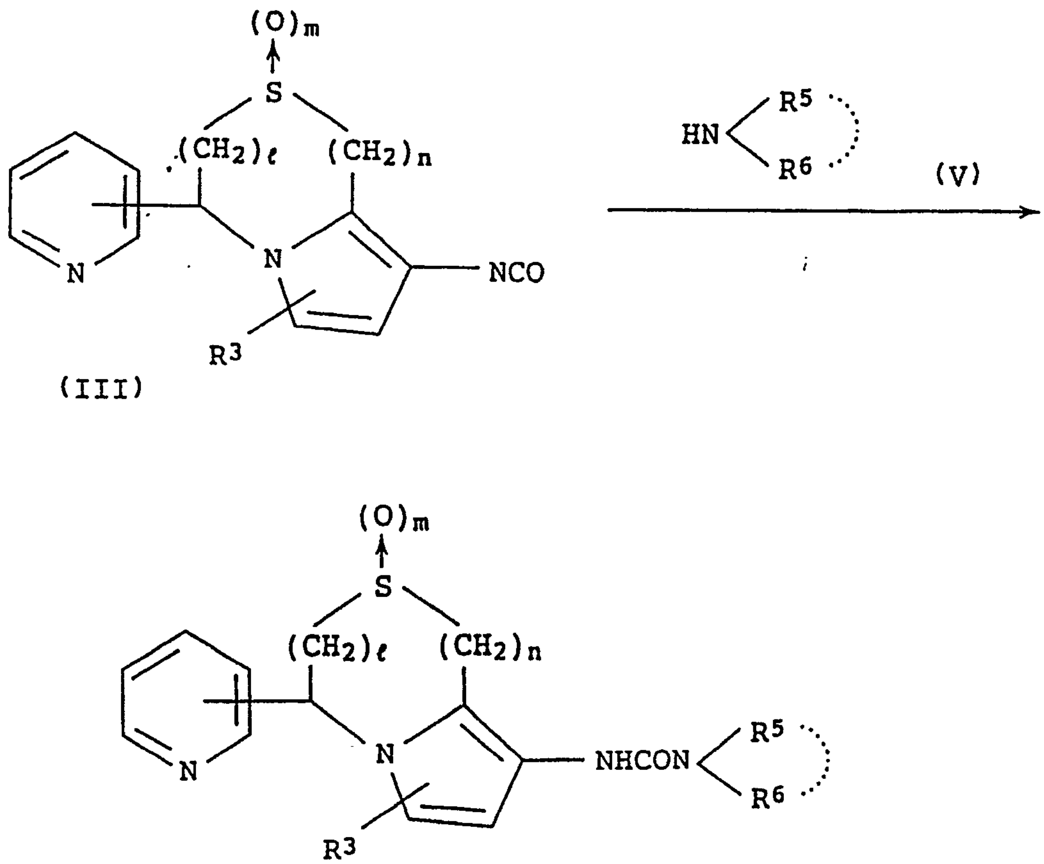

(III)

(V)

(Ic)

In the above formulas, $R^3$, $R^5$, $R^6$, $\ell$, m and n are as defined in the above formula (I).

Among the compounds according to the invention, those ureido compounds of formula (Ic) can be produced by reacting the isocyanate of formula (III) with an amine of the formula (V).

In this process, the isocyanate compound (III) is reacted with an equimolar or excessive amount of the compound (V) in an aprotic organic solvent, such as benzene, toluene, xylene, methylene chloride, dichloroethane, chloroform, carbon tetrachloride or acetonitrile, at room temperature or with warming.

Process 3

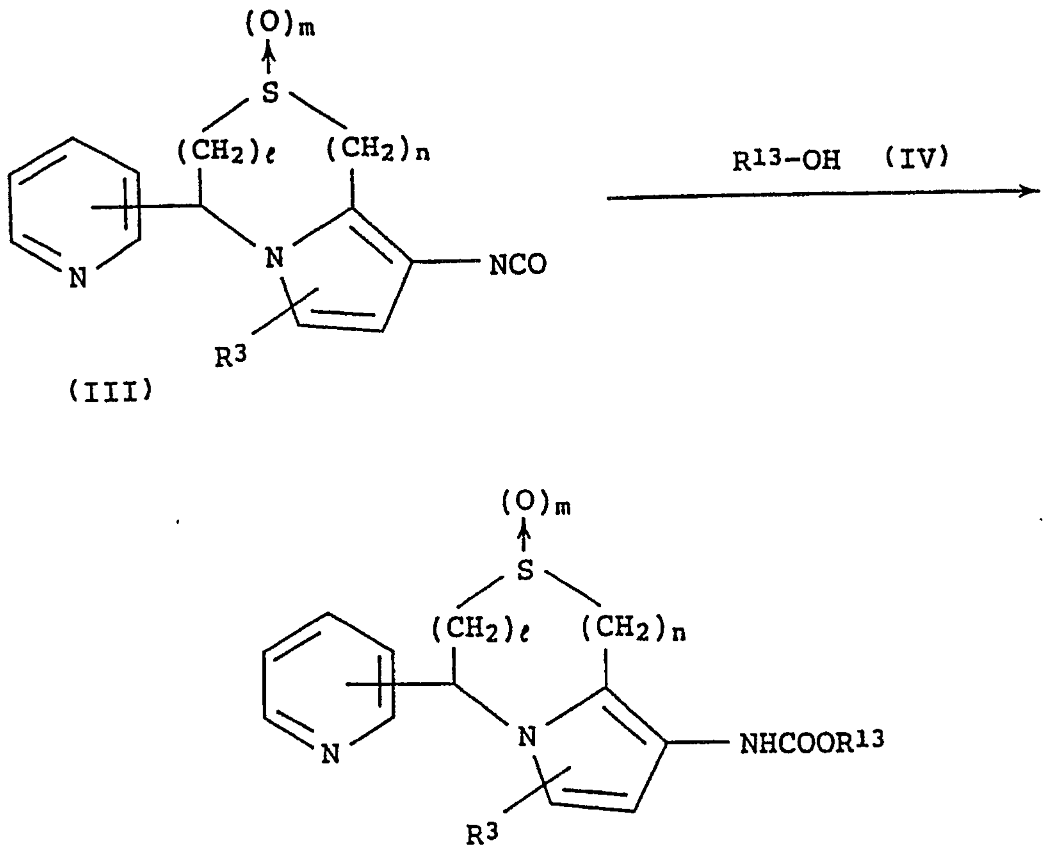


(III)

(Id)

In the above formulas, $R^3$, ℓ, m and n are as defined in the above formula (I) and $R^{13}$ is a lower alkyl, aralkyl or aryl group.

Among the compounds according to the invention, those carbamic acid ester derivatives of the formula (Id) can be produced by reacting the isocyanate (III) with a hydroxy compound of the formula (VI).

Thus, the use of an alcohol or phenol compound or the like hydroxy compound in lieu of tert-butanol in the modified Curtius rearrangement for amine production in Process 1 can give the carbamic acid esters.

In this process, the isocyanate compound (III), or the mixture of compound (II), diphenylphosphoryl azide and base in Process 1, is reacted with an equimolar or excessive amount of the compound (VI) at room temperature or with warming, if necessary in the presence of an aprotic organic solvent as in Process 2.

Process 4

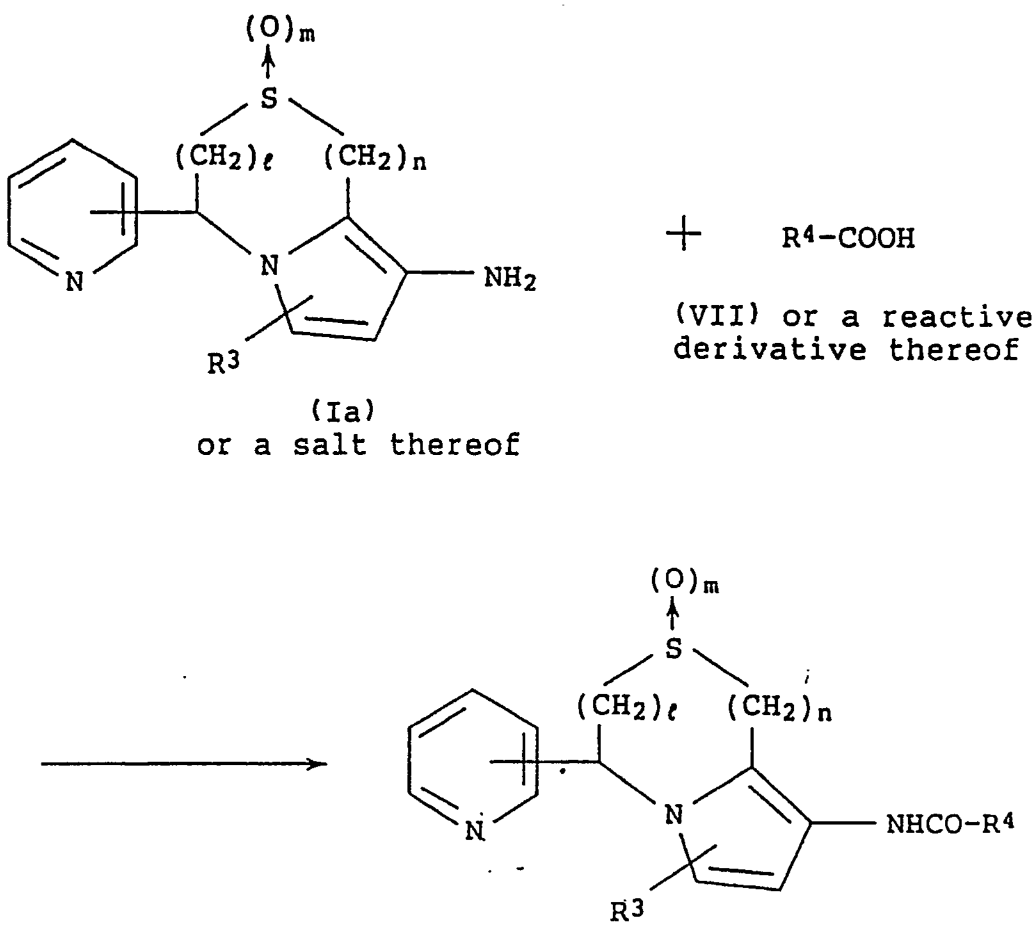


(Ia)
or a salt thereof

(VII) or a reactive derivative thereof

(Ie)

In the above formulas, $R^3$, $R^4$, $\ell$, m and n are as defined in the above formula (I).

Among the compounds according to the invention, those amide compounds of the general formula (Ie) can be produced by amidating the amine (Ia) or a salt thereof with a carboxylic acid of the general formula (VII) or a reactive derivative thereof in the conventional manner.

As the reactive derivative of compound (VII), there may be mentioned acid halides, such as acid chloride and acid bromide; acid azide; active esters, such as esters with N-hydroxybenzotriazole, N-hydroxysuccinimide and the like; asymmetric acid anhydride; mixed acid anhydrides with alkylcarbonic acids, p-toluenesulfonic acid and the like.

When the compound (VII) is used in the free carboxylic acid form, the reaction should advantageously be carried out in the presence of a condensing agent such as dicyclohexylcarbodiimide, 1,1′-carbonyldiimidazole or diphenylphosphoryl azide (DPPA).

The reaction conditions may vary to some extent depending on the starting materials, in particular the type of the reactive derivative of compound (VII). Generally, however, it is advantageous that the starting compound (Ia) be reacted with an equimolar or excessive amount of the other starting compound (VII) in an organic solvent inert to the reaction, for example pyridine, tetrahydrofuran, dioxane, ether, N,N-dimethylformamide, benzene, toluene, xylene, methylene chloride, dichloroethane, chloroform, ethyl acetate or acetonitrile.

Depending on the reactive derivative or where the starting compound (Ia) is used in the form of a salt, it is advantageous in some instances that the reaction be conducted in the presence of a base, for example an organic base, such as trimethylamine, triethylamine, pyridine, picoline, lutidine, dimethylaniline or N-methylmorpholine, or an inorganic base, such as potassium carbonate, sodium carbonate, sodium hydrogen carbonate, sodium hydroxide or potassium hydroxide. Pyridine may serve also as a solvent.

The optimum reaction temperature may vary depending on the type of the reactive derivative but can

be selected in an appropriate manner.

## Process 5

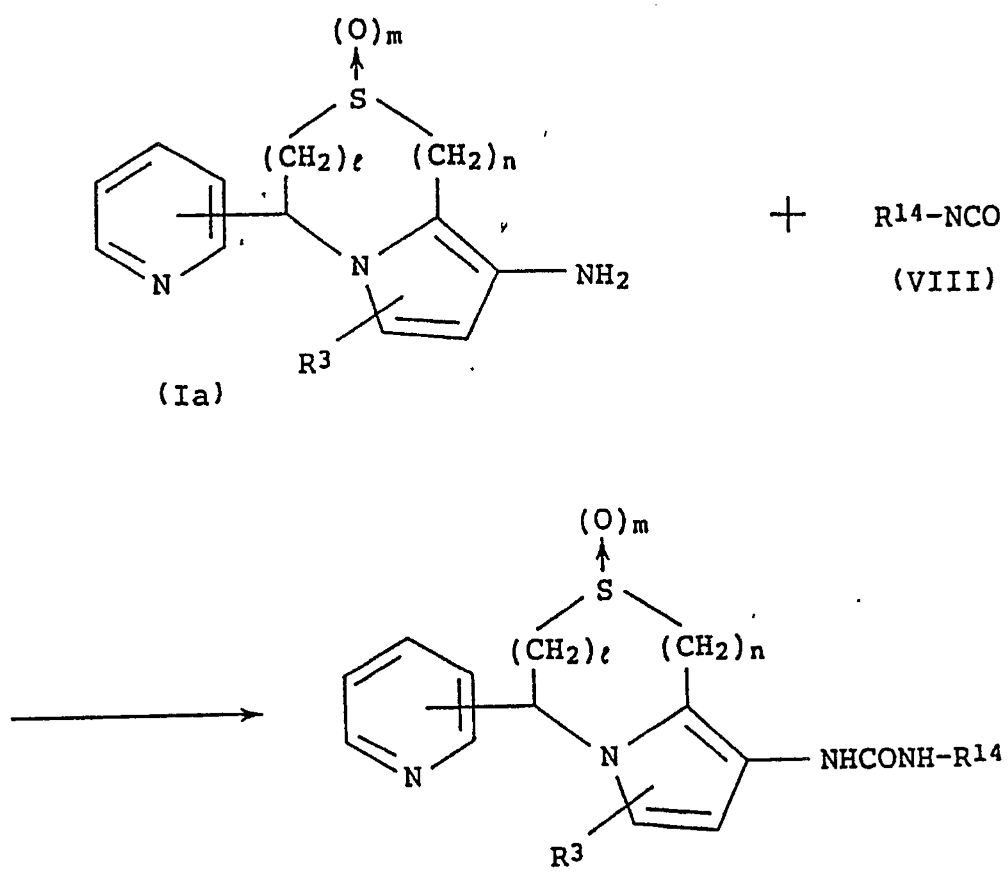

(Ia) (VIII) (If)

In the above formulas, $R^3$, ℓ, m and n are as defined in the above formula (I) and $R^{14}$ is a lower alkyl, aryl or aralkyl group.

The ureido compounds (If) according to the invention can be produced by reacting the amine obtained in Process 1 with an isocyanate of the formula (VIII).

The reaction may be carried out substantially in the same manner as Process 2.

Process 6

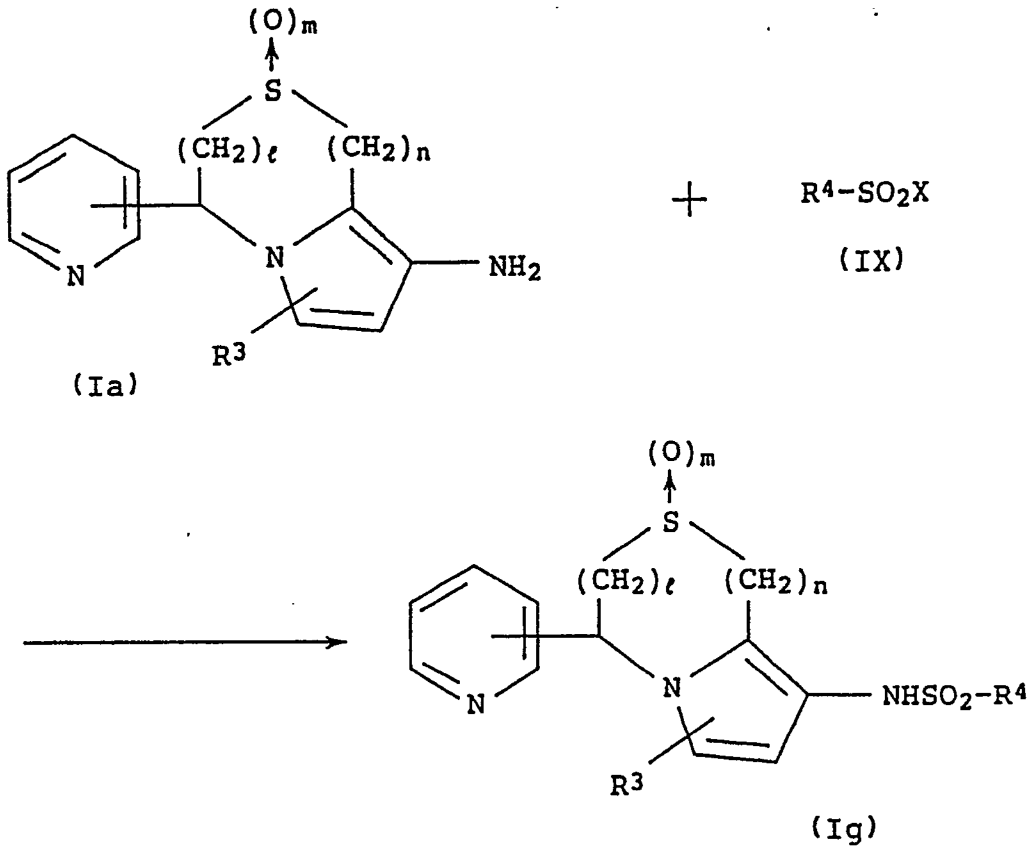

(Ia)

$R^4-SO_2X$

(IX)

(Ig)

In the above formulas, $R^3$, $R^4$, ℓ, m and n are as defined in the above formula (I) and X is a halogen atom.

The halogen atom X is, for example, a chlorine atom or a bromine atom.

The sulfonamide compound of the above general formula (Ig) can be produced by reacting the amine obtained in Process 1 with a sulfonyl halide compound of the general formula (IX).

The reaction may be conducted in the same manner as that case of Prooess 4 in which an acid halide is used. Thus, generally, the reaction is carried out advantageously in a solvent inert to the reaction, for example acetone, methyl ethyl ketone, ether, dioxane, tetrahydrofuran, dimethylformamide or methylene chloride, in the presence of a base capable of promoting the reaction by accepting the byproduct hydrogen halide, for example sodium hydroxide, potassium hydroxide, pyridine, triethylamine, trimethylamine, picoline, lutidine, dimethylaniline or N-methylmorpholine, at room temperature or with heating.

Although it is also possible to carry out the reaction using an excess of the starting compound (Ia) in lieu of the above-mentioned base for promoting the reaction, it is preferable that the starting compound (Ia) be reacted with a substantially equimolar amount of the compound (IX) in the presence of a base.

Process 7

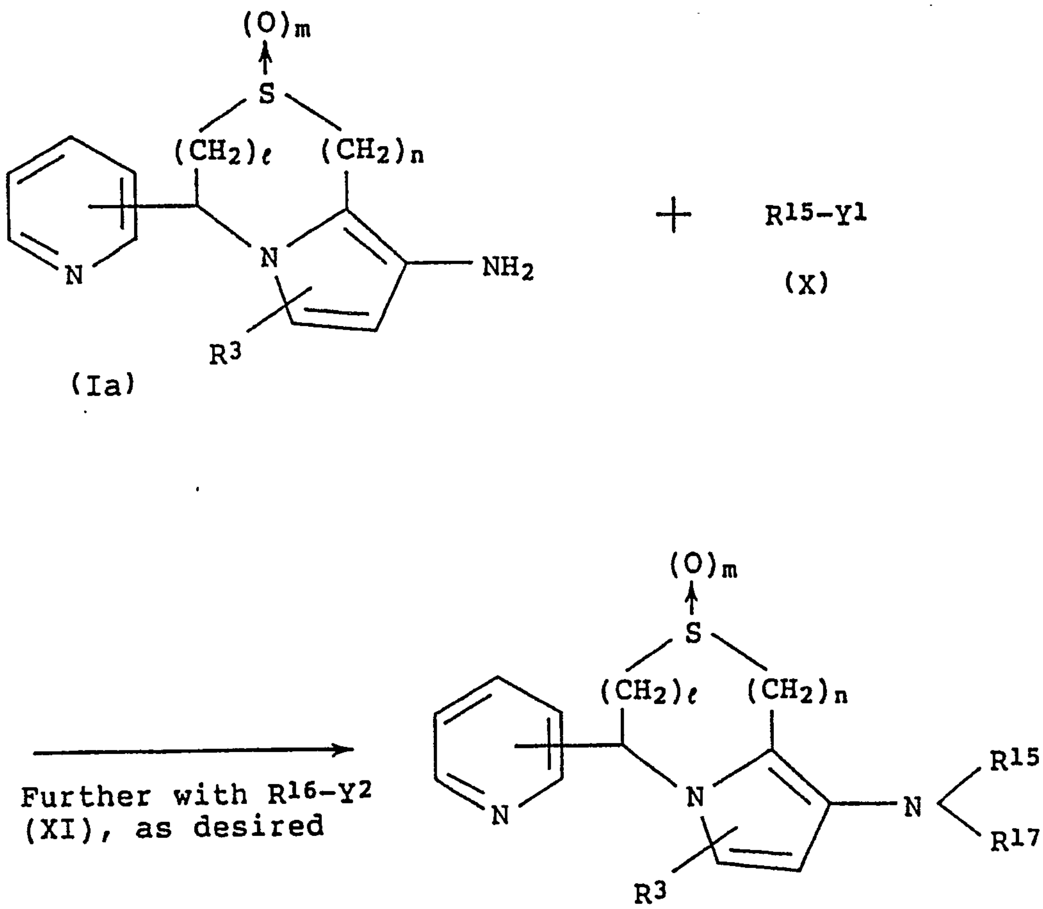


(Ia) (X) (Ih)

In the above formulas, $R^3$, ℓ, m and n are as defined in the above formula (I), $R^{15}$ is a lower alkyl group, $R^{16}$ is a lower alkyl group differing from $R^{15}$, $R^{17}$ is a hydrogen atom, the same group as $R^{15}$ or the same group as $R^{16}$, and $Y^1$ and $Y^2$ are the same or different and each is a halogen atom or an organic sulfonic acid residue.

The halogen atom just mentioned above is, for example, an iodine atom, a bromine atom or a chlorine atom. As the organic sulfonic acid residue, there may be mentioned alkanesulfonic acid residues, such as methanesulfonyloxy and ethanesulfonyloxy, and aromatic sulfonic acid residues, such as benzenesulfonyloxy and toluenesulfonyloxy, in particular p-toluenesulfonyloxy.

Among the compounds according to the invention, those mono- or di-lower alkylamino-substituted compounds of the formula (Ih) can be produced in the conventional manner by N-alkylation starting with the amine obtained in Process 1.

For producing mono-lower alkylamino or symmetric di-lower alkylamino derivatives, the compound (Ia) is reacted with a substantially equimolar amount of or about two moles per mole of (Ia) of the lower alkyl halide or sulfonate of the formula (X).

For producing asymmetric lower alkylamino derivatives, the mono-lower alkylamino compound obtained in the above manner is reacted with a lower alkyl halide or sulfonate other than the compound (X) used in the above reaction.

Both N-alkylation,reactions can be carried out under the same reaction conditions.

Thus, when $Y^1$ or $Y^2$ in the reactant (X) or (XI) is a halogen atom, the reaction is generally carried out advantageously in an organic solvent, such as benzene, toluene, xylene, dimethylformamide, dimethyl sulfoxide, acetonitrile, methylene chloride or dichloroethane, although the reaction can proceed even in the

absence of such a solvent.

The reaction is preferably carried out at room temperature or with warming or with warming under reflux.

For smooth progress of the reaction, it is advantageous in certain instances to add to the reaction system a secondary or tertiary base, such as pyridine, picoline, N,N-dimethylaniline, N-methylmorpholine, trimethylamine, triethylamine or dimethylamine, or an inorganic base, such as potassium carbonate, sodium carbonate or sodium hydrogen carbonate.

When the reactant (X) or (XI) is an organic sulfonic acid residue-substituted compound, the reaction may be advantageously carried out in an organic solvent inert to the reaction, for example ether, methanol, ethanol, toluene or tetrahydrofuran, with cooling or at room temperature. The reaction period can be selected in an appropriate manner taking other reaction conditions into consideration.

For producing mono-lower alkylamino derivatives, it is also possible to introduce a protective group into the amino group of the compound (Ia) prior to N-alkylation for avoiding tertiary amine formation and eliminate the protective group following the N-alkylation reaction. As such protective group, there may be mentioned toluenesulfonyl, acetyl, phenacylsulfonyl, trifluoromethanesulfonyl, bisbenzenesulfonyl and the like. The protective group elimination (deprotection) can be readily accomplished in the conventional manner by hydrolysis using an acid or base, for instance.

<u>Process 8</u> (Alkylation)

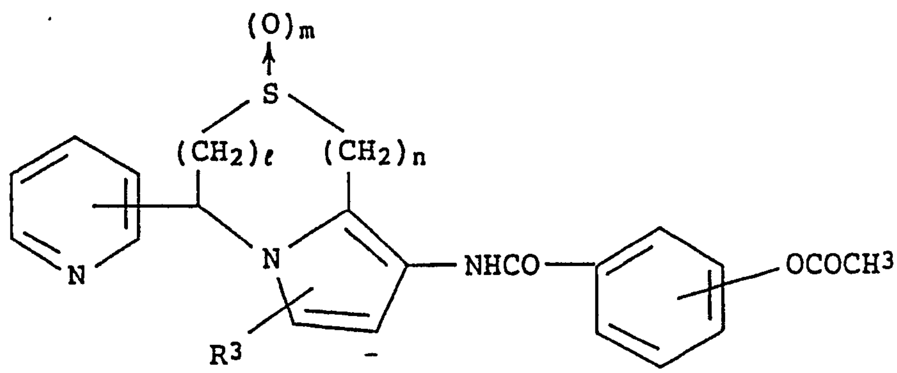

(Ii)

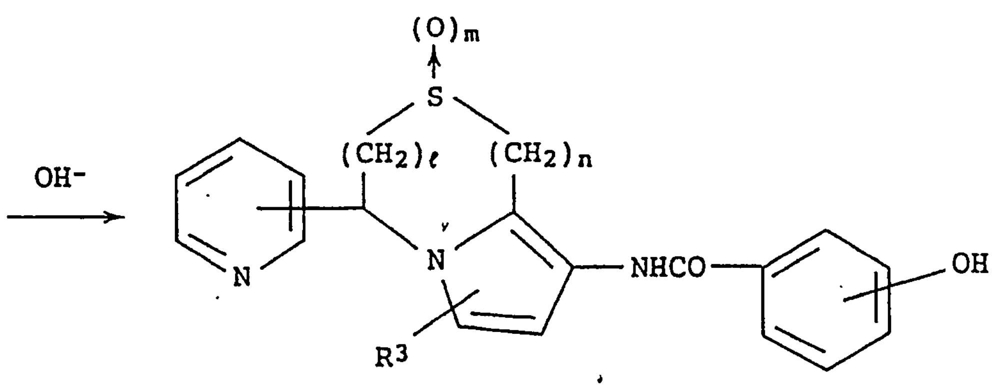


(Ij)

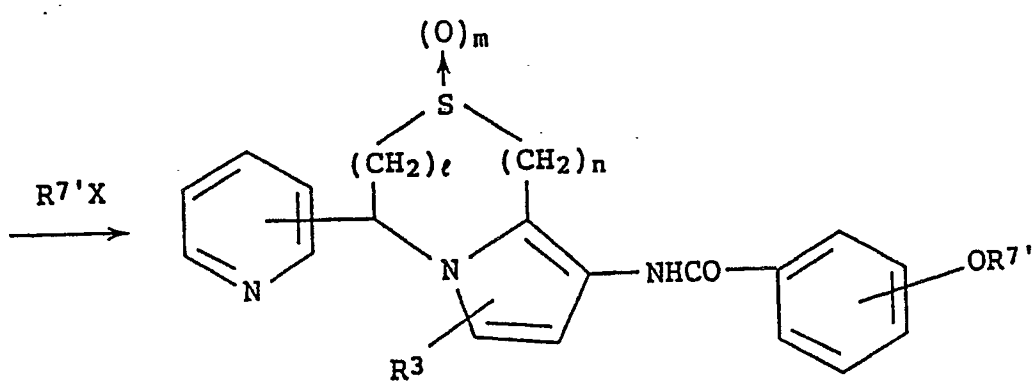


(Ik)

In the above formulas, $R^{7'}$ is a lower alkyl group, an aralkyl group, an aryl group which may optionally have one or more substituents each independently selected from the group consisting of a lower alkyl group and a halogen atom, or an acyl group, and ℓ, m, n, $R^3$ and X are as defined in the above formula (I).

Among the compounds of the formula (I), those compounds (Ik) in which the substituent on the aryl group is -O-$R^7$ can be produced by hydrolyzing a compound of the formula (Ii) shown above with an alkali such as sodium hydrogen carbonate or caustic soda and further reacting the resulting compound (Ij) with a halide of the formula $R^{7'}X$.

Process 9 (Coupling)

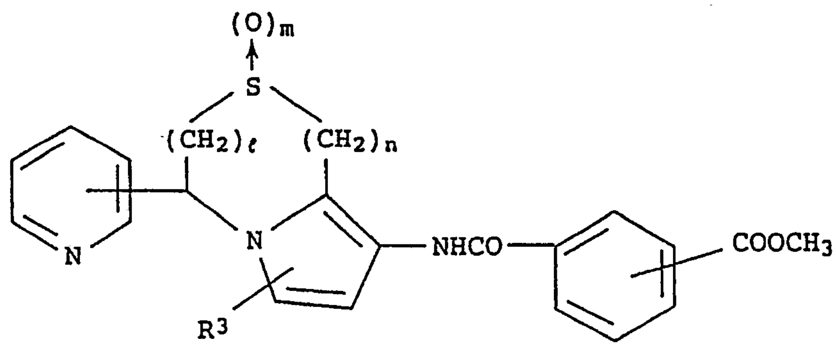

(Il)

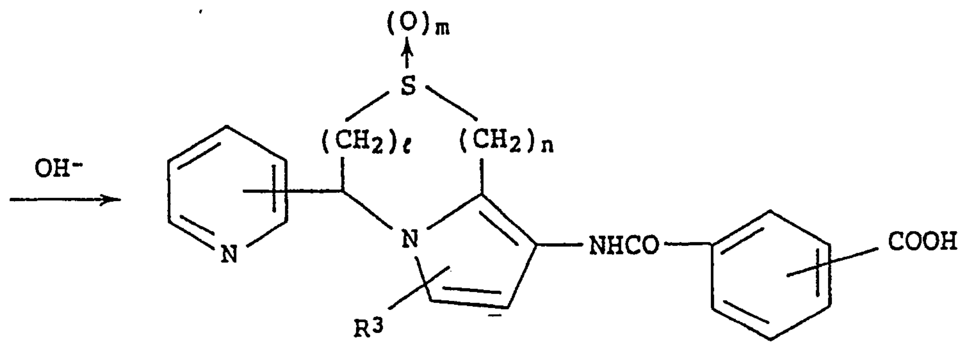


(Im)

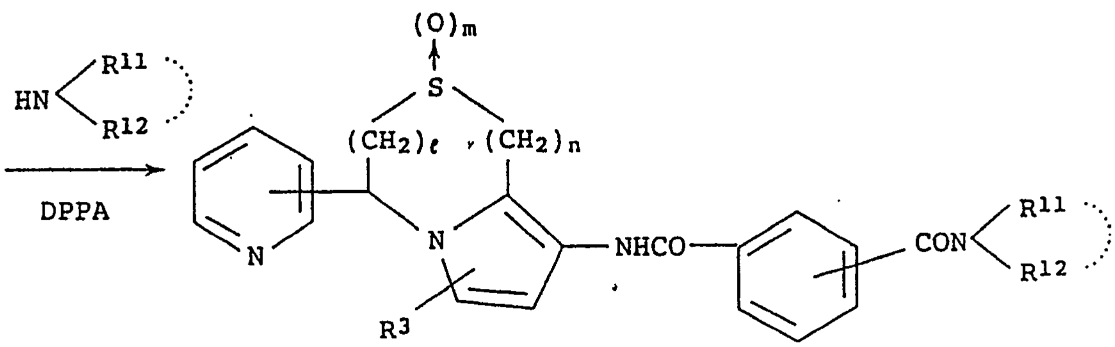


(In)

Among the compounds of the formula (I), those compounds (In) in which the substituent on the aryl group is

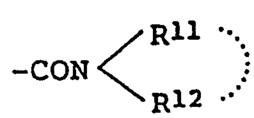

can be produced by treating a compound of the above formula (II) with a base such as caustic soda, caustic potash or sodium hydrogen carbonate and reacting the resulting compound (Im) with an amine of the formula

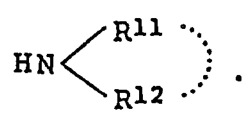

The amidation in the latter step can be carried out in the same manner as Process 4.

<u>Process 10</u> (Alkylation)

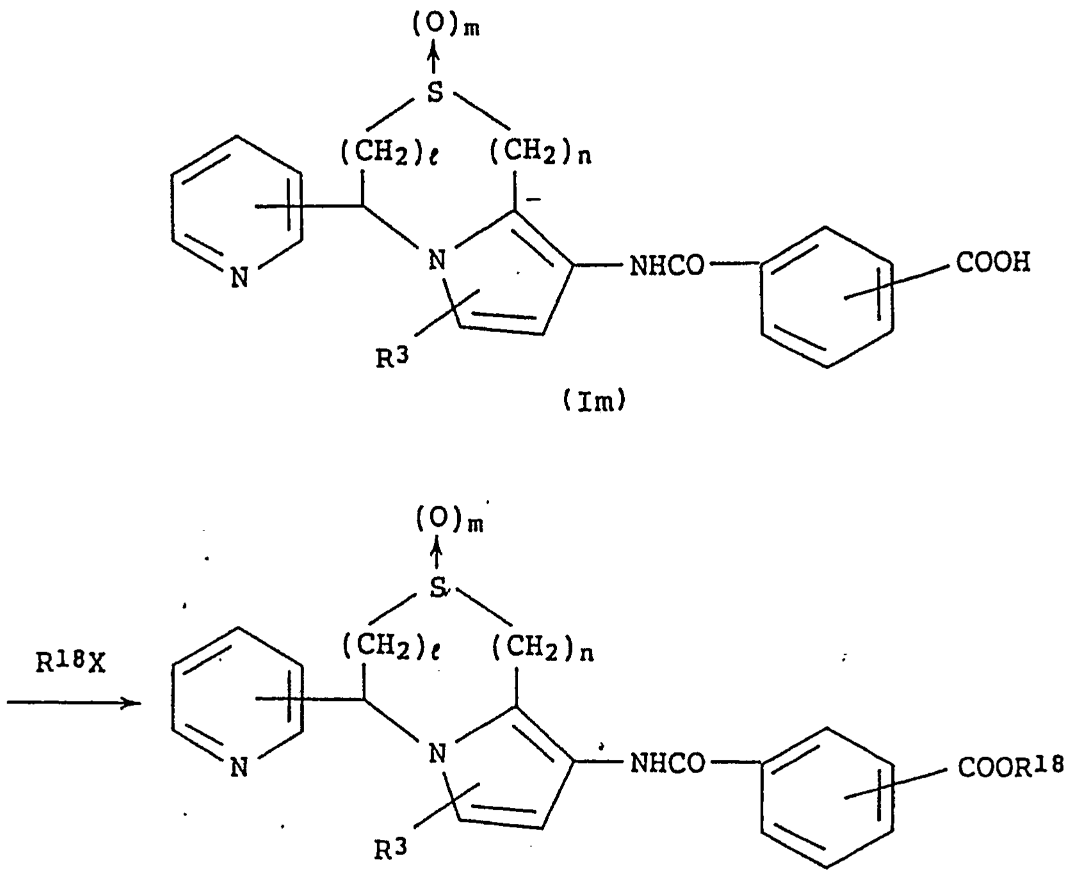


(Im)

(Io)

In the above formulas, $R^{18}$ is a lower alkyl group, and ℓ, m, n, $R^3$ and X are as defined in the above formula (I).

Among the compounds of the formula (I), those compounds (Io) in which the substituent on the aryl group is a lower alkoxycarbonyl group ($-COOR^{18}$) can be produced also by reacting the compound (Im) with a lower alkyl halide ($R^{18}X$). The reaction can proceed with ease in a solvent, such as dimethylformamide, methyl ethyl ketone, ethyl acetate or benzene, in the presence of a base.

Process 11

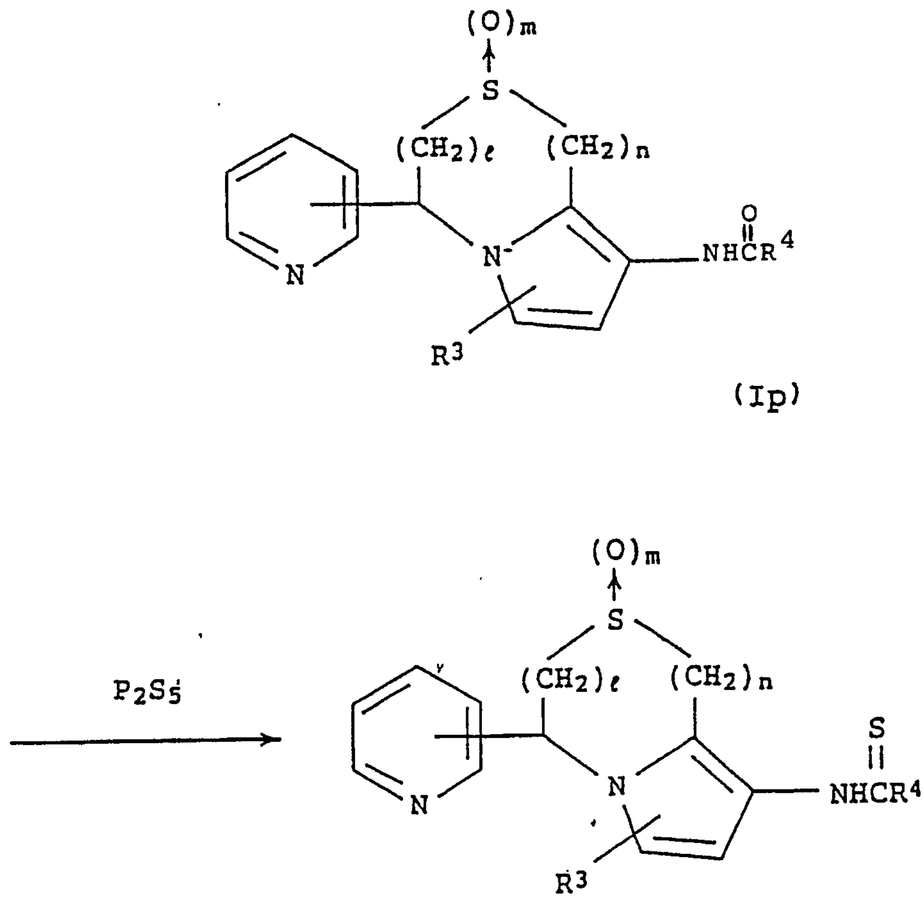


(Ip)

(Iq)

Among the compounds of formula (I), those compounds (Iq) in which A is a sulfur atom can be produced by treating the corresponding compound (Ip) in which A is an oxygen atom with phosphorus pentasulfide in the presence of a base.

Process 12

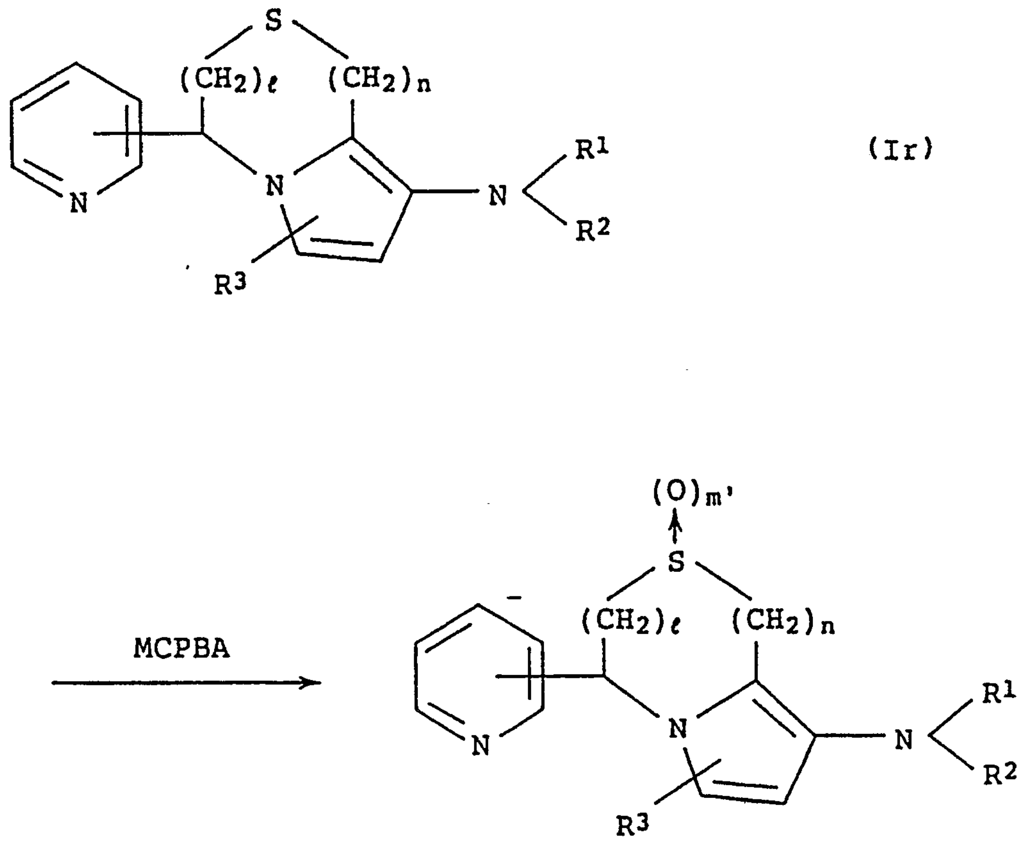


(Ir)

(Is)

Among the compounds of the formula (I), those S-oxide compounds (Is) in which m′ is 1 or 2 can be produced by treating the corresponding thio compound (Ir) with an oxidizing agent in the conventional manner in a solvent such as chloroform or dichloromethane. Generally usable as the oxidizing agent are 10 to 40% hydrogen peroxide, perbenzoic acid, m-chloroperbenzoic acid and the like. In this case, the monooxide (m = 1) or dioxide (m = 2) can be produced as desired by appropriately selecting such reaction conditions as reaction period, temperature and amount of oxidizing agent.

The reaction product produced in each of the processes mentioned above is isolated and purified either in the free form or in the form of a salt. The product in free form can be converted to salts thereof by subjecting the free-form compounds to a conventional salt formation reaction.

The isolation and purification can be effected by such chemical procedures as extraction, concentration, crystallization, filtration, recrystallization and various types of chromatography.

The compounds (I) according to the invention, inclusive of salts thereof, have PAF-antagonizing activity and are useful in the treatment and prevention of various diseases induced by PAF. In particular, they can be used as antiasthmatic agents, antiinflammatory agents, antinuclear agents, shock symptom relievers, therapeutic agents for ischemic heart diseases, ischemic cerebral disease, liver disease, thrombosis and nephritis, and rejection suppressing agents in organ transplantation, among others.

Among the compounds according to the present invention, there are compounds having active oxygen production inhibiting activity as well. They are particularly useful as antiinflammatory agents, antiulcer agents, shock symptom relievers, and therapeutic agents for ischemic heart diseases, ischemic cerebral disease, liver disease and nephritis. Some compounds of the present invention possess an activity in depressing endothelin activities.

The anti-PAF activity of the compounds according to the invention has been confirmed in the following manner.

Inhibition of PAF-induced platelet aggregation

Test method:

Nine volumes of blood was taken from male Japanese white rabbits weighing about 3 kg via the auricular artery into a plastic syringe containing 1 volume of 3.8% aqueous solution of sodium citrate. The blood was centrifuged at 270 x g for 10 minutes to give a supernatant, which was the so-called "platelet rich plasma (PRP)". The remaining portion of the blood was further centrifuged as 1,100 x g for 15 minutes to give platelet poor plasma (PPP). The number of platelets was adjusted to 500,000 per microliter by diluting PRP with PPP and the PAF-induced platelet aggregation was estimated by the method of Born and Cross [Journal of Physiology, vol. 168, pages 178-195 (1963)].

Thus, the change in light transmittance of PRP as caused by PAF ($10^{-8}$ M) was measured using an NBS hematotracer (Niko Bioscience). Each test compound was added 2 minutes prior to addition of PAF and $IC_{50}$ (50% inhibitory concentration) value was determined based on the percent inhibition data relative to the maximum light transmittance after addition of PAF in the control. The results thus obtained with a number of representative compounds are shown below in Table 1.

Table 1

| Example No. | $IC_{50}$ ($\mu$M) | Example No. | $IC_{50}$ ($\mu$M) | Example No. | $IC_{50}$ ($\mu$M) |
|---|---|---|---|---|---|
| 6 | 0.092 | 71 | 0.069 | 108 | 0.049 |
| 26 | 0.088 | 72 | 0.088 | 117 | 0.099 |
| 34 | 0.035 | 73 | 0.099 | 119 | 0.088 |
| 40 | 0.085 | 74 | 0.075 | 120 | 0.076 |
| 41 | 0.086 | 75 | 0.061 | 121 | 0.069 |
| 42 | 0.081 | 81 | 0.083 | 122 | 0.018 |
| 61 | 0.074 | 92 | 0.092 | | |
| 63 | 0.032 | 93 | 0.086 | | |
| 68 | 0.062 | 103 | 0.094 | | |
| 69 | 0.072 | 104 | 0.099 | | |

The compounds according to the present invention do not inhibit ADP (3 $\mu$M)-induced, arachidonic acid (100 $\mu$M)-induced or collagen (10 $\mu$g/ml)-induced platelet aggregation and therefore appear to antagonize PAF specifically.

Some of the compounds according to the present invention show a long duration of action and are stable in aqueous solution. Such compounds can be used not only in the form of oral preparations but also in the form of injections or the like liquid preparations.

The compounds (I) according to the present invention can be safely administered to humans orally or nonorally either as such or in admixture with known pharmaceutically acceptable carriers, excipients and/or the like, namely in the form of pharmaceutical preparations [e.g. tablets, capsules, powders, granules, pills, ointments, syrups, injections (lyophilizate form and solution form), inhalations, suppositories]. The dose may vary depending on the subject to be treated, route of administration, symptom and other factors. Generally, however, a daily dose of 0.1 to 500 mg, preferably 1 to 200 mg, per adult human is administered orally or nonorally in two or three divided doses.

| Dosage Form Example 1 | |
|---|---|
| Tablets | |
| Compound of Example 119 | 20 mg |
| Lactose | 57 mg |
| Corn starch | 38 mg |
| Hydroxypropylcellulose | 4 mg |
| Magnesium stearate | 1 mg |
| Total (per tablet) | 120 mg |

The compound of Example 119 (20 g), 57 g of lactose and 38 g of corn starch are mixed up homogeneously. Then 40 g of 10% hydroxypropylcellulose solution is added and the resulting mixture is wet-granulated. After sieving, the granules are dried and mixed with 1 g of magnesium stearate. The resulting mixture is tableted using a die-punch system (7 mm, 5.6 R).

| Dosage Form Example 2 | |
|---|---|
| Capsules | |
| Compound of Example 119 | 15 mg |
| Crystalline cellulose | 40 mg |
| Crystalline lactose | 144 mg |
| Magnesium stearate | 1 mg |
| Total (per capsule) | 200 mg |

The compound of Example 119 (15 g), 40 g of crystalline cellulose, 144 g of crystalline lactose and 1 g of magnesium stearate are mixed up homogeneously and the mixture is filled into No. 3 capsules using a capsule filling machine.

| Dosage Form Example 3 | |
|---|---|
| Lyophilized preparation | |
| Compound of Example 119 | 1 mg |
| D-Mannitol | 50 mg |
| Total (per vial) | 51 mg |

The compound of Example 119 (1 g) and 50 g of D-Mannitol are dissolved, in that order, in 800 ml of water. Water is added to the solution to make one liter. The resulting solution is subjected to bacterial filtration, then distributed in 1-ml portions into vials and lyophilized. The thus-obtained lyophilizate in each vial is dissolved in distilled water for injection or the like prior to use.

The following examples are further illustrative of the present invention. Unless otherwise indicated, the ratios used hereinafter are by volume.

## Reference Example 1

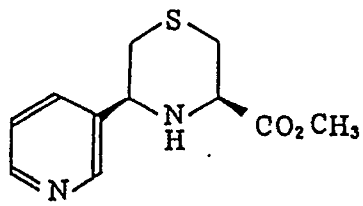

A mixture of 28.1 g of 3-bromoacetylpyridine hydrobromide, 17.2 g of L-cysteine methyl ester hydrochloride and 100 ml of water was stirred at room temperature for 4 hours. The reaction mixture was ice-cooled, 25 ml of pyridine and 3.8 g of sodium borohydride were added thereto, and the mixture was stirred overnight at room temperature. The reaction mixture was diluted with 500 ml of water and extracted with methylene chloride. The extract was dried over anhydrous sodium sulfate and the solvent was then distilled off under reduced pressure. The residue was subjected to silica gel column chromatography. Elution with ethyl acetate-n-hexane (1:2) gave 4.9 g of (3R,5S)-3-methoxycarbonyl-5-(3-pyridyl)-thiomorpholine.
Melting point: 98-101 °C

| Elemental analysis (for $C_{11}H_{14}N_2O_2S$) | | | | |
|---|---|---|---|---|
| | C (%) | H (%) | N (%) | S (%) |
| Calcd.: | 55.44 | 5.92 | 11.76 | 13.46 |
| Found: | 55.23 | 5.94 | 11.70 | 13.45 |

Reference Example 2

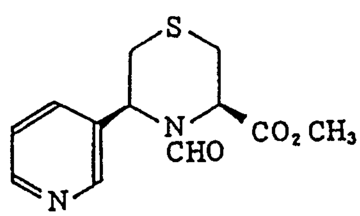

A mixture of 0.6 ml of formic acid and 1.5 ml of acetic anhydride was added to a mixture of 1.9 g of (3R,5S)-3-methoxycarbonyl-5-(3-pyridyl)thiomorpholine and 16 ml of methylene chloride with ice cooling, and the resultant mixture was stirred at room temperature for 4 hours. The solvent was then distilled off under reduced pressure, the residue was dissolved in 30 ml of methylene chloride, and the solution was neutralized with saturated aqueous sodium hydrogen carbonate solution and then extracted with methylene chloride. The extract was dried over anhydrous sodium sulfate and the solvent was distilled off under reduced pressure. The residue was recrystallized from methylene chloride-n-hexane to give 2.1 g of (3R,5S)-4-formyl-3-methoxycarbonyl-5-(3-pyridyl)thiomorpholine.
Melting point: 101 - 104 °C

| Elemental analysis (for $C_{12}H_{14}N_2O_3S$) | | | | |
|---|---|---|---|---|
| | C (%) | H (%) | N (%) | S (%) |
| Calcd.: | 54.12 | 5.30 | 10.52 | 12.04 |
| Found: | 54.04 | 5.24 | 10.47 | 12.22 |

## Reference Example 3

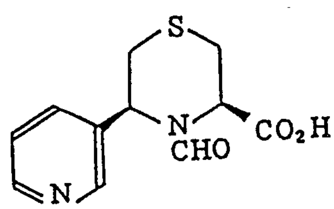

To a mixture of 2.1 g of (3R,5S) 4-formyl-3-methoxycarbonyl-5-(3-pyridyl)thiomorpholine and 24 ml of methanol was added 9 ml of 1 N aqueous sodium hydroxide. The resultant mixture was stirred at room temperature for 3 hours. 1 N Hydrochloric acid (9 ml) wad added to the reaction mixture with ice cooling and the solvent was distilled off under reduced pressure. Hot ethanol (80 ml) was added to the residue, the insoluble matter was removed, and the solvent was distilled off under reduced pressure to give 2 g of (3R,5S)-4-formyl-5-(3-pyridyl)thiomorpholine-3-carboxylic acid.
Melting point: 180-183 °C (decomposition)

| Elemental analysis (for $C_{11}H_{12}N_2O_3S$) | | | | |
|---|---|---|---|---|
| | C (%) | H (%) | N (%) | S (%) |
| Calcd.: | 52.37 | 4.79 | 11.10 | 12.71 |
| Found: | 52.23 | 4.73 | 11.10 | 12.45 |

## Reference Example 4

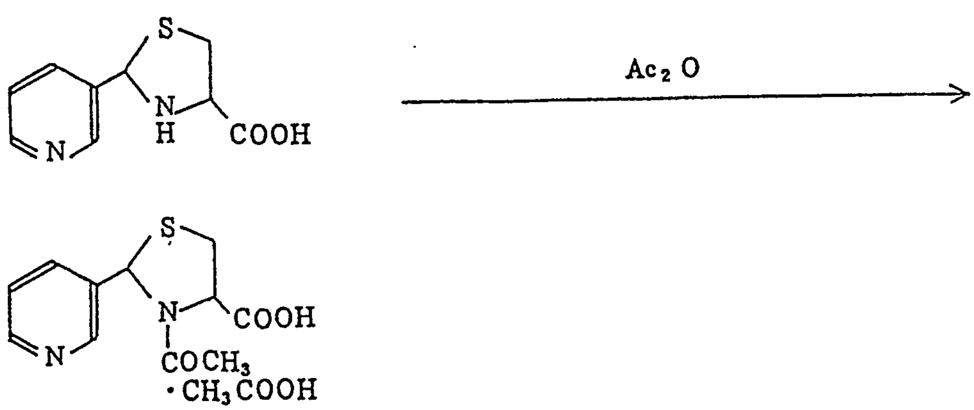


Acetic anhydride (6 ml) was added to a mixture of 5 g of 2-(3-pyridyl)thiazolidine-4-carboxylic acid and 50 ml of methylene chloride with ice cooling. The resultant mixture was stirred overnight at room temperature. The reaction mixture was concentrated under reduced pressure, ether was added to the residue for washing the same, the ether was then removed by decantation, and the residue was dried to give 5.84 g of 3-acetyl-2-(3-pyridyl)thiazolidine-4-carboxylic acid acetate.
Mass analysis, m/z: 252 $(M-CH_3COOH)^+$

## Reference Example 5

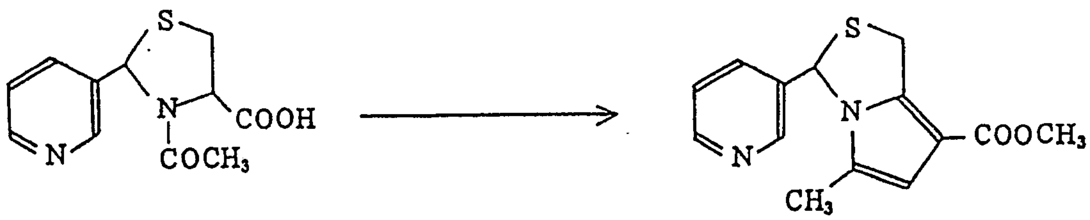

A mixture of 5.84 g of 3-acetyl-2-(3-pyridyl)thiazolidine-4-carboxylic acid, 7 ml of methylene chloride and 4.16 g of triethylamine was added dropwise over 1.75 hours to a mixture of 7.49 g of p-toluenesulfonyl chloride, 15 ml of methylene chloride and 3.38 g of methyl 2,3-dichloropropionate at 40-42 °C. After completion of the addition, the resultant mixture was stirred at the same temperature for 20 minutes. Then, 6.21 g of triethylamine was added to said mixture over 1 hour at the same temperature. Water (10 ml) was added to the reaction mixture and, after phase separation, the organic layer was separated, washed with water, dried over anhydrous magnesium sulfate and concentrated under reduced pressure. The oily residue was subjected to silica gel column chromatography (with 150 g of silica gel). Elution with chloroform gave 3.3 g of methyl 5-methyl-3-(3-pyridyl)-1H,3H-pyrrolo[1,2-c]-7-thiazolecarboxylate.
NMR spectrum ($CDCl_3$)
δ: 1.83 (3H, s), 3.78 (3H, s), 4.38-4.5 (2H, m), 6.27 (1H, d), 6.36 (1H, d), 7.1-7.4 (2H, m), 8.38 (1H, s), 8.54 (1H, t)

Reference Example 6

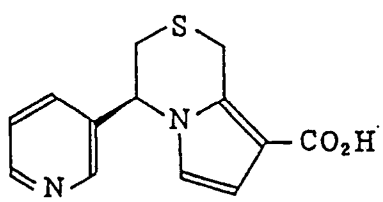

A mixture of 1.8 g of (3R,5S)-4-formyl-5-(3-pyridyl)thiomorpholine-3-carboxylic acid, 1.2 ml of triethylamine and 14 ml of methylene chloride was added to a mixture of 1.5 g of p-toluenesulfonyl chloride, 1 ml of methyl 2,3-dichloropropionate and 7 ml of methylene chloride under reflux and the resultant mixture was refluxed for 1 hour with stirring. Triethylamine (2.3 ml) was added to the reaction mixture. The whole mixture was further refluxed for 2 hours with stirring, then cooled to room temperature, diluted with 100 ml of water and extracted with methylene chloride. The extract was dried over anhydrous sodium sulfate and the solvent was then distilled off under reduced pressure. The residue was subjected to silica gel column chromatography. Elution with ethyl acetate-n-hexane (1:1) gave 1.24 g of a light-yellow oil. The oil was dissolved in 13 ml of methanol, 6.6 ml of 1 N aqueous sodium hydroxide was added, and the mixture was refluxed overnight with stirring and then cooled to room temperature. 1 N Hydrochloric acid (6.6 ml) was added, and the resultant mixture was stirred for 30 minutes. The solvent was distilled off under reduced pressure and the residue was subjected to silica gel column chromatography. Elution with chloroform-methanol (10:1) gave 810 mg of (4S)-3,4-dihydro-4-(3-pyridyl)-1H-pyrrolo[2,1-c]thiazine-8-carboxylic acid.
Mass analysis, m/z: 260 ($M^+$)
NMR spectrum (DMSO-$d_6$)
δ: 3.30 (2H, ddd), 4.18 (2H, s), 5.65 (1H, t), 6.35 (1H, d), 6.39 (1H, d), 7.34 (2H, br), 8.0-8.7 (2H, m), 11.96 (1H, br)

Reference Example 7

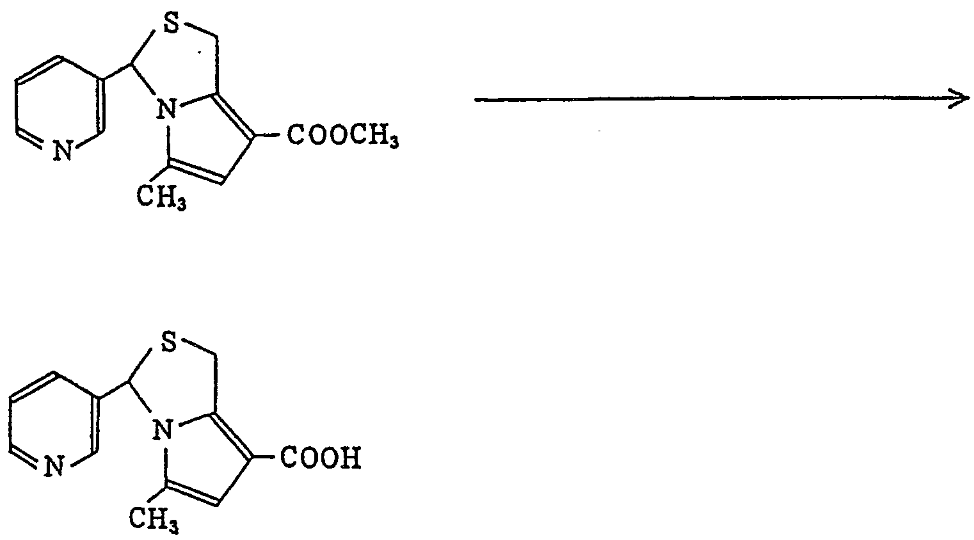

A solution of 2.4 g of potassium hydroxide in 14 ml of water was added to a mixture of 3.32 g of methyl 5-methyl-3-(3-pyridyl)-1H,3H-pyrrolo[1,2-c]-7-thiazolecarboxylate and 9 ml of ethanol and the resultant mixture was stirred at 40° C for 17 hours. The reaction mixture was cooled and adjusted to pH 4 by addition of concentrated hydrochloric acid. The resultant crystals were collected by filtration, washed with ethanol and dried to give 2.45 g of 5-methyl-3-(3-pyridyl)-1H,3H-pyrrolo[1,2-c]-7-thiazolecarboxylic acid.
Melting point: 225° C.

Example 1

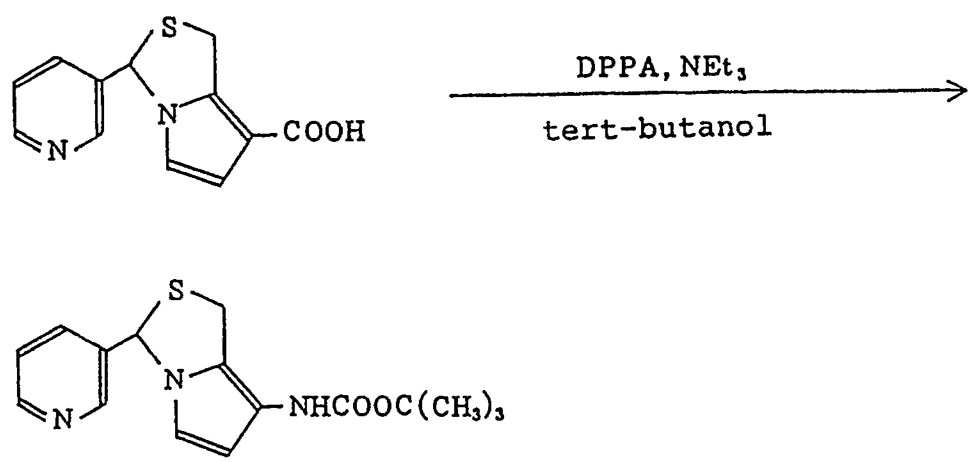


A mixture of 2.06 g of 3-(3-pyridyl)-1H,3H-pyrrolo[1,2-c]-7-thiazolecarboxylic acid, 2.31 g of diphenylphosphoryl azide, 0.85 g of triethylamine and 10 ml of tert-butanol was heated at 80° C for 16 hours with stirring. The reaction mixture was concentrated under reduced pressure and the residue was dissolved in 150 ml of ethyl acetate. The ethyl acetate solution was washed in sequence with dilute aqueous sodium hydrogen carbonate solution and water, and dried over anhydrous magnesium sulfate. The solvent was then distilled off under reduced pressure and the residue was subjected to silica gel column chromatography (with 30 g of silica gel). Elution with toluene-ethyl acetate (9:1) gave 1.415 g of 7-tert-butoxycarbonylamino-3-(3-pyridyl)-1H,3H-pyrrolo[1,2-c]thiazole.

| Elemental analysis (for $C_{16}H_{19}N_3O_2S$) | | | | |
|---|---|---|---|---|
| | C (%) | H (%) | N (%) | S (%) |
| Calcd.: | 60.54 | 6.03 | 13.24 | 10.10 |
| Found: | 60.74 | 5.97 | 13.10 | 10.14 |

Mass analysis, m/z: 317 ($M^+$)

Example 2

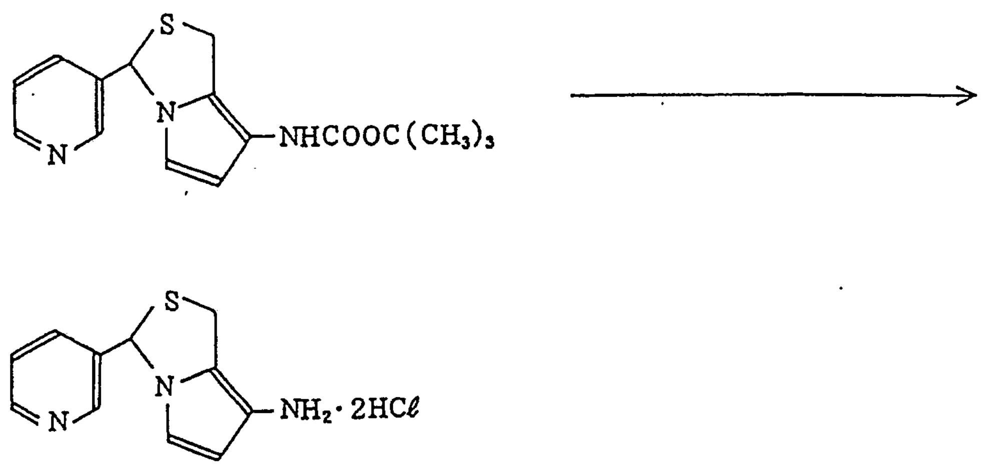

Trifluoroacetic acid (10 ml) was added to 1.4 g of 7-tert-butoxycarbonylamino-3-(3-pyridyl)-1H,3H-pyrrolo[1,2-c]thiazole with ice cooling. The mixture was stirred at room temperature for 1 hour and the solvent was then distilled off under reduced pressure. The residue was dissolved in ethyl acetate, 4 N hydrogen chloride solution in dioxane was added in large excess, and the resultant crystals were collected by filtration, washed with ethyl acetate and dried to give 1.3 g of 7-amino-3-(3-pyridyl)-1H,3H-pyrrolo[1,2-c]-thiazole dihydrochloride.

NMR spectrum (DMSO-$d_6$)

δ: 4.37 (2H, q), 6.28 (1H, d), 6.71 (1H, d), 6.85 (1H, s), 7.81-8.14 (2H, m), 8.70 (1H,d), 8.82 (1H, dd)

Mass spectrum, m/z: 217 $(M-2HCl)^+$

Treatment of the dihydrochloride with an alkali gave 7-amino-3-(3-pyridyl)-1H,3H-pyrrolo[1,2-c]thiazole.

NMR spectrum ($CDCl_3$)

δ: 4.18 (2H, s), 6.02 (1H, d), 6.23 (1H, d), 6.36 (1H, d), 7.39 (1H, dd), 7.72 (1H, dt), 8.62 (1H, dd), 8.69 (1H, dd)

Mass spectrum, m/z: 217 ($M^+$)

The corresponding fumarate was produced in the following manner.

7-Amino-3-(3-pyridyl)-1H,3H-pyrrolo[1,2-c]thiazole (430 mg) was dissolved in 5 ml of isopropanol. To the solution was added a mixture of 230 mg of fumaric acid and isopropanol. The resultant mixture was stirred at room temperature for 1 hour. Collection of the resultant crystals by filtration gave 350 mg of 7-amino-3-(3-pyridyl)-1H,3H-pyrrolo[1,2-c]thiazole fumarate.

Melting point: 120-125 °C (decomposition)

| Elemental analysis | |
|---|---|
| | S (%) |
| calcd.: | 9.62 |
| Found : | 9.60 |

Example 3

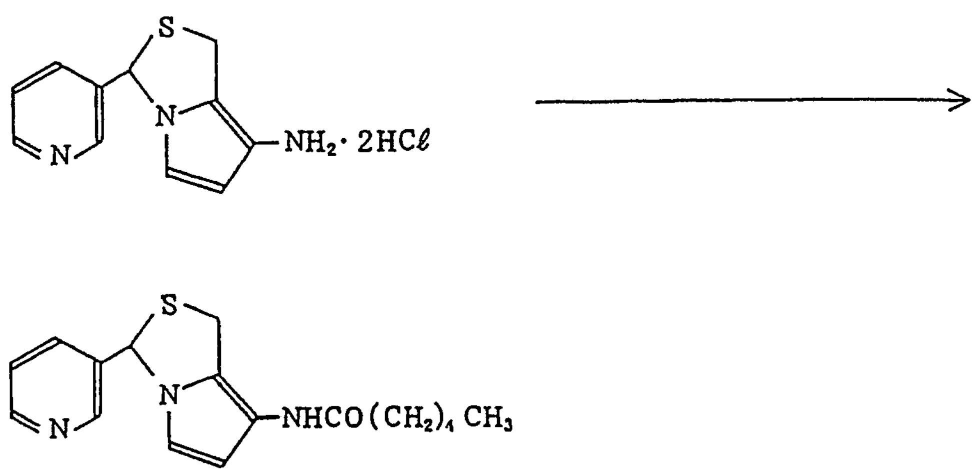

n-Caproic anhydride (0.4 g) was added to a mixture of 300 mg of 7-amino-3-(3-pyridyl)-1H,3H-pyrrolo-[1,2-c]thiazole dihydrochloride, 3 ml of chloroform and 1 ml of triethylamine and the resultant mixture was stirred at room temperature for 2 days. Ethyl acetate (50 ml) was added, and the mixture was washed with dilute aqueous sodium hydrogen carbonate solution and water in that order and dried over anhydrous magnesium sulfate. The solvent was distilled off under reduced pressure and the residue was subjected to silica gel column chromatography. Elution with chloroform gave 210 mg of 7-hexanamido-3-(3-pyridyl)-1H,3H-pyrrolo[1,2-c]thiazole.

| Elemental analysis (for $C_{17}H_{21}N_3OS$) | |
|---|---|
| | S (%) |
| Calcd.: | 10.17 |
| Found: | 9.88 |

NMR spectrum ($CDCl_3$)

δ: 0.90 (3H, t), 1.1-1.5 (4H, m), 1.5-2.0 (2H,m), 2.29 (2H, t), 4.24 (2H, s), 6.04 (1H, d), 6.14 (1H, d), 6.24 (1H, s), 7.1-7.3 (2H, m), 7.51 (1H, dt), 8.4-8.6 (2H, m)

Example 4

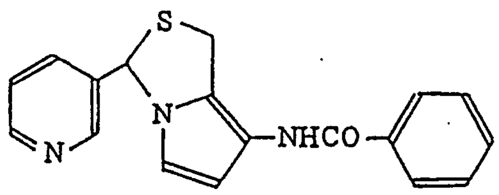

Benzoyl chloride (200 mg) was added to a mixture of 300 mg of 7-amino-3-(3-pyridyl)-1H,3H-pyrrolo-[1,2-c]thiazole dihydrochloride, 3 ml of chloroform and 1 ml of triethylamine with ice cooling. The resultant mixture was stirred overnight at room temperature and then treated in the same manner as Example 3 to give N-[3-(3-pyridyl)-1H,3H-pyrrolo[1,2-c]thiazol-7-yl]benzamide. Recrystallization from isopropyl alcohol gave 140 mg of crystals.
Melting point: 150-151 °C

| Elemental analysis (for $C_{18}H_{15}N_3OS$) | | | | |
|---|---|---|---|---|
| | C (%) | H (%) | N (%) | S (%) |
| Calcd.: | 67.24 | 4.70 | 13.07 | 9.98 |
| Found: | 67.08 | 4.65 | 13.02 | 9.86 |

Example 5

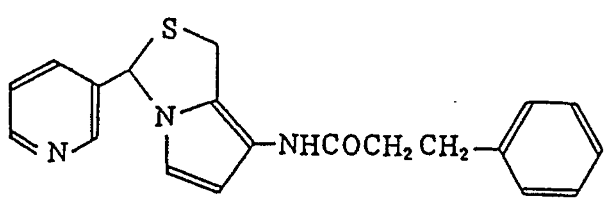

One drop of N,N-dimethylformamide was added to a mixture of 300 mg of 3-phenylpropionic acid and 5 ml of methylene chloride, followed by addition of 2 ml of oxalyl chloride with ice cooling. The resultant mixture was stirred at room temperature for 1 hour, the solvent and excess oxalyl chloride were distilled off under reduced pressure to give 3-phenylpropionyl chloride. The acid chloride thus obtained and 330 mg of 7-amino-3-(3-pyridyl)-1H,3H-pyrrolo[1,2-c]thiazole dihydrochloride were used as starting materials and the procedure of Example 4 was followed to give 38 mg of 7-(3-phenylproprionamido)-3-(3-pyridyl)-1H,3H-pyrrolo[1,2-c]thiazole.
Melting point: 148-149 °C

| Elemental analysis (for $C_{20}H_{19}N_3OS$) | |
|---|---|
| | S (%) |
| Calcd.: | 9.18 |
| Found : | 9.42 |

Example 6

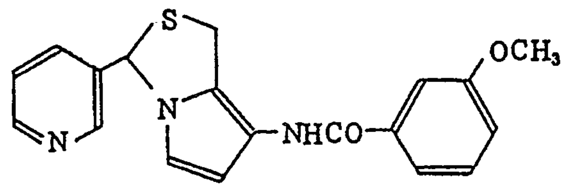

m-Anisic acid (500 mg) and 500 mg of 7-amino-3-(3-pyridyl)-1H,3H-pyrrolo[1,2-c]thiazole were used as starting materials and the procedure of Example 5 was followed to give 60 mg of m-methoxy-N-[3-(3-pyridyl)-1H,3H-pyrrolo[1,2-c]thiazol-7-yl]benzamide.
Melting point: 134-135° C

| Elemental analysis (for $C_{19}H_{17}N_3O_2S$) | | | | |
|---|---|---|---|---|
| | C (%) | H (%) | N (%) | S (%) |
| Calcd.: | 64.94 | 4.88 | 11.96 | 9.12 |
| Found: | 65.00 | 4.84 | 11.90 | 9.14 |

Example 7

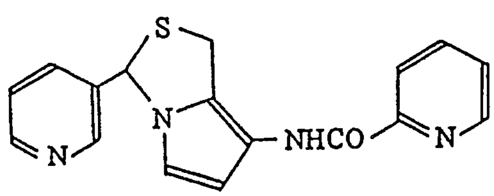

7-Amino-3-(3-pyridyl)-1H,3H-pyrrolo[1,2-c]thiazole (500 mg) and 430 mg of picolinoyl chloride hydrochloride were used as starting materials and the procedure of Example 4 was followed to give 410 mg of 7-(2-pyridinecarboxamido)-3-(3-pyridyl)-1H,3H-pyrrolo[1,2-c]thiazole.
NMR spectrum ($CDCl_3$)
δ: 4.46 (2H, s), 6.27 (1H, d), 6.36 (1H, s), 6.38 (1H, s), 7.11-7.98 (4H, m), 8.26 (1H, dt), 8.32-8.63 (3H, m)
Mass analysis, m/z: 322 ($M^+$)

Example 8

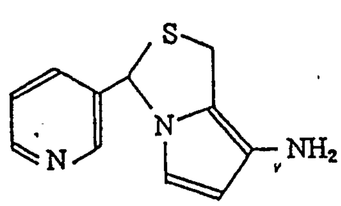

+

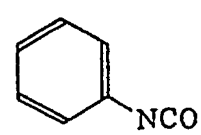

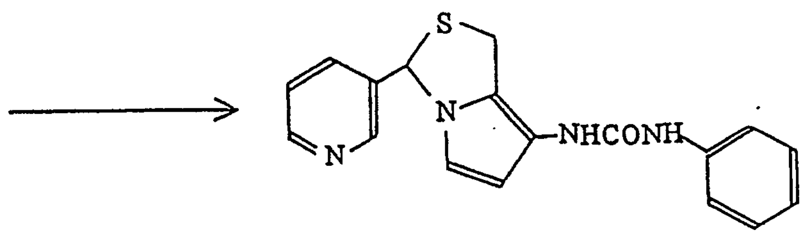

Phenyl isocyanate (275 mg) was added to a solution of 500 mg of 7-amino-3-(3-pyridyl)-1H,3H-pyrrolo-[1,2-c]thiazole in 5 ml of methylene chloride with ice cooling and the mixture was stirred. After 30 minutes, the resultant crystals were collected by filtration, washed with ethanol and recrystallized from ethanol to give 260 mg of 7-(3-phenylureido)-3-(3-pyridyl)-1H,3H-pyrrolo[1,2-c]thiazole.
Melting point: 213-215 ° C

| Elemental analysis (for $C_{18}H_{16}N_4OS$) | | | | |
|---|---|---|---|---|
| | C (%) | H (%) | N (%) | S (%) |
| Calcd.: | 64.26 | 4.79 | 16.65 | 9.53 |
| Found: | 63.99 | 4.82 | 16.48 | 9.70 |

Example 9

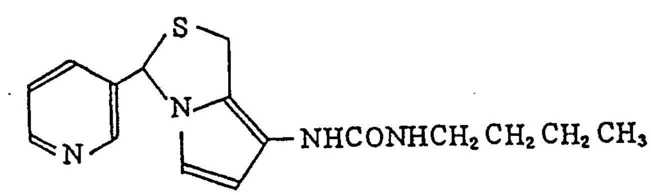

n-Butyl isocyanate (150 mg) was added to a mixture of 300 mg of 7-amino-3-(3-pyridyl)-1H,3H-pyrrolo-[1,2-c]thiazole dihydrochloride, 3 ml of chloroform and 1 ml of triethylamine and the resultant mixture was stirred overnight at room temperature. Ethyl acetate (100 ml) was added to the reaction mixture and the resultant mixture was washed with dilute aqueous sodium hydrogen carbonate solution and water in that order and dried over anhydrous magnesium sulfate. The solvent was then distilled off under reduced pressure and the solid residue was recrystallized from isopropyl alcohol in the presence of activated carbon to give 50 mg of 7-(3-butylureido)-3-(3-pyridyl)-1H,3H-pyrrolo[1,2-c]thiazole.
Melting point: 191-192 ° C

| Elemental analysis (for $C_{16}H_{20}N_4OS$) | | | | |
|---|---|---|---|---|
| | C(%) | H(%) | N(%) | S(%) |
| Calcd.: | 60.73 | 6.37 | 17.71 | 10.13 |
| Found: | 60.44 | 6.30 | 17.53 | 9.88 |

Example 10

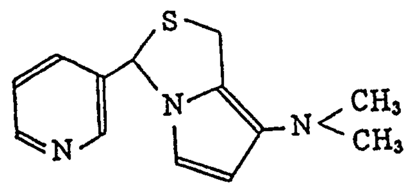

A mixture of 500 mg of 7-amino-3-(3-pyridyl)-1H,3H-pyrrolo[1,2-c]thiazole, 330 mg of methyl iodide, 5 ml of N,N-dimethylformamide, 330 mg of anhydrous potassium carbonate and a catalytic amount of tetra-n-butylammonium bromide was stirred overnight at room temperature. Ethyl acetate (50 ml) was added to the reaction mixture, the whole mixture was washed with water and dried over anhydrous magnesium sulfate, and the solvent was distilled off. The residue was subjected to silica gel column chromatography (with 20 g of silica gel). Elution with toluene-ethyl acetate (4:1) gave 90 mg of 7-dimethylamino-3-(3-pyridyl)-1H,3H-pyrrolo[1,2-c]thiazole.

NMR spectrum ($CDCl_3$)

δ: 2.74 (6H, s), 4.29 (2H, s), 6.01 (1H, d), 6.18 (1H, d), 7.21-7.36 (1H, m), 7.55 (1H,dt), 8.51 (1H, dd), 8.58 (1H, dd)

Mass analysis, m/z: 245 ($M^+$)

Example 11

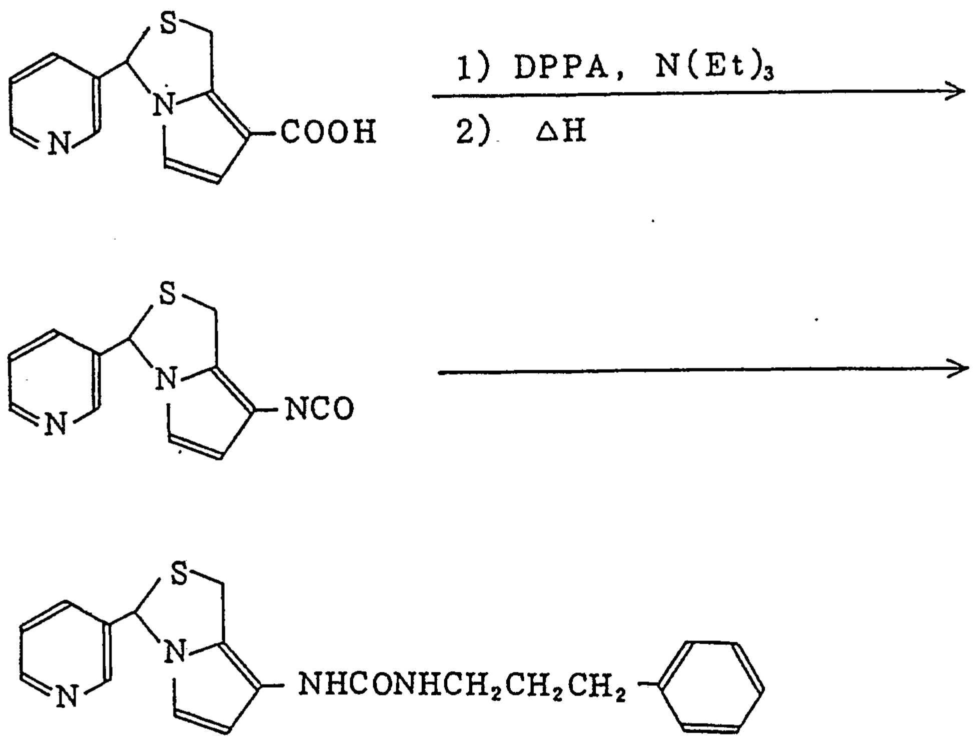


Diphenylphosphoryl azide (0.69 g) was added to a mixture of 0.61 g of 3-(3-pyridyl)-1H,3H-pyrrolo[1,2-c]thiazolecarboxylic acid, 5 ml of N,N-dimethylformamide and 0.26 g of triethylamine, and the resultant mixture was stirred overnight at room temperature. Ethyl acetate (200 ml) was added to the reaction mixture and the whole mixture was washed in sequence with dilute aqueous sodium hydrogen carbonate solution and water and dried over anhydrous magnesium sulfate. The solvent was then distilled off, 5 ml of xylene was added to the residue and the mixture was heated under reflux for 1 hour. 3-Phenylpropylamine (0.6 g) and 5 ml of toluene were added to the reaction mixture, whereupon crystals precipitated out. The crystals were collected by filtration and recrystallized from ethanol in the presence of activated carbon to give 300

mg of 7-(3-phenethylureido)-3-(3-pyridyl)-1H,3H-pyrrolo[1,2-c]thiazole.
Melting point: 174-175 °C

| Elemental analysis (for $C_{21}H_{22}N_4OS$) | | | | |
|---|---|---|---|---|
| | C (%) | H (%) | N (%) | S (%) |
| Calcd.: | 66.64 | 5.86 | 14.80 | 8.47 |
| Found : | 66.38 | 5.80 | 14.75 | 8.49 |

Example 12

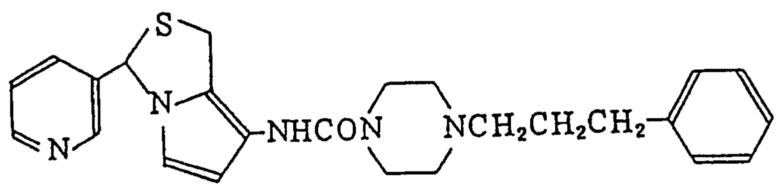

Diphenylphosphoryl azide (0.68 g) was added to a mixture of 0.61 g of 3-(3-pyridyl)-1H,3H-pyrrolo[1,2-c]thiazolecarboxylic acid, 5 ml of N,N-dimethylformamide and 0.26 g of triethylamine, and the resultant mixture was stirred overnight at room temperature. Ethyl acetate (200 ml) was added to the reaction mixture, the whole mixture was washed in sequence with dilute aqueous sodium hydrogen carbonate solution and water and dried over anhydrous magnesium sulfate, and the solvent was distilled off under reduced pressure.

Xylene (5 ml) was added to the residue and the mixture was heated under reflux for 1 hour. To this reaction mixture was added a solution of 0.6 g of 1-(3-phenylpropyl)piperazine in 5 ml of toluene with ice cooling. The resultant mixture was heated under reflux for 1 hour. Then, the solvent was distilled off, 15 ml of 1 N hydrochloric acid was added, and the mixture was washed with ethyl acetate, made alkaline by adding sodium hydrogen carbonate in large excess and extracted with ethyl acetate. The extract was washed with water and dried over anhydrous magnesium sulfate, and the solvent was distilled off under reduced pressure. The residue was subjected to silica gel column chromatography (with 15 g of silica gel). Elution with chloroform-methanol (19:1) gave 700 mg of 7-[[[4-(3-phenyl)propyl]-1-piperazinyl]carboxamido]-3-(3-pyridyl)-1H,3H-pyrrolo[1,2-c]thiazole.

NMR spectrum ($CDCl_3$)

δ: 2.6-3.0 (2H, m), 2.2-2.8 (8H, m), 3.42 (4H, d), 4.18 (2H, s), 5.99 (1H, d), 6.10 (1H, d), 6.22 (1H, s), 7.0-7.3 (6H, m), 7.48 (1H, dt), 8.4-8.58 (2H, m)

Mass analysis, m/z: 447 ($M^+$)

Example 13

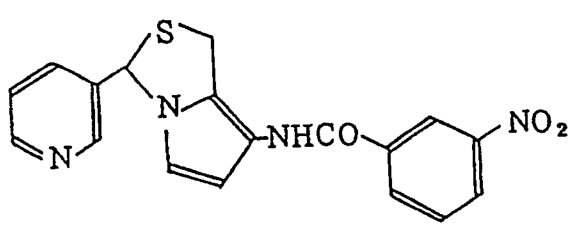

m-Nitrobenzoyl chloride (190 mg) was added to a mixture of 220 mg of 7-amino-3-(3-pyridyl)-1H,3H-pyrrolo[1,2-c]thiazole, 2 ml of methylene chloride and 0.2 ml of triethylamine with ice cooling. The resultant mixture was stirred overnight at room temperature. Saturated aqueous sodium hydrogen carbonate solution (60 ml) was added, and the whole mixture was extracted with methylene chloride. The extract was dried over anhydrous sodium sulfate. The solvent was distilled off under reduced pressure and the residue was subjected to silica gel column chromatography. Elution with methylene chloride-ethyl acetate (2:1) followed by recrystallization from methylene chloride-n-hexane gave 230 mg of 7-(m-nitrobenzoyl)amino-3-(3-pyridyl)-1H,3H-pyrrolo[1,2-c]thiazole.
Melting point: 181-184 °C

| Elemental analysis (for $C_{18}H_{14}N_4O_3S \cdot 0.4H_2O$) | | | | |
|---|---|---|---|---|
| | C (%) | H (%) | N (%) | S (%) |
| Calcd.: | 57.87 | 3.99 | 15.00 | 8.58 |
| Found: | 57.95 | 3.81 | 14.88 | 8.55 |

Example 14

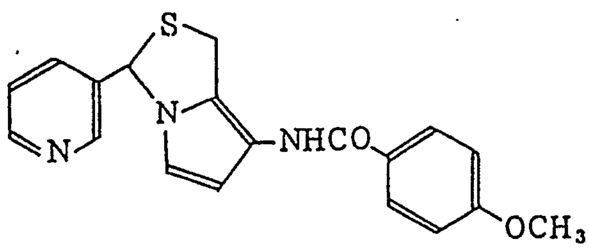

One drop of N,N-dimethylformamide was added to a mixture of 180 mg of p-anisic acid and 2 ml of methylene chloride, followed by addition of 0.4 ml of oxalyl chloride with ice cooling. The mixture was stirred at room temperature for 1 hour and then the solvent and excess oxalyl chloride were distilled off under reduced pressure to give p-anisoyl chloride. The thus-obtained acid chloride and 220 mg of 7-amino-3-(3-pyridyl)-1H,3H-pyrrolo[1,2-c]thiazole were used as starting materials and the procedure of Example 13 was followed to give 210 mg of 7-(p-methoxybenzoyl)amino-3-(3-pyridyl)-1H,3H-pyrrolo[1,2-c]thiazole.
Melting point: 158-161 °C

| Elemental analysis (for $C_{19}H_{17}N_3O_2S$) | |
|---|---|
| | S (%) |
| Calcd.: | 9.12 |
| Found: | 9.06 |

The compounds of Examples 15 to 39 were produced in the same manner as mentioned in above Example 14.

Example 15

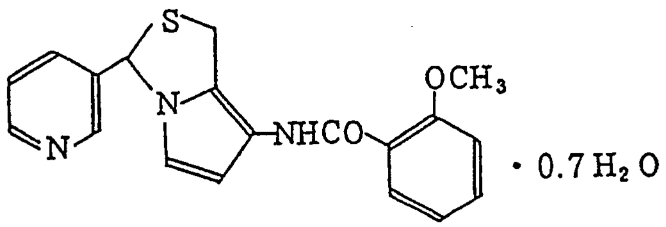

o-Methoxy-N-[3-(3-pyridyl)-1H,3H-pyrrolo[1,2-c]thiazol-7-yl]benzamide 0.7 hydrate.
Starting compounds: 7-amino-3-(3-pyridyl)-1H,3H-pyrrolo[1,2-c]thiazole and o-anisic acid.

| Elemental analysis (for $C_{19}H_{17}N_3O_2S \cdot 0.7H_2O$) | | | | |
|---|---|---|---|---|
| | C (%) | H (%) | N (%) | S (%) |
| Calcd.: | 62.69 | 5.09 | 11.54 | 8.81 |
| Found: | 62.66 | 4.80 | 11.25 | 8.51 |

Mass analysis, m/z: 351 ($M^+$)

## Example 16

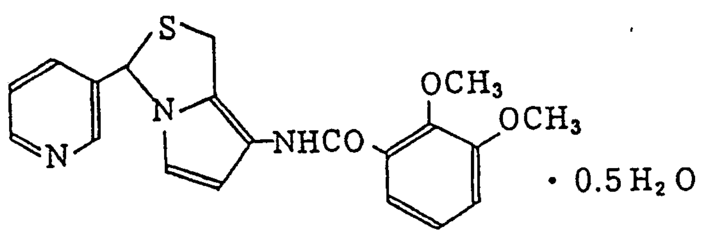

2,3-Dimethoxy-N-[3-(3-pyridyl)-1H,3H-pyrrolo[1,2-c]thiazol-7-yl]benzamide hemihydrate.
Starting compounds: 7-amino-3-(3-pyridyl)-1H,3H-pyrrolo[1,2-c]thiazole and 2,3-dimethoxybenzoic acid.

| Elemental analysis (for $C_{20}H_{19}N_3O_3S \cdot 0.5H_2O$) | | | | |
|---|---|---|---|---|
| | C (%) | H (%) | N (%) | S (%) |
| Calcd.: | 61.51 | 5.17 | 10.76 | 8.21 |
| Found: | 61.50 | 4.99 | 10.57 | 8.04 |

Mass analysis, m/z: 381 ($M^+$)

## Example 17

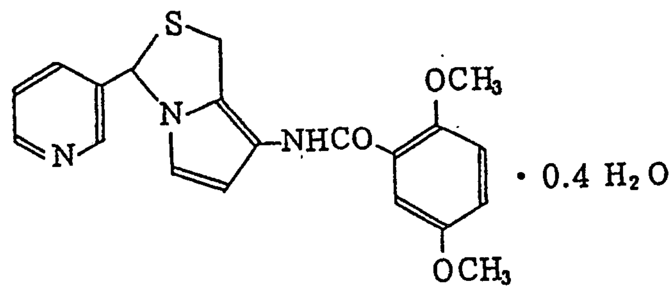

2,5-Dimethoxy-N-[3-(3-pyridyl)-1H,3H-pyrrolo[1,2-c]thiazol-7-yl]benzamide 0.4 hydrate.

Starting compounds: 7-amino-3-(3-pyridyl)-1H,3H-pyrrolo[1,2-c]thiazole and 2,5-dimethoxybenzoic acid.

| Elemental analysis (for $C_{20}H_{19}N_3O_3S \cdot 0.4H_2O$) | | | | |
|---|---|---|---|---|
| | C (%) | H (%) | N (%) | S (%) |
| Calcd.: | 61.81 | 5.13 | 10.81 | 8.25 |
| Found: | 61.89 | 5.01 | 10.70 | 8.20 |

Mass analysis, m/z: 381 ($M^+$)

Example 18

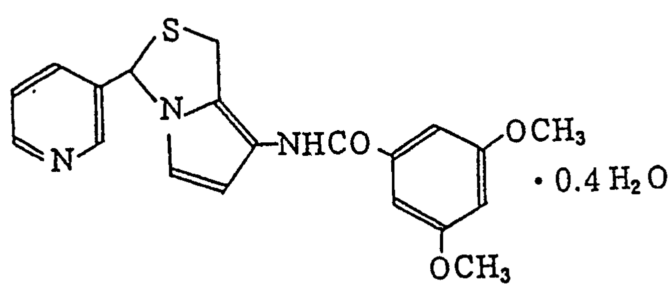

3,5-Dimethoxy-N-[3-(3-pyridyl)-1H,3H-pyrrolo[1,2-c]thiazol-7-yl]benzamide 0.4 hydrate.

Starting compounds: 7-amino-3-(3-pyridyl)-1H,3H-pyrrolo[1,2-c]thiazole and 3,5-dimethoxybenzoic acid.

Melting point: 163°C

| Elemental analysis (for $C_{20}H_{19}N_3O_3S$) | | | | |
|---|---|---|---|---|
| | C (%) | H (%) | N (%) | S (%) |
| Calcd.: | 62.97 | 5.02 | 11.02 | 8.41 |
| Found: | 62.69 | 4.92 | 10.84 | 8.36 |

Example 19

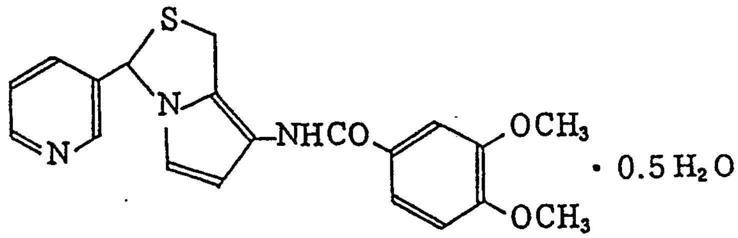

3,4-Dimethoxy-N-[3-(3-pyridyl)-1H,3H-pyrrolo[1,2-c]thiazol-7-yl]benzamide hemihydrate.
Starting compounds: 7-amino-3-(3-pyridyl)-1H,3H-pyrrolo[1,2-c]thiazole and 3,4-dimethoxybenzoic acid.

| Elemental analysis (for $C_{20}H_{19}N_3O_3S.\cdot 0.5H_2O$) | |
|---|---|
| | S (%) |
| Calcd.: | 8.21 |
| Found: | 8.26 |

Mass analysis, m/z: 381 ($M^+$)

Example 20

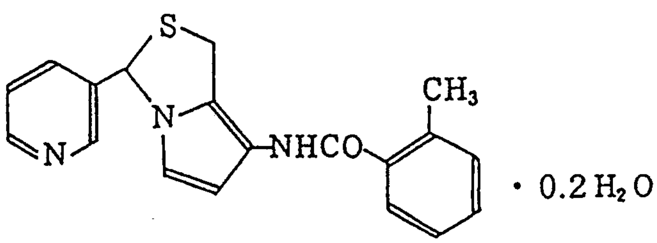

o-Methyl-N-[3-(3-pyridyl)-1H,3H-pyrrolo[1,2-c]thiazol-7-yl]benzamide 0.2 hydrate.
Starting compounds: 7-amino-3-(3-pyridyl)-1H,3H-pyrrolo[1,2-c]thiazole and o-toluic acid.
Melting point: 168-171 °C

| Elemental analysis (for $C_{19}H_{17}N_3OS\cdot 0.2H_2O$) | | | | |
|---|---|---|---|---|
| | C (%) | H (%) | N (%) | S (%) |
| Calcd.: | 67.31 | 5.17 | 12.39 | 9.46 |
| Found: | 67.33 | 5.03 | 12.30 | 9.59 |

Example 21

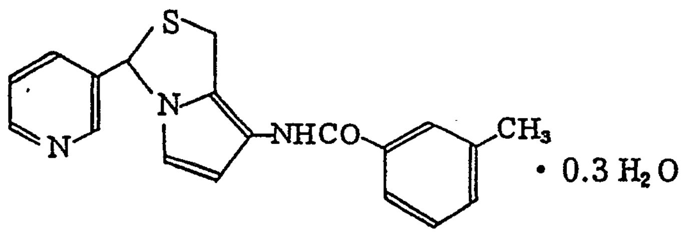

m-Methyl-N-[3-(3-pyridyl)-1H,3H-pyrrolo[1,2-c]thiazol-7-yl]benzamide 0.3 hydrate.
Starting compounds: 7-amino-3-(3-pyridyl)-1H,3H-pyrrolo[1,2-c]thiazole and m-toluic acid.
Melting point: 102-106 °C

| Elemental analysis (for $C_{19}H_{17}N_3OS \cdot 0.3H_2O$) | | | | |
|---|---|---|---|---|
| | C (%) | H (%) | N (%) | S (%) |
| Calcd.: | 66.96 | 5.20 | 12.33 | 9.41 |
| Found: | 66.79 | 5.08 | 12.17 | 9.42 |

Example 22

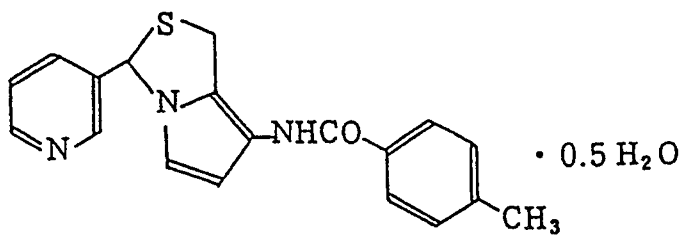

p-Methyl-N-[3-(3-pyridyl)-1H,3H-pyrrolo[1,2-c]thiazol-7-yl]benzamide hemihydrate.
Starting compounds: 7-amino-3-(3-pyridyl)-1H,3H-pyrrolo[1,2-c]thiazole and p-toluic acid.
Melting point: 152-154 °C

| Elemental analysis (for $C_{19}H_{17}N_3OS \cdot 0.5H_2O$) | |
|---|---|
| | S (%) |
| Calcd.: | 9.31 |
| Found: | 9.11 |

Example 23

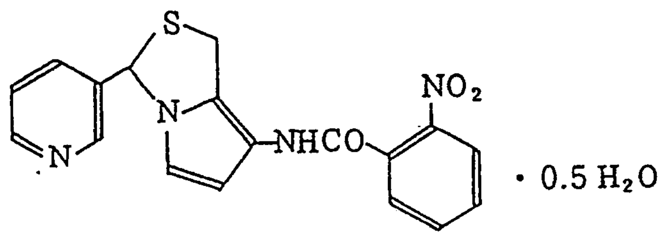

o Nitro-N-[3-(3-pyridyl)-1H,3H-pyrrolo[1,2-c]thiazol-7-yl]benzamide hemihydrate.

Starting compounds: 7-amino-3-(3-pyridyl)-1H,3H-pyrrolo[1,2-c]thiazole and o-nitrobenzoic acid.
Melting point: 198-201 ° C

| Elemental analysis (for $C_{18}H_{14}N_4O_3S \cdot 0.5H_2O$) | | | | |
|---|---|---|---|---|
| | C (%) | H (%) | N (%) | S (%) |
| Calcd.: | 57.58 | 4.03 | 14.93 | 8.54 |
| Found: | 57.76 | 3.84 | 14.69 | 8.38 |

## Example 24

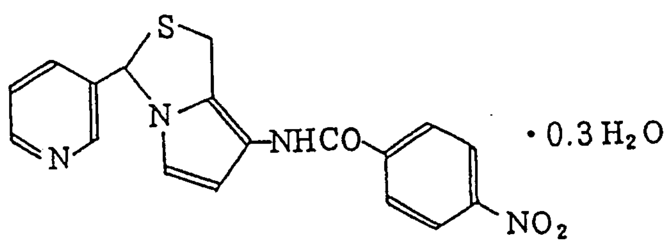

p Nitro-N-[3-(3-pyridyl)-1H,3H-pyrrolo[1,2-c]thiazole-7-yl]benzamide 0.3 hydrate.

Starting compounds: 7-amino-3-(3-pyridyl)-1H,3H-pyrrolo[1,2-c]thiazole and p-nitrobenzoic acid
Melting point: 202-206 ° C

| Elemental analysis (for $C_{18}H_{14}N_4O_3S \cdot 0.3H_2O$) | | | | |
|---|---|---|---|---|
| | C (%) | H (%) | N (%) | S (%) |
| Calcd.: | 58.15 | 3.96 | 15.07 | 8.62 |
| Found: | 58.12 | 3.81 | 14.92 | 8.63 |

## Example 25

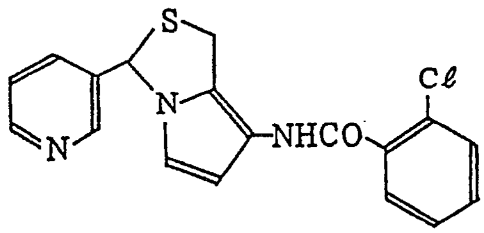

o-Chloro-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and o-chlorobenzoyl chloride.

Melting point: 161-166 °C

| Elemental analysis (for $C_{18}H_{14}N_3OSCl$) | | | | | |
|---|---|---|---|---|---|
| | C (%) | H (%) | N (%) | S (%) | Cl (%) |
| Calcd.: | 60.76 | 3.97 | 11.81 | 9.01 | 9.96 |
| Found: | 60.57 | 3.92 | 11.69 | 9.02 | 10.07 |

Example 26

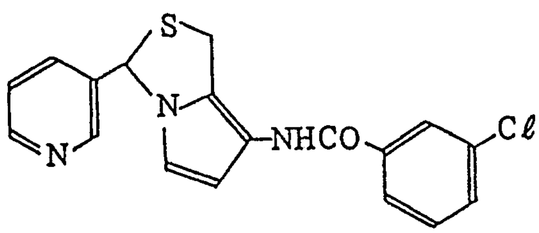

m-Chloro-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and m-chlorobenzoyl chloride.

Melting point: 164-166 °C

| Elemental analysis (for $C_{18}H_{14}N_3OSCl$) | | | | | |
|---|---|---|---|---|---|
| | C (%) | H (%) | N (%) | S (%) | Cl (%) |
| Calcd.: | 60.76 | 3.97 | 11.81 | 9.01 | 9.96 |
| Found: | 60.71 | 4.04 | 11.69 | 8.83 | 10.04 |

Example 27

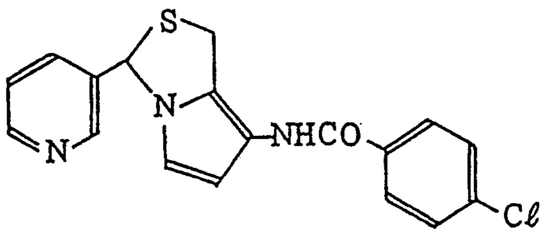

p-Chloro-N-[3-(3-pyridyl)-1H,3H-pyrrolo[1,2-c]thiazol-7-yl]benzamide.

Starting compounds: 7-amino-3-(3-pyridyl]-1H,3H-pyrrolo[1,2-c]thiazole and p-chlorobenzoic acid.

Melting point: 196-198 °C

| Elemental analysis for ($C_{18}H_{14}N_3OSCl$) | | | | | |
|---|---|---|---|---|---|
| | C (%) | H (%) | N (%) | S (%) | Cl (%) |
| Calcd: | 60.76 | 3.97 | 11.81 | 9.01 | 9.96 |
| Found: | 60.60 | 3.96 | 11.65 | 8.93 | 10.05 |

## Example 28

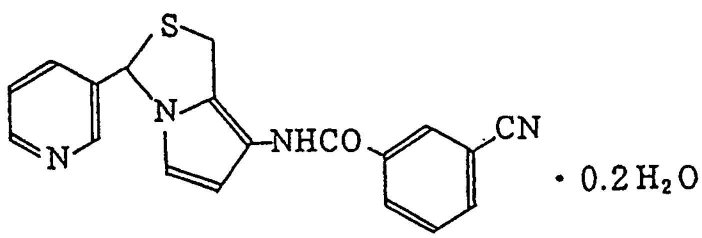

m-Cyano-N-[3-(3-pyridyl)-1H,3H-pyrrolo[1,2-c]thiazol-7-yl]benzamide 0.2 hydrate.

Starting compounds: 7-amino-3-(3-pyridyl)-1H,3H-pyrrolo[1,2-c]thiazole and m-cyanobenzoic acid.

Melting point: 188-193 °C

| Elemental analysis (for $C_{19}H_{14}N_4OS \cdot 0.2H_2O$) | | | | |
|---|---|---|---|---|
| | C (%) | H (%) | N (%) | S (%) |
| Calcd.: | 65.20 | 4.15 | 16.01 | 9.16 |
| Found: | 65.22 | 4.09 | 15.89 | 9.29 |

## Example 29

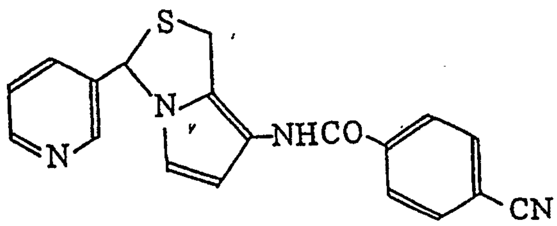

p Cyano-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and p-cyanobenzoic acid.

Melting point: 191-195 °C

Mass analysis, m/z: 346 ($M^+$)

## Example 30

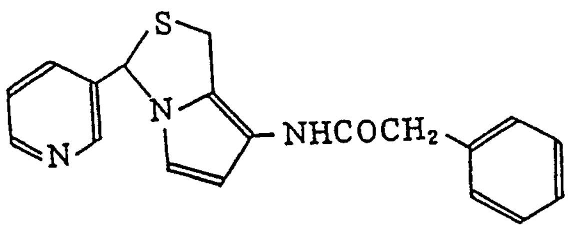

$\cdot$ 0.7 $H_2O$

7-(Phenylacetyl)amino-3-(3-pyridyl)-1H,3H-pyrrolo[1,2-c]thiazole 0.7 hydrate.

Starting compounds: 7-amino-3-(3-pyridyl)-1H,3H-pyrrolo[1,2-c]thiazole and phenylacetyl chloride.

Melting point: 105-108 °C

| Elemental analysis (for $C_{19}H_{17}N_3OS \cdot 0.7H_2O$) | | | | |
|---|---|---|---|---|
| | C (%) | H (%) | N (%) | S (%) |
| Clacd.: | 65.56 | 5.34 | 12.08 | 9.21 |
| Found: | 65.60 | 5.02 | 11.84 | 8.95 |

## Example 31

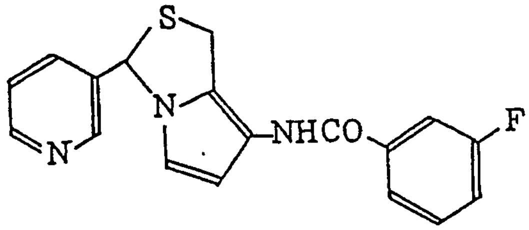

m-Fluoro-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and m-fluorobenzoic acid.

Melting point: 165-168°C

| Elemental analysis (for $C_{18}H_{14}N_3OSF$) | | | | | |
|---|---|---|---|---|---|
| | C (%) | H (%) | N (%) | S (%) | F (%) |
| Calcd.: | 63.70 | 4.16 | 12.38 | 9.45 | 5.60 |
| Found: | 63.52 | 4.14 | 12.28 | 9.64 | 5.57 |

Example 32

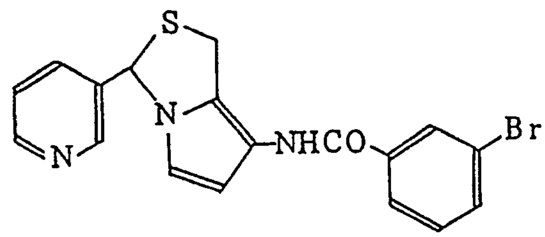

m-Bromo-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and m-bromobenzoic acid.

Melting point: 170-173°C

| Elemental analysis (for $C_{18}H_{14}N_3OSBr$) | | | | | |
|---|---|---|---|---|---|
| | C (%) | H (%) | N (%) | S (%) | Br (%) |
| Calcd.: | 54.01 | 3.53 | 10.50 | 8.01 | 19.96 |
| Found: | 53.96 | 3.50 | 10.43 | 7.94 | 20.13 |

Example 33

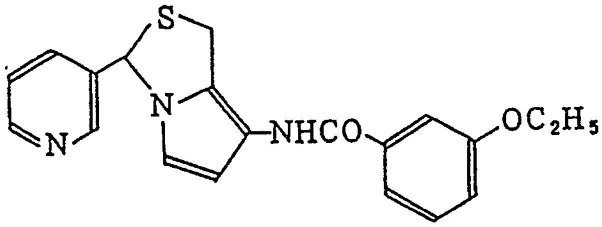

m-Ethoxy-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and m-ethoxybenzoic acid.

Melting point: 145-147°C

| Elemental analysis (for $C_{20}H_{19}N_3O_2S$) | | | | |
|---|---|---|---|---|
| | C (%) | H (%) | N (%) | S (%) |
| Calcd.: | 65.73 | 5.24 | 11.56 | 8.77 |
| Found: | 65.75 | 5.28 | 11.36 | 8.64 |

Example 34

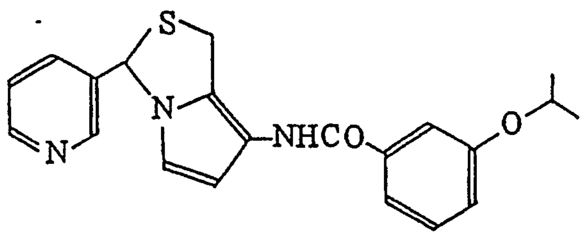

m-Isopropyloxy-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and m-isopropoxybenzoic acid.

Mass analysis, m/z: 379 ($M^+$)

NMR spectrum ($CDCl_3$, internal standard: TMS)

δ: 1.35 (6H, d), 4.38 (2H, s), 4.63 (1H, quint.), 6.21 (1H, d), 6.25 (1H, d), 6.34 (1H, s), 6.9-7.7 (6H, m), 7.78 (1H, s), 8.57 (2H, br)

Example 35

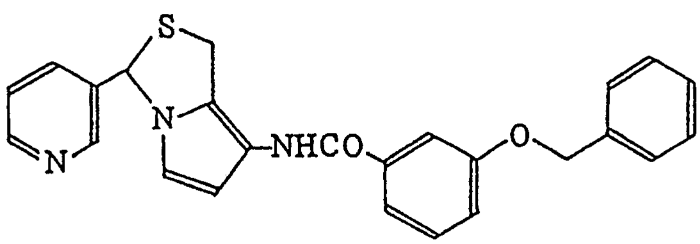

m-Benzyloxy-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and m-benzyloxybenzoic acid.

Melting point: 114-117 °C

| Elemental analysis (for $C_{25}H_{21}N_3O_2S$) | | | | |
|---|---|---|---|---|
| | C (%) | H (%) | N (%) | S (%) |
| Calcd.: | 70.24 | 4.95 | 9.83 | 7.50 |
| Found: | 70.06 | 4.91 | 9.65 | 7.61 |

Example 36

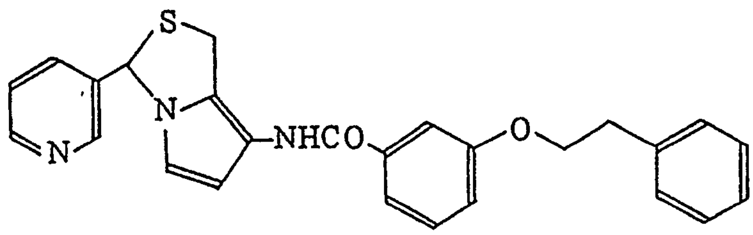

m-(2-Phenethyloxy)-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and m-phenethyloxybenzoic acid.

Melting point: 130-133 °C

| Elemental analysis (for $C_{26}H_{23}N_3O_2S$) | | | | |
|---|---|---|---|---|
| | C (%) | H (%) | N (%) | S (%) |
| Calcd.: | 70.72 | 5.25 | 9.52 | 7.26 |
| Found: | 70.76 | 5.28 | 9.37 | 7.26 |

Example 37

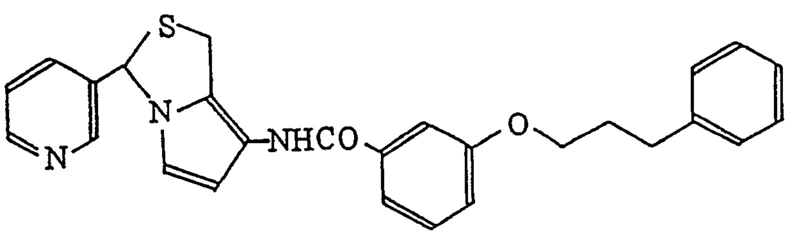

m-(3-Phenylpropyloxy)-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and m-(3-phenylpropyloxy)-benzoic acid.

Melting point: 128-131 °C

| Elemental analysis (for $C_{27}H_{25}N_3O_2S$) | | | | |
|---|---|---|---|---|
| | C (%) | H (%) | N (%) | S (%) |
| Calcd.: | 71.18 | 5.53 | 9.22 | 7.04 |
| Found: | 71.12 | 5.52 | 9.03 | 7.01 |

Example 38

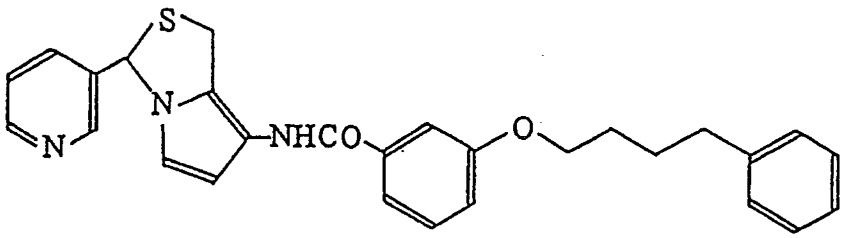

m-(4-Phenylbutyloxy)-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole and m-(4-phenylbutyloxy)-benzoic acid.

Melting point: 137-140 °C

| Elemental analysis (for $C_{28}H_{27}N_3O_2S$) | | | | |
|---|---|---|---|---|
| | C (%) | H (%) | N (%) | S (%) |
| Calcd.: | 71.61 | 5.80 | 8.95 | 6.83 |
| Found: | 71.59 | 5.75 | 8.86 | 6.80 |

Example 39

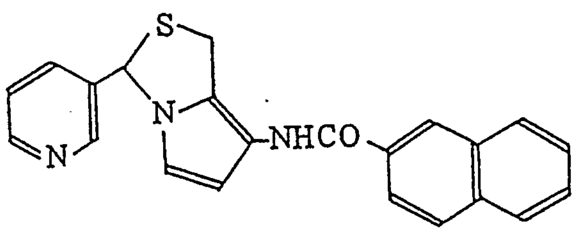

N-[3-(3-Pyridyl)-1H,3H-pyrrolo[1,2-c]thiazol-7-yl]naphthoamide.

Starting compounds: 7-amino-3-(3-pyridyl)-1H,3H-pyrrolo[1,2-c]thiazole and 2-naphthoic acid.

Melting point: 211-213 °C

Mass analysis, m/z: 371 ($M^+$)

Example 40

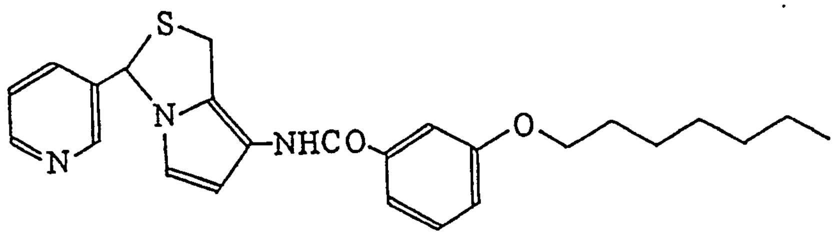

The procedure of Example 14 was followed using 280 mg of m-heptyloxybenzoic acid and 333 mg of 7-amino-3-(3-pyridyl)-1H,3H-pyrrolo[1,2-c]thiazole fumarate as starting compounds, together with 0.6 ml of

triethylamine, to give 170 mg of m-heptyloxy-N-[3-(3-pyridyl)-1H,3H-pyrrolo[1,2-c]thiazol-7-yl]benzamide.
Melting point: 111-114° C

| Elemental analysis (for $C_{25}H_{29}N_3O_2S$) | | | | |
|---|---|---|---|---|
| | C (%) | H (%) | N (%) | S (%) |
| Calcd.: | 68.94 | 6.71 | 9.65 | 7.36 |
| Found: | 68.93 | 6.72 | 9.57 | 7.29 |

## Example 41

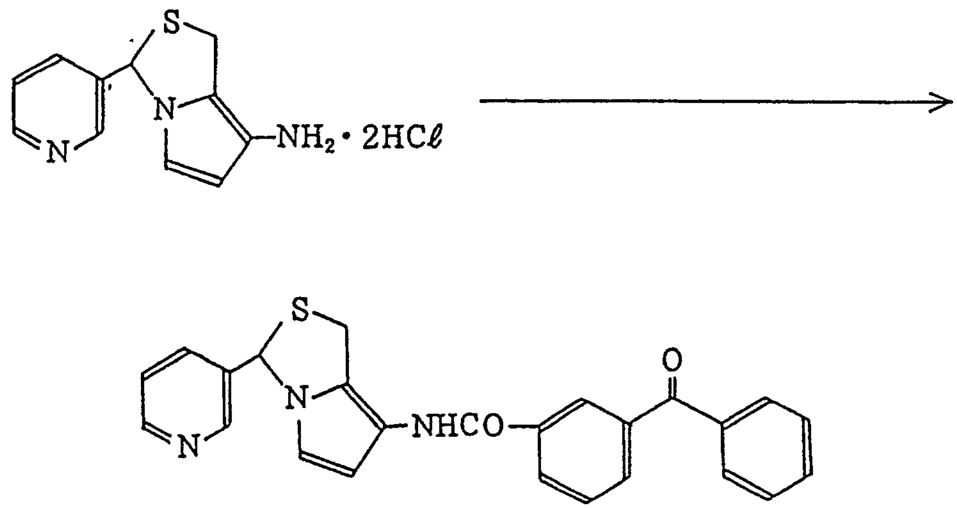

One drop of N,N-dimethylformamide was added to a mixture of 600 mg of m-benzoylbenzoic acid and 10 ml of methylene chloride and then 1 ml of oxalyl chloride was added with ice cooling. The resultant mixture was stirred at room temperature for 2 hours. The solvent and excess oxalyl chloride were then distilled off under reduced pressure. The thus-obtained mixture of m-benzoylbenzoyl chloride and methylene chloride was added dropwise to a mixture of 500 mg of 7-amino-3-(3-pyridyl)-1H,3H-pyrrolo[1,2-c]thiazole dihydrochloride, 1 ml of triethylamine and 10 ml of methylene chloride with ice cooling. The resultant mixture was stirred overnight at room temperature. Ethyl acetate (100 ml) was added to the reaction mixture and the whole mixture was washed with dilute aqueous sodium hydrogen carbonate solution and with water, dried over anhydrous magnesium sulfate and concentrated under reduced pressure. The residue was subjected to silica gel column chromatography (with 15 g of silica gel). Elution with toluene-ethyl acetate (2:1) gave 330 mg of m-benzoyl-N-[3-(3-pyridyl)-1H,3H-pyrrolo[1,2- c]thiazol-7-yl]-benzamide, which was further purified by recrystallization from ethanol.
Melting point: 98-100° C

| Elemental analysis (for $C_{25}H_{19}N_3O_2S$) | |
|---|---|
| | S (%) |
| Calcd.: | 7.54 |
| Found: | 7.26 |

The compounds of Examples 42 to 44 were produced in the same manner as mentioned in above Example 41.

Example 42

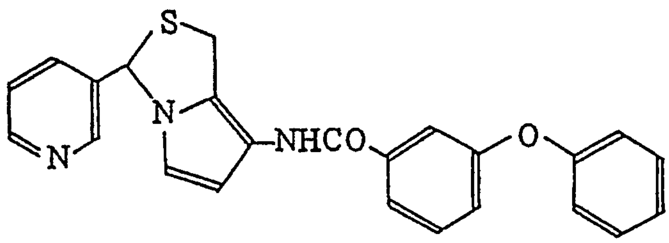

m-Phenoxy-N-[3-(3-pyridyl)-1H,3H-pyrrolo[1,2-c]thiazol-7-yl]benzamide.

Starting compounds: 7-amino-3-(3-pyridyl)-1H,3H-pyrrolo[1,2-c]thiazole dihydrochloride and m-phenoxybenzoic acid.

Melting point: 129-131 ° C

| Elemental analysis (for $C_{24}H_{19}N_3O_2S$) | |
|---|---|
| | S (%) |
| Calcd.: | 7.75 |
| Found: | 7.77 |

Example 43

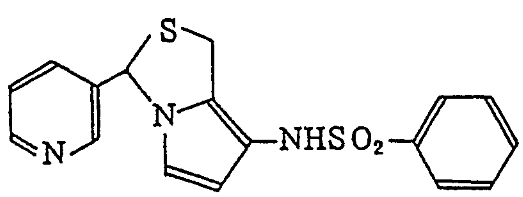

N-[3-(3-Pyridyl)-1H,3H-pyrrolo[1,2-c]thiazol-7-yl]benzenesulfonamide.

Starting compounds: 7-amino-3-(3-pyridyl)-1H,3H-pyrrolo[1,2-c]thiazole dihydrochloride and benzenesulfonyl chloride.

Mass analysis, m/z: 357 ($M^+$)

NMR spectrum ($CDCl_3$, internal standard: TMS)

δ: 3.98 (2H, s), 5.82 (1H, d), 6.12 (1H, d), 6.28 (1H, s), 7.1-7.8 (7H, m), 8.44 (1H, dd), 8.59 (1H, dd)

Example 44

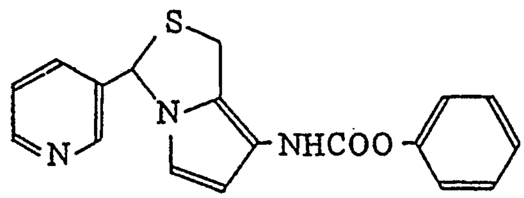

Phenyl N-[3-(3-pyridyl)-1H,3H-pyrrolo[1,2-c]thiazol-7-yl]carbamate.

Starting compounds: 7-amino-3-(3-pyridyl)-1H,3H-pyrrolo[1,2-c]thiazole dihydrochloride and phenyl chlorocarbonate.

Mass analysis, m/z: 337 ($M^+$)

NMR spectrum ($CDCl_3$, internal standard: TMS)

δ: 4.29 (2H, s), 6.19 (1H, d), 6.23 (1H, d), 6.32 (1H, s), 7.1-7.6 (7H, m), 8.53-8.63 (2H,m)

## Example 45

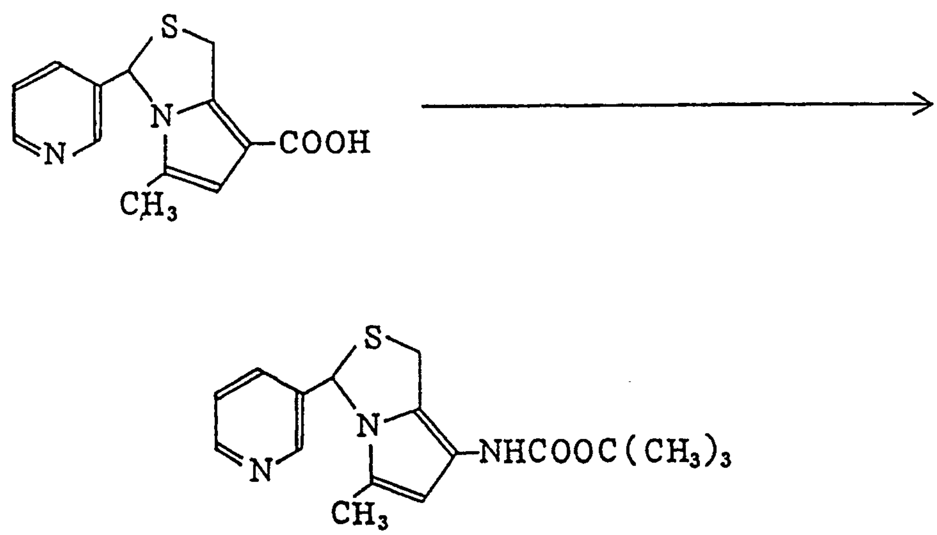

Triethylamine (2.56 g) and 6.97 g of diphenylphosphoryl azide were added in that order to a mixture of 5.99 g of 5-methyl-3-(3-pyridyl)-1H,3H-pyrrolo[1,2-c]-7-thiazolecarboxylic acid and 50 ml of tert-butanol with ice cooling. The resultant mixture was stirred at 80°C for 16 hours. The reaction mixture was concentrated under reduced pressure, 300 ml of ethyl acetate was added to the concentrate, and the mixture was washed with dilute aqueous sodium hydrogen carbonate and with water, dried over anhydrous magnesium sulfate and concentrated under reduced pressure. The residue was subjected to silica gel column chromatography (with 75 g of silica gel). Elution with toluene-ethyl acetate (3:1) gave 2 g of 7-tert-butoxycarbonylamino-5-methyl-3-(3-pyridyl)-1H,3H-pyrrolo[1,2-c]thiazole.

Melting point: 158-160°C

| Elemental analysis (for $C_{17}H_{21}N_3O_2S$) | | | | |
|---|---|---|---|---|
| | C (%) | H (%) | N (%) | S (%) |
| Calcd.: | 61.61 | 6.39 | 12.68 | 9.67 |
| Found: | 62.03 | 6.27 | 12.30 | 9.51 |

## Example 46

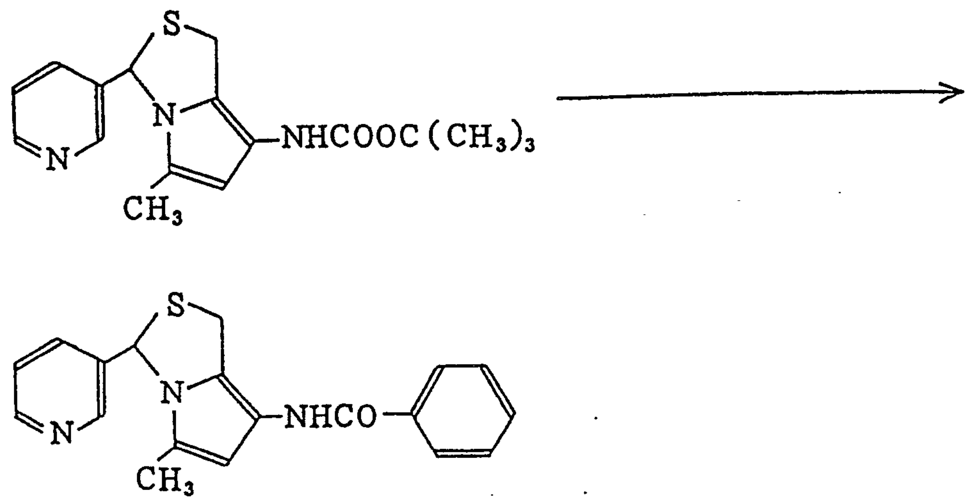

Trifluoroacetic acid (2 ml) was added to 200 mg of 7-tert-butoxycarbonylamino-5-methyl-3-(3-pyridyl)-1H,3H-pyrrolo[1,2-c]thiazole with ice cooling and the mixture was stirred at room temperature for 1 hour. The reaction mixture was concentrated under reduced pressure, 10 ml of methylene chloride was added to the residue, then 200 mg of triethylamine was added with ice cooling and 130 mg of benzoyl chloride was further added. The mixture was stirred at room temperature for 1 hour. The reaction mixture was concentrated under reduced pressure, 50 ml of ethyl acetate was added to the residue, and the resultant solution was washed with dilute aqueous sodium hydrogen carbonate solution, dried over anhydrous magnesium sulfate and concentrated under reduced pressure. The residue was subjected to silica gel column chromatography (with 20 g of silica gel). Elution with toluene-ethyl acetate (3:1) gave 150 mg of 7-benzoylamino-5-methyl-3-(3-pyridyl)-1H,3H-pyrrolo[1,2-c]thiazole. This product was further purified by recrystallization from 2-propanol.
Melting point: 195-197° C

| Elemental analysis (for $C_{19}H_{17}N_3OS$) | | | | |
|---|---|---|---|---|
| | C (%) | H (%) | N (%) | S (%) |
| Calcd.: | 68.03 | 5.11 | 12.53 | 9.56 |
| Found: | 67.57 | 4.90 | 12.48 | 9.62 |

## Example 47

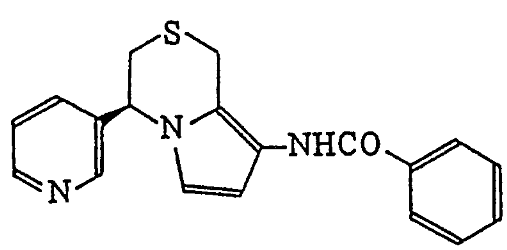

A mixture of 1.1 g of (4S)-3,4-dihydro-4-(3-pyridyl)-1H-pyrrolo[1,2-c][1,4]thiazine-8-carboxylic acid, 9 ml of N,N-dimethylformamide, 1 ml of diphenylphosphoryl azide and 0.6 ml of triethylamine was stirred at room temperature for 1 hour, then 9 ml of tert-butanol was added, and the resultant mixture was stirred at 100 °C for 2 hours. The mixture was cooled to room temperature, 100 ml of water was added, and the resultant mixture was extracted with ethyl acetate. The organic layer was washed with water and saturated aqueous sodium chloride solution and dried over anhydrous sodium sulfate. The solvent was then distilled off and the residue was subjected to silica gel column chromatography. Elution with chloroform-methanol (300:1) gave 310 mg of a light-yellow oil. Trifluoroacetic acid was added to the oil, the mixture was stirred at room temperature for 2 hours, then 30 ml of methylene chloride was added, and the whole mixture was poured in to 50 ml of saturated aqueous sodium hydrogen carbonate solution. The resultant mixture was extracted with methylene chloride, the extract was dried over anhydrous sodium sulfate, and the solvent was distilled off. The residue was dissolved in 2 ml of pyridine, 0.12 ml of benzoyl chloride was added with ice cooling, and the mixture was stirred at room temperature for 2 hours. Water (50 ml) was added to the reaction mixture, and the whole mixture was extracted with methylene chloride. The extract was dried over anhydrous sodium sulfate and then the solvent was distilled off. The residue was subjected to silica gel column chromatography. Elution with methylene chloride-ethyl acetate (3:1) gave 190 mg of (4S)-8-benzoylamino-3,4-dihydro-4-(3-pyridyl)-1H-pyrrolo[1,2-c][1,4]thiazine.
Mass analysis, m/z: 335 ($M^+$)
NMR spectrum ($CDCl_3$, internal standard: TMS)
δ: 3.20 (2H, ddd), 3.85 (2H, s), 5.39 (1H, dd), 6.20 (1H, d), 6.27 (1H, d), 7.1-8.1 (8H, m), 8.4-8.8 (2H, m)

Example 48

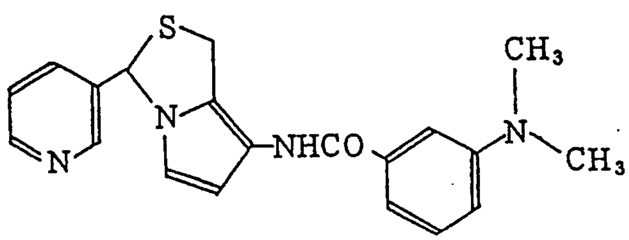

Dicyclohexylcarbodiimide (370 mg) was added to a mixture of 600 mg of m-dimethylaminobenzoic acid and 3 ml of methylene chloride and the resultant mixture was stirred at room temperature for 2 hours. Ethyl acetate was added to the reaction mixture, the insoluble matter was filtered off, and the solvent was distilled off. A solution of the thus-obtained m-dimethylaminobenzoic anhydride in 3 ml of methylene chloride and 0.25 ml of triethylamine were added, with ice cooling, to a solution of 330 mg of 7-amino-3-(3-pyridyl)-1H,3H-pyrrolo[1,2-c]thiazole in 1 ml of methylene chloride, and the mixture was stirred overnight at room temperature. Saturated aqueous sodium hydrogen carbonate solution (60 ml) was added to the reaction mixture, the whole mixture was extracted with methylene chloride, the organic layer was dried over anhydrous sodium sulfate, and the solvent was distilled off. The residue was subjected to silica gel column chromatography. Elution with methylene chloride-ethyl acetate (2:1) followed by recrystallization from 2-propanol gave 320 mg of m-dimethylamino-N-[3-(3-pyridyl)-1H,3H-pyrrolo[1,2-c]thiazol-7-yl]benzamide.
Melting point: 173-176 °C

| Elemental analysis (for $C_{20}H_{20}N_4OS$) | | | | |
|---|---|---|---|---|
| | C (%) | H (%) | N (%) | S (%) |
| Calcd.: | 65.91 | 5.53 | 15.37 | 8.80 |
| Found: | 65.91 | 5.58 | 15.22 | 8.76 |

Example 49

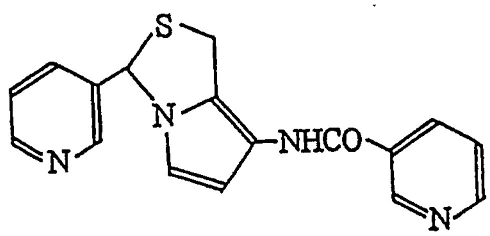

A mixture of 0.4 g of 7-tert-butoxycarbonylamino-4-(3-pyridyl)-1H,3H-pyrrolo[1,2-c]thiazole and 1.3 ml of trifluoroacetic acid was stirred at room temperature for 1 hour. The reaction mixture was poured into 30 ml of saturated aqueous sodium hydrogen carbonate solution, followed by extraction with three 10 ml portions of methylene chloride. The extract was dried over anhydrous sodium sulfate, the solvent was distilled off, and the residue was dissolved in 1 ml of methylene chloride. To the solution was added a mixture of 0.2 g of nicotinoyl chloride and 2 ml of methylene chloride, and 0.7 ml of triethylamine. The mixture was stirred at room temperature for 4 hours. Saturated aqueous sodium hydrogen carbonate solution (50 ml) was added to the reaction mixture, followed by extraction with two 20-ml portions of methylene chloride. The extract was dried over anhydrous sodium sulfate, the solvent was distilled off, and the residue was subjected to silica gel column chromatography. Elution with methylene chloride-ethyl acetate (2:1) followed by recrystallization from 2-propanol gave 0.16 g of N-[3-(3-pyridyl)-1H,3H-pyrrolo[1,2-c]thiazol-7-yl]nicotinamide.
Melting point: 181-183 °C

| Elemental analysis (for $C_{17}H_{14}N_4OS$) | | | | |
|---|---|---|---|---|
| | C (%) | H (%) | N (%) | S (%) |
| Calcd.: | 63.34 | 4.38 | 17.38 | 9.95 |
| Found: | 63.26 | 4.41 | 17.14 | 9.86 |

Example 50

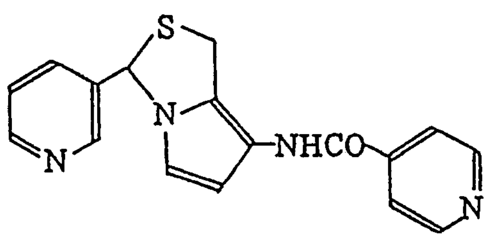

The procedure of Example 49 was followed using 7-tert-butoxycarbonylamino-3-(3-pyridyl)-1H,3H-pyrrolo[1,2-c]thiazole and isonicotinoyl chloride to give N-[3-(3-pyridyl)-1H,3H-pyrrolo[1,2-c]thiazol-7-yl]-isonicotinamide.
Mass analysis, m/z: 322 ($M^+$)
NMR spectrum ($CDCl_3$, internal standard: TMS)
δ: 4.32 (2H, s), 6.17 (1H, d), 6.23 (1H, d), 6.31 (1H, s), 7.29 (1H, dd), 7.4-7.8 (3H, m), 8.3-8.8 (5H, m)

Example 51

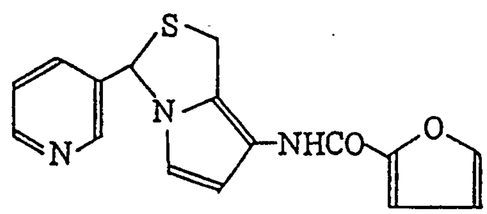

The procedure of Example 49 was followed starting with 7-tert-butoxycarbonylamino-3-(3-pyridyl)-1H,3H-pyrrolo[1,2- c]thiazole and furan-2-carbonyl chloride to give N-[3-(3-pyridyl)-1H,3H-pyrrolo[1,2-c]-thiazol-7-yl]furan-2-carboxamide.
Melting point: 175-179 ° C

| Elemental analysis (for $C_{16}H_{13}N_3O_2S$) | | | | |
|---|---|---|---|---|
| | C (%) | H (%) | N (%) | S (%) |
| Calcd.: | 61.72 | 4.21 | 13.50 | 10.30 |
| Found: | 61.75 | 4.31 | 13.59 | 10.21 |

Example 52

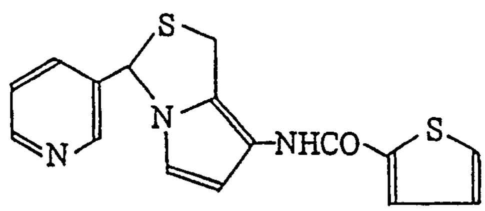

The procedure of Example 49 was followed starting with 7-tert-butoxycarbonylamino-3-(3-pyridyl)-1H,3H-pyrrolo[1,2-c]thiazole and thiophene-2-carbonyl chloride to give N-[3-(3-pyridyl)-1H,3H-pyrrolo[1,2-c]-thiazol-7-yl]thiophene-2-carboxamide.
Melting point: 150-153 ° C

| Elemental analysis (for $C_{16}H_{13}N_3OS$) | | | | |
|---|---|---|---|---|
| | C (%) | H (%) | N (%) | S (%) |
| Calcd.: | 58.69 | 4.00 | 12.83 | 19.59 |
| Found: | 58.45 | 3.98 | 12.61 | 19.56 |

Example 53

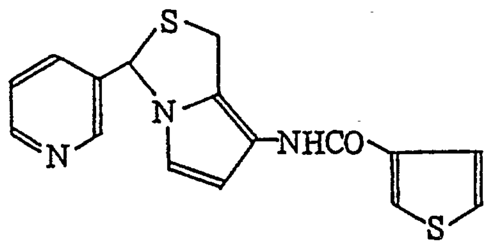

The procedure of Example 49 was followed starting with 7-tert-butoxycarbonylamino-3-(3-pyridyl)-1H,3H-pyrrolo[1,2-c]thiazole and thiophene-3-carbonyl chloride to give N-[3-(3-pyridyl)-1H,3H-pyrrolo[1,2-c]-thiazol-7-yl]thiophene-3-carboxamide.
Melting point: 172-174 °C

| Elemental analysis (for $C_{16}H_{13}N_3OS_2$) | | | | |
|---|---|---|---|---|
| | C (%) | H (%) | N (%) | S (%) |
| Calcd.: | 58.69 | 4.00 | 12.83 | 19.59 |
| Found: | 58.67 | 4.04 | 12.71 | 19.63 |

## Example 54

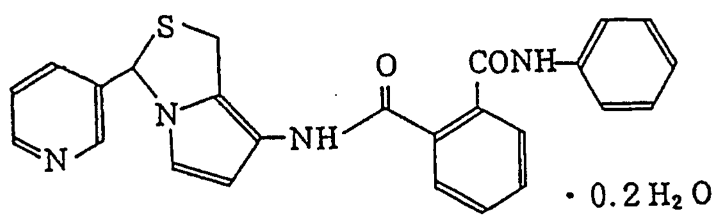

The procedure of Example 49 was followed starting with N-phenylphthalamoyl chloride and 7-tert-butoxycarbonylamino-3- (3-pyridyl)-1H,3H-pyrrolo[1,2-c]thiazole to give o-(N-phenylcarbamoyl)-N-[3-(3-pyridyl)-1H,3H-pyrrolo[1,2-c]thiazol-7-yl]benzamide 0.2 hydrate.
Melting point: 110 °C

| Elemental analysis (for $C_{25}H_{20}N_4O_2S_2 \cdot 0.2H_2O$) | | | | |
|---|---|---|---|---|
| | C (%) | H (%) | N (%) | S (%) |
| Calcd.: | 67.61 | 4.63 | 12.62 | 7.22 |
| Found: | 67.35 | 4.64 | 12.44 | 7.37 |

## Example 55

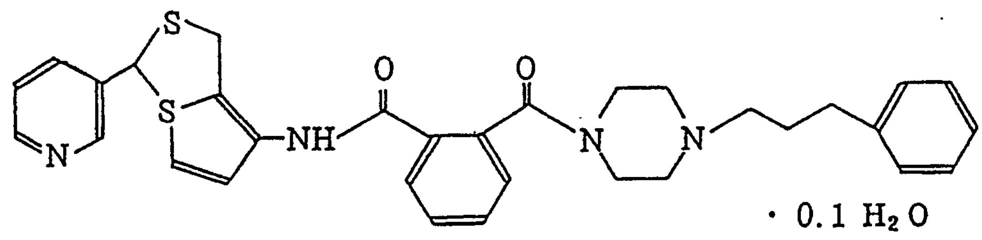

The procedure of Example 49 was followed starting with 1-(o-chlorocarbonylbenzoyl)-4-(3-phenylpropyl)piperazine and 7-tert-butoxycarbonylamino-3-(3-pyridyl)-1H,3H-pyrrolo[1,2-c]thiazole to give o-[[1-(3-phenylpropyl)piperazin-4-yl]carbonyl]-N-[3-(3-pyridyl)-1H,3H-pyrrolo[1,2-c]thiazol-7-yl]benzamide 0.1 hydrate.

| Elemental analysis (for $C_{32}H_{33}N_5O_2 \cdot 0.1H_2O$) | | | | |
|---|---|---|---|---|
| | C (%) | H (%) | N (%) | S (%) |
| Calcd.: | 69.44 | 6.05 | 12.65 | 5.79 |
| Found: | 69.11 | 6.06 | 12.57 | 5.84 |

NMR spectrum ($CDCl_3$, internal standard: TMS)
δ: 1.60-1.92 (2H, m), 2.10-2.68 (10H, m), 3.21 (2H, t), 3.62-3.96 (1H, br), 4.32 (2H,s), 6.18 (1H, d), 6.30 (1H, s), 6.38 (1H,d), 7.06-7.63 (10H, m), 7.77-7.95 (1H, m), 8.51-8.59 (2H, m)

Example 56

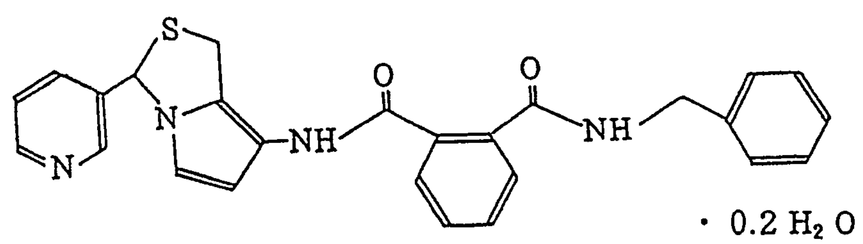

The procedure of Example 49 was followed starting with N-benzylphthalamoyl chloride and 7-tert-butoxycarbonylamino-[3-(3-pyridyl)-1H,3H-pyrrolo[1,2-c]thiazole to give o-(N-benzylcarbamoyl)-N-[3-(3-pyridyl)-1H,3H-pyrrolo[1,2-c]thiazol-7-yl]benzamide 0.2 hydrate.
Melting point: 88°C

| Elemental analysis (for $C_{26}H_{22}N_4O_2S \cdot 0.2H_2O$) | | | | |
|---|---|---|---|---|
| | C (%) | H (%) | N (%) | S (%) |
| Calcd.: | 68.16 | 4.93 | 12.23 | 7.00 |
| Found: | 68.10 | 4.96 | 12.01 | 6.86 |

Example 57

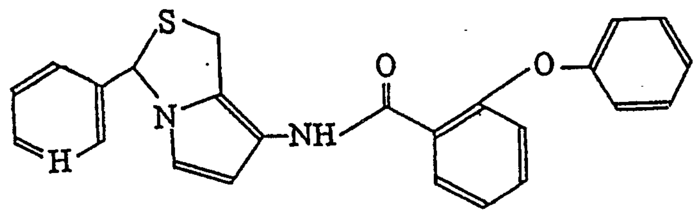

The procedure of Example 49 was followed starting with o-phenoxybenzoyl chloride and 7-tert-butoxycarbonylamino-3-(3-pyridyl)-1H,3H-pyrrolo[1,2-c]thiazole to give o-phenoxy-N-[3-(3-pyridyl)-1H,3H-pyrrolo[1,2-c]thiazol-7-yl]benzamide.

| Elemental analysis (for $C_{24}H_{19}N_3O_2S$) | | | | |
|---|---|---|---|---|
| | C (%) | H (%) | N (%) | S (%) |
| Calcd.: | 69.71 | 4.63 | 10.16 | 7.75 |
| Found: | 69.55 | 4.72 | 10.00 | 7.50 |

NMR spectrum ($CDCl_3$, internal standard: TMS)
δ: 4.30 (1H, s), 4.31 (1H, s), 6.17-6.32 (3H,m), 6.81-7.63 (10H, m), 8.28 (dd, 1H), 8.50-8.58 (2H, m)

## Example 58

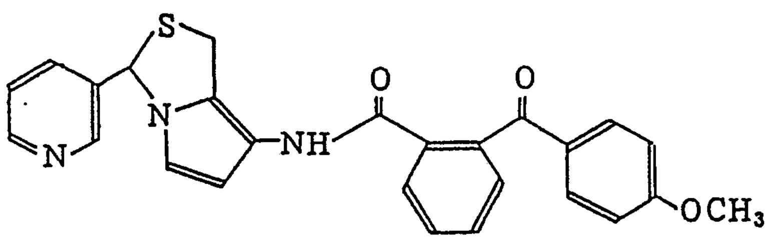

The procedure of Example 49 was followed starting with o-(p-methoxybenzoyl)benzoyl chloride and 7-tert-butoxycarbonylamino-3-(3-pyridyl)-1H,3H-pyrrolo[1,2-c]thiazole to give o-(p-methoxybenzoyl)-N-[3-(3-pyridyl)-1H,3H-pyrrolo[1,2-c]thiazol-7-yl]benzamide.
Mass analysis, (m/z): 456 ($M^+$)
NMR spectrum
δ: 3.73-3.88 (m, 5H), 6.00-6.40 (m, 3H), 6.90-7.93 (m, 10H), 8.52-8.69 (m, 2H)

## Example 59

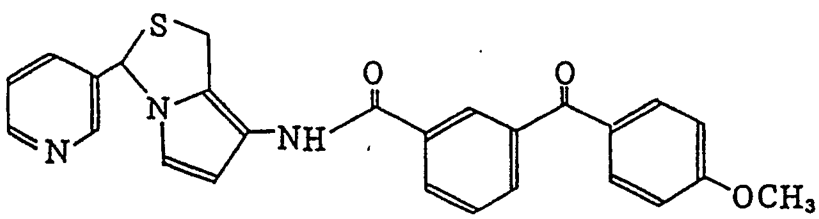

$\cdot$ 0.4 $H_2O$

The procedure of Example 49 was followed starting with m-(p-methoxybenzoyl)benzoyl chloride and 7-tert- butoxycarbonylamino-3-(3-pyridyl)-1H,3H-pyrrolo[1,2-c]thiazole to give m-(p-methoxybenzoyl)-N-[3-(3-pyridyl)-1H,3H-pyrrolo[1,2-c]thiazol-7-yl]benzamide 0.4 hydrate.
Melting point: 85 °C

| Elemental analysis (for $C_{26}H_{21}N_3O_3S \cdot 0.4H_2O$) | | | | |
|---|---|---|---|---|
| | C (%) | H (%) | N (%) | S (%) |
| Calcd.: | 67.49 | 4.75 | 9.08 | 6.93 |
| Found: | 67.67 | 5.04 | 8.70 | 6.84 |

## Example 60

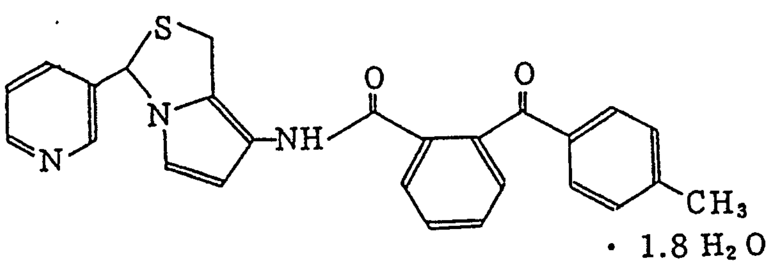

$\cdot$ 1.8 $H_2O$

The procedure of Example 49 was followed starting with o-(p-methylbenzoyl)benzoyl chloride and 7-tert-butoxycarbonylamino-3-(3-pyridyl)-1H,3H-pyrrolo[1,2-c]thiazole to give o-(p-methylbenzoyl)-N-[3-(3-pyridyl)-1H,3H-pyrrolo[1,2-c]thiazol-7-yl]benzamide 1.8 hydrate.
Melting point: 130 °C

| Elemental analysis (for $C_{26}H_{21}N_3O_2S \cdot 1.8H_2O$) | | | | |
|---|---|---|---|---|
| | C (%) | H (%) | N (%) | S (%) |
| Calcd.: | 64.00 | 5.08 | 8.61 | 6.57 |
| Found: | 63.72 | 4.70 | 8.25 | 6.33 |

## Example 61

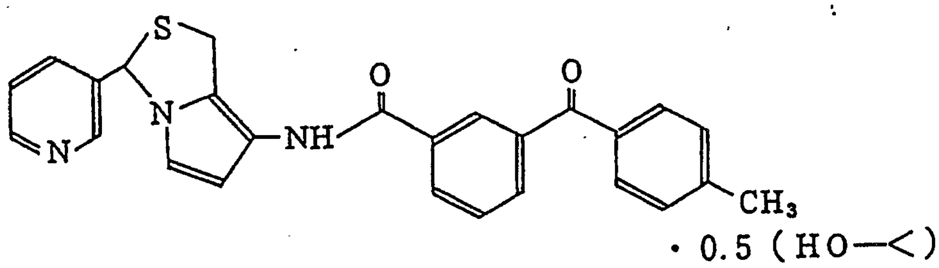

The procedure of Example 49 was followed starting with m-(p-methylbenzoyl)benzoyl chloride and 7-tert-butoxycarbonylamino-3-(3-pyridyl)-1H,3H-pyrrolo[1,2-c]thiazole to give m-(p-methylbenzoyl)-N-[3-(3-pyridyl)-1H,3H-pyrrolo[1,2-c]thiazol-7-yl]benzamide containing 0.5 molecule of 2-propanol.
Melting point: 90 °C

| Elemental analysis (for $C_{26}H_{21}N_3O_2S \cdot 0.5(C_3H_7OH)$) | | | | |
|---|---|---|---|---|
| | C (%) | H (%) | N (%) | S (%) |
| Calcd.: | 70.34 | 5.37 | 8.95 | 6.83 |
| Found: | 70.23 | 5.22 | 8.90 | 6.65 |

## Example 62

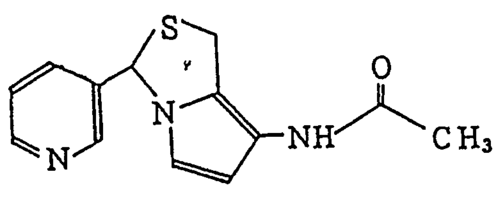

The procedure of Example 49 was followed starting with acetyl chloride and 7-tert-butoxycarbonylamino-3-(3-pyridyl)-1H,3H-pyrrolo[1,2-c]thiazole to give N-[3-(3-pyridyl)-1H,3H-pyrrolo[1,2-c]thiazol-7-yl]acetamide.
Melting point: 121-123 °C

| Elemental analysis (for $C_{13}H_{13}N_3OS$) | | | | |
|---|---|---|---|---|
| | C (%) | H (%) | N (%) | S (%) |
| Calcd.: | 60.21 | 5.05 | 16.20 | 12.36 |
| Found: | 60.01 | 5.18 | 15.95 | 12.12 |

## Example 63

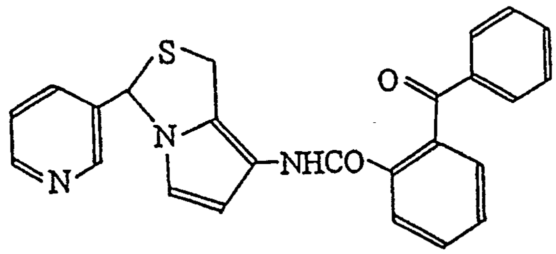

The procedure of Example 49 was followed starting with 7-tert-butoxycarbonylamino-3-(3-pyridyl)-1H,3H-pyrrolo[1,2-c]thiazole and o-benzoylbenzoyl chloride to give o-benzoyl-N-[3-(3-pyridyl)-1H,3H-pyrrolo[1,2-c]thiazol-7-yl]benzamide.

Mass analysis, m/z: 425 ($M^+$)

NMR spectrum ($CDCl_3$, internal standard: TMS)

δ: 3.70-4.26 (2H, m), 6.09-6.25 (2H, m), 7.14 (1H, s), 7.16-7.91 (13H, m)

## Example 64

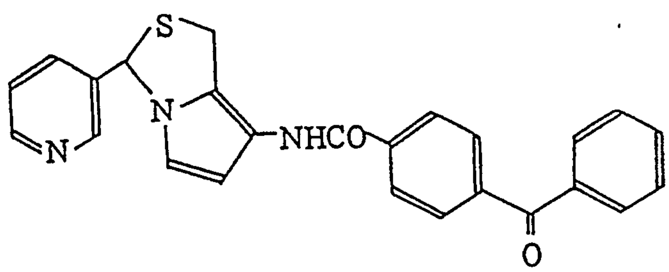

The procedure of Example 49 was followed starting with 7-tert-butoxycarbonylamino-3-(3-pyridyl)-1H,3H-pyrrolo[1,2-c]thiazole and p-benzoylbenzoyl chloride to give p-benzoyl-N-[3-(3-pyridyl)-1H,3H-pyrrolo[1,2-c]thiazol-7-yl]benzamide.

Melting point: 215-218° C

| Elemental analysis (for $C_{25}H_{19}N_3O_2S$) | | | | |
|---|---|---|---|---|
| | C (%) | H (%) | N (%) | S (%) |
| Calcd.: | 70.57 | 4.50 | 9.88 | 7.54 |
| Found: | 70.63 | 4.48 | 9.69 | 7.64 |

## Example 65

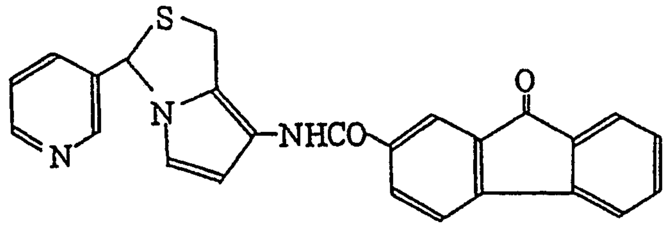

The procedure of Example 49 was followed starting with 7-tert-butoxycarbonylamino-3-(3-pyridyl)-1H,3H-pyrrolo[1,2- c]thiazole and 9-fluorenone-2-carbonyl chloride to give 9-oxo-N-[3-(3-pyridyl)-1H,3H-pyrrolo[1,2-c]thiazol-7-yl]fluorene-3-carboxamide.
Melting point: 222-224° C

| Elemental analysis (for $C_{25}H_{17}N_3O_2S$) | | | | |
|---|---|---|---|---|
| | C (%) | H (%) | N (%) | S (%) |
| Calcd.: | 70.90 | 4.05 | 9.92 | 7.57 |
| Found: | 70.96 | 4.07 | 9.86 | 7.68 |

Example 66

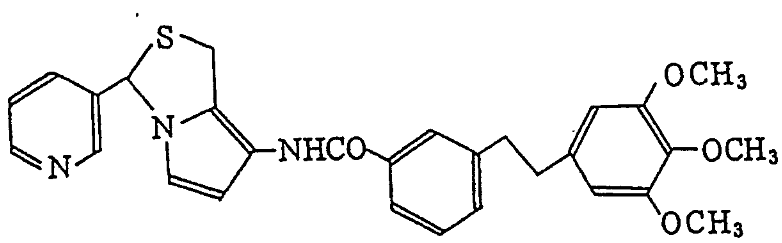

The procedure of Example 49 was followed starting with 7-tert-butoxycarbonylamino-3-(3-pyridyl)-1H,3H-pyrrolo[1,2-c]thiazole and m-[2-(3,4,5-trimethoxyphenyl)ethyl]benzoyl chloride to give m-[2-(3,4,5-trimethoxyphenyl)ethyl]-N-[3-(3-pyridyl)-1H,3H-pyrrolo[1,2-c]thiazol-7-yl]benzamide.
Mass analysis, m/z: 515 ($M^+$)
NMR spectrum ($CDCl_3$, internal standard: TMS)
δ: 2.8-3.1 (4H, m), 3.82 (9H, s), 4.40 (2H, dd), 6.23 (1H, d), 6.27 (1H, d), 6.39 (1H, s), 7.1-7.8 (8H, m), 8.5-8.7 (2H, m)

Example 67

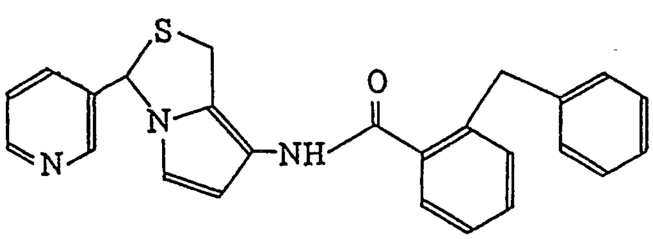

The procedure of Example 49 was followed starting with o-benzylbenzoyl chloride and 7-tert-butoxycarbonylamino-3-(3-pyridyl)-1H,3H-pyrrolo[1,2-c]thiazole to give o-benzyl-N-[3-(3-pyridyl)-1H,3H-pyrrolo[1,2-c]thiazol-7-yl]benzamide. Melting point: 148° C

| Elemental analysis (for $C_{25}H_{21}N_3OS$) | | | | |
|---|---|---|---|---|
| | C (%) | H (%) | N (%) | S (%) |
| Calcd.: | 72.97 | 5.14 | 10.21 | 7.79 |
| Found: | 72.94 | 5.16 | 10.13 | 7.73 |

Example 68

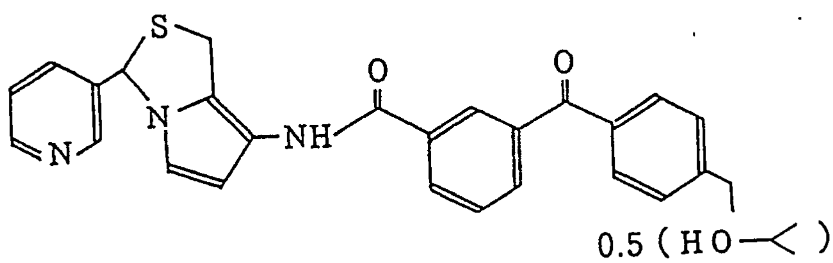

The procedure of Example 49 was followed starting with m-(p-ethylbenzoyl)benzoyl chloride and 7-tert-butoxycarbonylamino-3-(3-pyridyl)-1H,3H-pyrrolo[1,2-c]thiazole to give m-(p-ethylbenzoyl)-N-[3-(3-pyridyl)-1H,3H-pyrrolo[1,2-c]thiazol-7-yl]benzamide containing 0.5 molecule of 2-propanol.
Melting point: 87° C

| Elemental analysis (for $C_{27}H_{23}N_3O_2S \cdot 0.5C_3H_2OH$)) | | | | |
|---|---|---|---|---|
| | C (%) | H (%) | N (%) | S (%) |
| Calcd.: | 70.78 | 5.63 | 8.69 | 6.63 |
| Found: | 70.50 | 5.52 | 8.69 | 6.87 |

Example 69

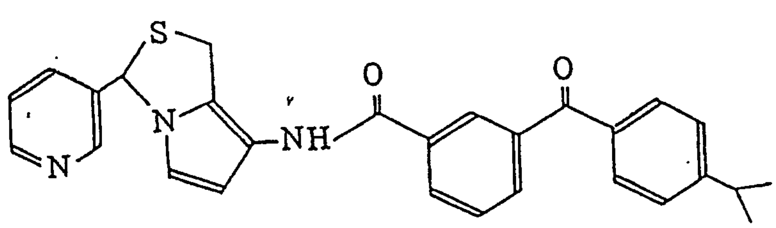

The procedure of Example 49 was followed starting with m-[p-(1-methylethyl)benzoyl]benzoyl chloride

and 7-tert-butoxycarbonylamino-3-(3-pyridyl)-1H,3H-pyrrolo[1,2-c]thiazole to give m-[p-(1-methylethyl)-benzoyl]-N-[3-(3-pyridyl)-1H,3H-pyrrolo[1,2-c]thiazol-7-yl]benzamide.
Melting point: 97° C

| Elemental analysis (for $C_{28}H_{25}N_3O_2S$) | | | | |
|---|---|---|---|---|
| | C (%) | H (%) | N (%) | S (%) |
| Calcd.: | 71.92 | 5.39 | 8.99 | 6.86 |
| Found: | 71.60 | 5.53 | 8.65 | 6.89 |

Example 70

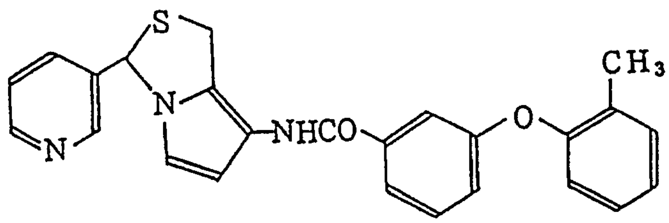

The procedure of Example 49 was followed starting with 7-tert-butoxycarbonylamino-3-(3-pyridyl)-1H,3H-pyrrolo[1,2-c]thiazole and m-(2-methylphenyloxy)benzoic acid to give m-(2-methylphenyloxy)-N-[3-(3-pyridyl)-1H,3H-pyrrolo [1,2-c]thiazol-7-yl]benzamide.

| Elemental analysis (for $C_{25}H_{21}N_3O_2S$) | |
|---|---|
| | S (%) |
| Calcd.: | 7.50 |
| Found: | 7.39 |

Mass analysis, m/z: 427 ($M^+$)

Example 71

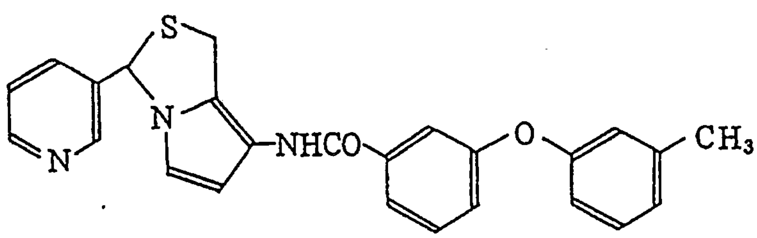

The procedure of Example 49 was followed starting with 7-tert-butoxycarbonylamino-3-(3-pyridyl)-1H,3H-pyrrolo[1,2-c]thiazole and m-(3-methylphenyloxy)benzoic acid to give m-(3-methylphenyloxy)-N-[3-(3-pyridyl)-1H,3H-pyrrolo[1,2-c]thiazol-7-yl]benzamide.

| Elemental analysis (for $C_{25}H_{21}N_3O_2S$) | |
|---|---|
| | S (%) |
| Calcd.: | 7.50 |
| Found: | 7.57 |

Mass analysis, m/z: 427 ($M^+$)

Example 72

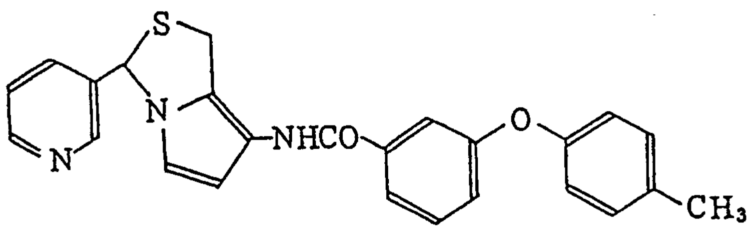

The procedure of Example 49 was followed starting with 7-tert-butoxycarbonylamino-3-(3-pyridyl)-1H,3H-pyrrolo[1,2-c]thiazole and m-(4-methylphenyloxy)benzoic acid to give m-(4-methylphenyloxy)-N-[3-(3-pyridyl)-1H,3H-pyrrolo[1,2-c]thiazol-7-yl]benzamide.
Melting point: 139-144° C

| Elemental analysis (for $C_{25}H_{21}N_3O_2S$) | | | | |
|---|---|---|---|---|
| | C (%) | H (%) | N (%) | S (%) |
| Calcd.: | 70.24 | 4.95 | 9.83 | 7.50 |
| Found: | 70.47 | 5.00 | 9.76 | 7.67 |

Example 73

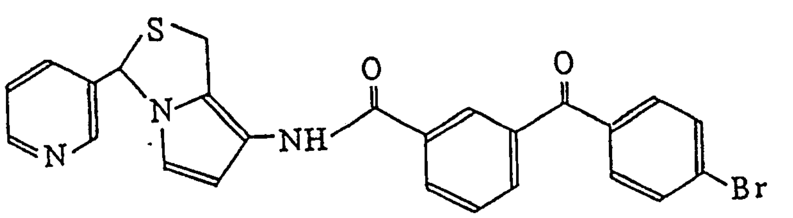

$\cdot 0.5\,(C_2H_5OC_2H_5)$

The procedure of Example 49 was followed starting with 7-tert-butoxycarbonylamino-3-(3-pyridyl)-1H,3H-pyrrolo[1,2-c]thiazole and m-(p-bromobenzoyl)benzoic acid to give m-(p-bromobenzoyl)-N-[3-(3-pyridyl)-1H,3H-pyrrolo[1,2-c]thiazol-7-yl]benzamide containing 0.5 molecule of diethyl ether.
Melting point: 101 °C

| Elemental analysis (for $C_{25}H_{18}N_3O_2S \cdot Br0.5(C_2H_5OC_2H_5)$) | | | | |
|---|---|---|---|---|
| | C (%) | H (%) | N (%) | S (%) |
| Calcd.: | 59.89 | 4.28 | 7.76 | 5.92 |
| Found: | 60.07 | 3.89 | 7.83 | 6.18 |

## Example 74

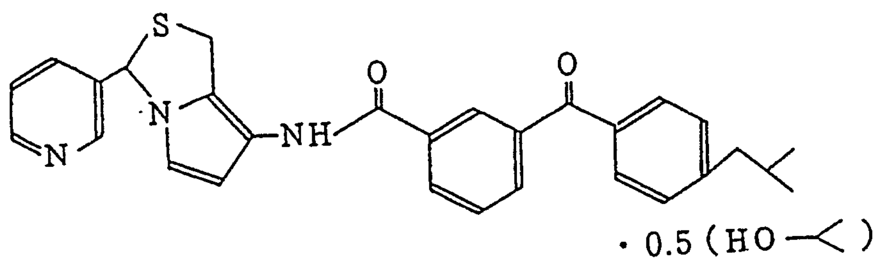

The procedure of Example 49 was followed starting with 7-tert-butoxycarbonylamino-3-(3-pyridyl)-1H,3H-pyrrolo[1,2-c]thiazole and m-[p-(2-methylpropyl)benzoyl]benzoic acid to give m-[p-(2-methylpropyl)-benzoyl]-N-[3-(3-pyridyl)-1H,3H-pyrrolo[1,2-c]thiazol-7-yl]benzamide containing 0.5 molecule of 2-propanol.
Melting point: 85 °C

| Elemental analysis (for $C_{29}H_{27}N_3O_2S \cdot 0.5(C_3H_7OH)$) | | | | |
|---|---|---|---|---|
| | C (%) | H (%) | N (%) | S (%) |
| Calcd: | 71.60 | 6.11 | 8.21 | 6.27 |
| Found: | 71.86 | 6.06 | 8.18 | 6.10 |

## Example 75

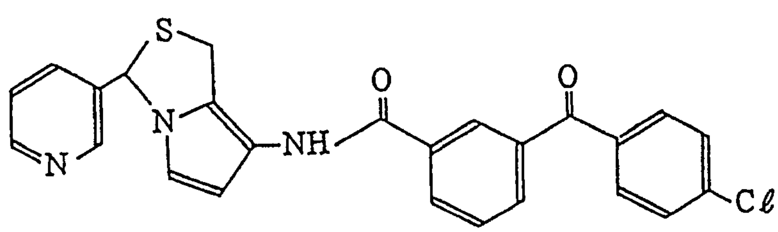

The procedure of Example 49 was followed starting with 7-tert-butoxycarbonylamino-3-(3-pyridyl)-1H,3H-pyrrolo[1,2-c]thiazole and m-(p-chlorobenzoyl)benzoic acid to give m-(p-chlorobenzoyl)-N-[3-(3-pyridyl)-1H,3H-pyrrolo[1,2c]thiazol-7-yl]benzamide.
Melting point: 100° C
NMR spectrum ($CDCl_3$, internal standard: TMS)
δ: 4.34-4.45 (2H, m), 6.24-6.29 (2H, m), 6.37 (1H, s), 7.31-7.36 (1H, m), 7.50 (2H, d), 7.60-7.67 (2H, m), 7.77 (2H, d), 7.93 (1H, d), 8.16 (1H, s), 8.27 (1H, s), 8.57-8.63 (2H, s)

## Example 76

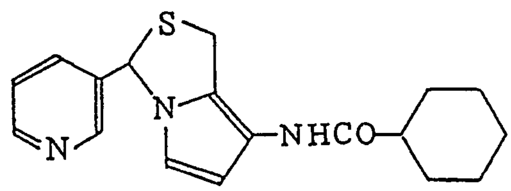

Dicyclohexylcarbodiimide (950 mg) was added to an ice-cooled solution of 600 mg of cyclohexanecarboxylic acid in 10 ml of methylene chloride and the mixture was stirred at room temperature for 2 hours. Ethyl acetate (20 ml) was added to the reaction mixture and the resultant crystals were filtered off. Separately, 400 mg of 7-tert-butoxycarbonylamino-4-(3-pyridyl)-1H,3H-pyrrolo[1,2-c]thiazole was treated for deprotection as described in Example 1 to give 7-amino-4-(3-pyridyl)-1H,3H-pyrrolo[1,2-c]thiazole dihydrochloride. This was added to the filtrate previously obtained as described above, 260 mg of triethylamine was added with ice cooling, and the mixture was stirred overnight at room temperature. The reaction mixture was washed in sequence with dilute aqueous sodium hydrogen carbonate solution and water and dried over anhydrous magnesium sulfate, and the solvent was distilled off. The residue was subjected to silica gel column chromatography. Elution with ethyl acetate gave 200 mg of 7-cyclohexanecarboxamido-3-(3-pyridyl)-1H,3H-pyrrolo[1,2-c]thiazole.
Mass analysis, m/z: 327 ($M^+$)
NMR spectrum ($CDCl_3$; internal standard: TMS)
δ: 1.0-2.4 (11H, m), 4.30 (2H, s), 6.13 (1H, d), 6.19 (1H, d), 6.31 (1H, s), 7.26-7.6 (2H, m), 8.5-8.8 (2H, m)

## Example 77

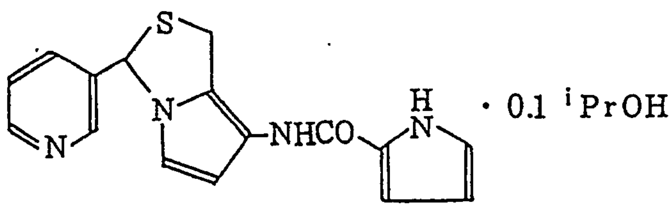

The procedure of Example 76 was followed starting with 7-tert-butoxycarbonylamino-3-(3-pyridyl)-1H,3H-pyrrolo[1,2-c]thiazole and pyrrole-2-carboxylic anhydride to give N-[3-(3-pyridyl)-1H,3H-pyrrolo[1,2-c]thiazol-7-yl]pyrrole-2-carboxamide.
Melting point: 211-214° C

| Elemental analysis (for $C_{16}H_{14}N_4OS \cdot 0.1C_3H_8O$) | | | | |
|---|---|---|---|---|
| | C (%) | H (%) | N (%) | S (%) |
| Calcd: | 61.88 | 4.71 | 17.71 | 10.14 |
| Found: | 61.60 | 4.68 | 17.72 | 10.02 |

Example 78

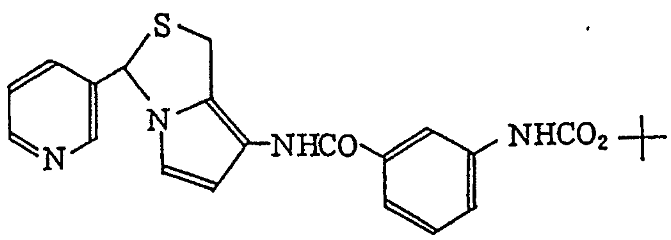

The procedure of Example 76 was followed starting with 7-amino-3-(3-pyridyl)-1H,3H-pyrrolo[1,2-c]-thiazole and m-tert-butoxycarbonylaminobenzoic acid to give m-tert-butoxycarbonylamino-N-[3-(3-pyridyl)-1H,3H-pyrrolo[1,2-c]thiazol-7-yl]benzamide.
Mass analysis, m/z: 436 ($M^+$)
NMR spectrum ($CDCl_3$, internal standard: TMS)
δ: 1.51 (9H, s), 4.33 (2H, s), 6.18 (1H, d), 6.21 (1H, d), 6.30 (1H, s), 7.15-7.7 (6H, m), 7.91 (2H, s), 8.51 (2H, br)

Example 79

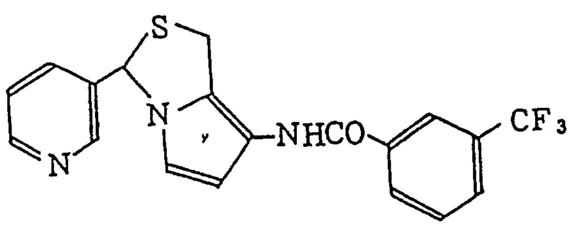

The procedure of Example 76 was followed starting with 7-amino-3-(3-pyridyl)-1H,3H-pyrrolo[1,2-c]-thiazole and m-trifluoromethylbenzoic acid to give m-trifluoromethyl-N-[3-(3-pyridyl)-1H,3H-pyrrolo[1,2-c]-thiazol-7-yl]benzamide.
Melting point: 125-127° C

| Elemental analysis (for $C_{19}H_{14}N_3OSF_3$) | | | | | |
|---|---|---|---|---|---|
| | C (%) | H (%) | N (%) | S (%) | F (%) |
| Calcd: | 58.61 | 3.62 | 10.79 | 8.23 | 14.64 |
| Found: | 58.74 | 3.57 | 10.49 | 8.23 | 14.61 |

Example 80

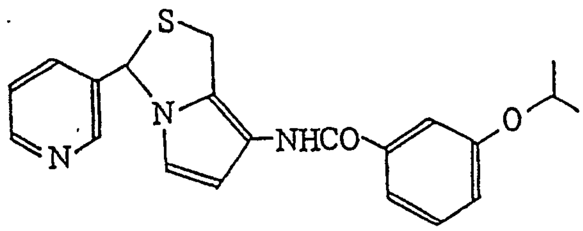

The procedure of Example 76 was followed starting with 7-amino-3-(3-pyridyl)-1H,3H-pyrrolo[1,2-c]-thiazole and m-isopropyloxybenzoic acid to give m-isopropyloxy-N-[3-(3-pyridyl)-1H,3H-pyrrolo[1,2-c]thiazol-7-yl]benzamide.

Mass analysis, m/z: 379 ($M^+$)

NMR spectrum ($CDCl_3$, internal standard: TMS)

δ: 1.35 (6H, d), 4.38 (2H, s), 4.26 (1H, m), 6.21 (1H, d), 6.31 (1H, d), 6.34 (1H, s), 6.95-7.75 (6H, m), 7.78 (1H, s), 8.57 (2H, br)

Example 81

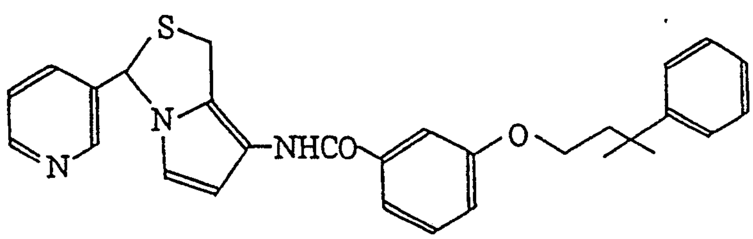

The procedure of Example 76 was followed starting with 7-amino-3-(3-pyridyl)-1H,3H-pyrrolo[1,2-c]-thiazole and m-(3-methyl-3-phenylbutyloxy)benzoic acid to give m-(3-methyl-3-phenylbutyloxy)-N-[3-(3-pyridyl)-1H,3H-pyrrolo[1,2-c]thiazol-7-yl]benzamide.

| Elemental analysis (for $C_{29}H_{29}N_3O_2S \cdot 0.25H_2O$) | | | | |
|---|---|---|---|---|
| | C (%) | H (%) | N (%) | S (%) |
| Calcd: | 71.36 | 6.09 | 8.61 | 6.57 |
| Found: | 71.32 | 6.13 | 8.31 | 6.48 |

Mass analysis, m/z: 483 ($M^+$)

Example 82

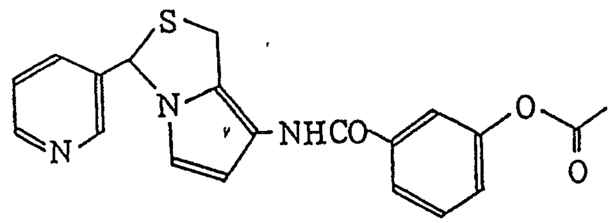

The procedure of Example 76 was followed starting with 7-amino-3-(3-pyridyl)-1H,3H-pyrrolo[1,2-c]-thiazole and m-acetoxybenzoic acid to give m-acetoxy-N-[3-(3-pyridyl)-1H,3H-pyrrolo[1,2-c]thiazol-7-yl]-benzamide.
Melting point: 151-153°C

| Elemental analysis (for $C_{20}H_{17}N_3O_3S$) | | | | |
|---|---|---|---|---|
| | C (%) | H (%) | N (%) | S (%) |
| Calcd: | 63.31 | 4.52 | 11.07 | 8.45 |
| Found: | 63.03 | 4.57 | 10.77 | 8.35 |

Example 83

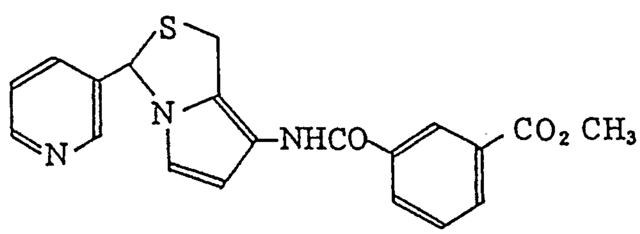

The procedure of Example 76 was followed starting with 7-amino-3-(3-pyridyl)-1H,3H-pyrrolo[1,2-c]-thiazole and monomethyl isophthalate to give m-methoxycarbonyl-N-[3-(3-pyridyl)-1H,3H-pyrrolo[1,2-c]-thiazol-7-yl]benzamide.
Melting point: 172-175°C

| Elemental analysis (for $C_{20}H_{17}N_3O_3S$) | | | | |
|---|---|---|---|---|
| | C (%) | H (%) | N (%) | S (%) |
| Calcd: | 63.31 | 4.52 | 11.07 | 8.45 |
| Found: | 63.36 | 4.51 | 11.04 | 8.47 |

Example 84

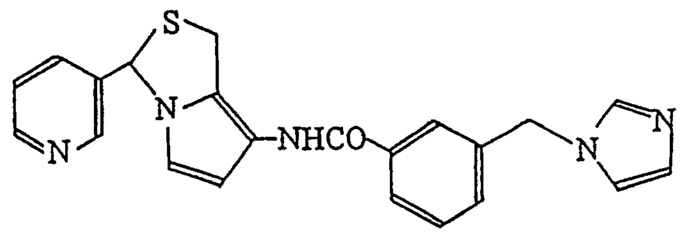

The procedure of Example 76 was followed starting with 7-tert-butoxycarbonylamino-3-(3-pyridyl)-1H,3H-pyrrolo[1,2-c]thiazole and m-(1-imidazolylmethyl)benzoic acid to give m-(1-imidazolylmethyl)-N-[3-(3-pyridyl)-1H,3H-pyrrolo[1,2-c]thiazol-7-yl]benzamide.

| Elemental analysis (for $C_{22}H_{19}N_5OS$) | |
|---|---|
| | S (%) |
| Calcd: | 7.99 |
| Found: | 7.93 |

Mass analysis, m/z: 401 ($M^+$)

Example 85

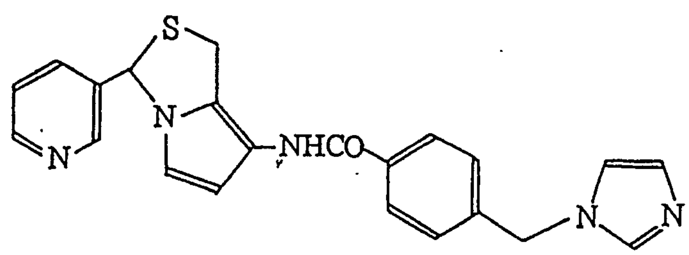

The procedure of Example 76 was followed starting with 7-tert-butoxycarbonylamino-3-(3-pyridyl)-1H,3H-pyrrolo[1,2-c]thiazole and p-(1-imidazolylmethyl)benzoic acid hydrochloride to give p-(1-imidazolylmethyl)-N-[3-(3-pyridyl)-1H,3H-pyrrolo[1,2-c]thiazol-7-yl]benzamide.
Melting point: 168-170 °C

| Elemental analysis (for $C_{22}H_{19}N_5OS$) | |
|---|---|
| | S (%) |
| Calcd: | 7.99 |
| Found: | 7.87 |

Example 86

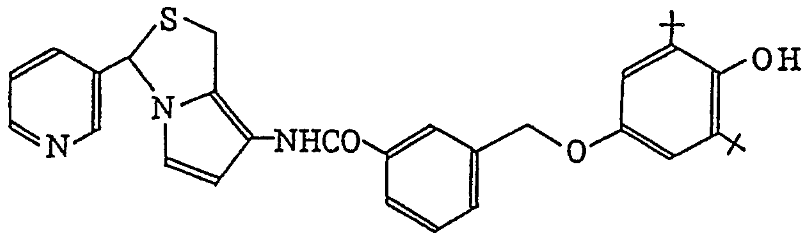

The procedure of Example 76 was followed starting with 7-tert-butoxycarbonylamino-3-(3-pyridyl)-1H,3H-pyrrolo[1,2-c]thiazole and m-[(3,5-di-tert-butyl-4-hydroxyphenoxy)methyl]benzoic acid to give m-[3,5-di-tert-butyl-4-hydroxyphenoxy)methyl]-N-[3-(3-pyridyl)-1H,3H-pyrrolo[1,2-c]thiazol-7-yl]benzamide.

| Elemental analysis (for $C_{33}H_{37}N_3O_3S$) | |
|---|---|
| | S (%) |
| Calcd: | 5.77 |
| Found: | 5.50 |

Mass analysis, m/z: 555 ($M^+$)

## Example 87

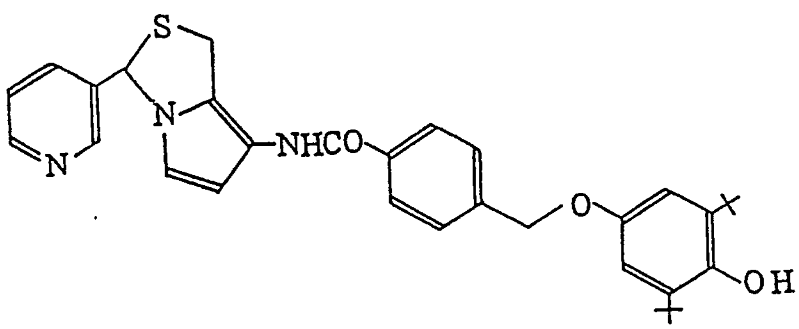

The procedure of Example 76 was followed starting with 7-tert-butoxycarbonylamino-3-(3-pyridyl)-1H,3H-pyrrolo[1,2-c]thiazole and p-[(3,5-di-tert-butyl-4-hydroxyphenoxy)methyl]benzoic acid to give p-[(3,5-di-tert-butyl-4-hydroxyphenoxy)methyl]-N-[3-(3-pyridyl)-1H,3H-pyrrolo[1,2-c]thiazol-7-yl]benzamide.
Melting point: 190-192°C

| Elemental analysis (for $C_{33}H_{37}N_3O_3S$) | | | | |
|---|---|---|---|---|
| | S (%) | H (%) | N (%) | S (%) |
| Calcd: | 71.32 | 6.71 | 7.56 | 5.77 |
| Found: | 71.20 | 6.75 | 7.29 | 5.65 |

Example 88

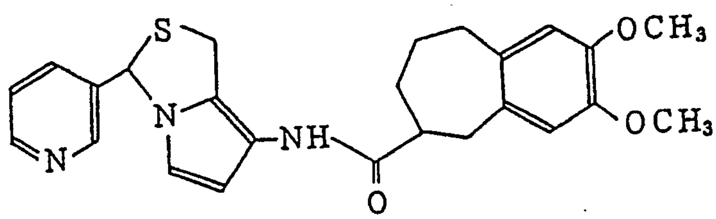

The procedure of Example 76 was followed starting with 2,3-dimethoxy-6,7-dihydro-5H-benzocycloheptene-8-carboxylic acid and 7-tert-butoxycarbonylamino-3-(3-pyridyl)-1H,3H-pyrrolo[1,2-c]thiazole to give 2,3-dimethoxy-N-[3-(3-pyridyl)-1H,3H-pyrrolo[1,2-c]thiazol-7-yl]-6,7-dihydro-5H-benzocycloheptene-8-carboxamide.
Melting point: 186° C

| Elemental analysis (for $C_{25}H_{25}N_3O_3S$) | | | | |
|---|---|---|---|---|
| | S (%) | H (%) | N (%) | S (%) |
| Calcd: | 67.09 | 5.63 | 9.39 | 7.16 |
| Found: | 66.84 | 5.57 | 9.07 | 6.93 |

Example 89

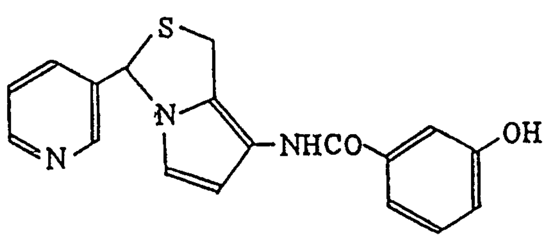

Sodium hydrogen carbonate (3.6 g) was added to a mixture of 3.6 g of m-acetoxy-N-[3-(3-pyridyl)-1H,3H-pyrrolo[1,2-c]thiazol-7-yl]benzamide, 90 ml of methanol and 30 ml of water and the resultant mixture was stirred overnight at room temperature. Water (300 ml) was added to the reaction mixture and the resultant crystals were collected by filtration, washed with water and dried under reduced pressure to give 2.7 g of m-hydroxy-N-[3-(3-pyridyl)-1H,3H-pyrrolo[1,2-c]thiazol-7-yl]benzamide.
Melting point: 175-178° C

| Elemental analysis (for $C_{18}H_{15}N_3O_2S$) | | | | |
|---|---|---|---|---|
| | C (%) | H (%) | N (%) | S (%) |
| Calcd: | 64.08 | 4.48 | 12.45 | 9.50 |
| Found: | 63.78 | 4.46 | 12.26 | 9.35 |

Example 90

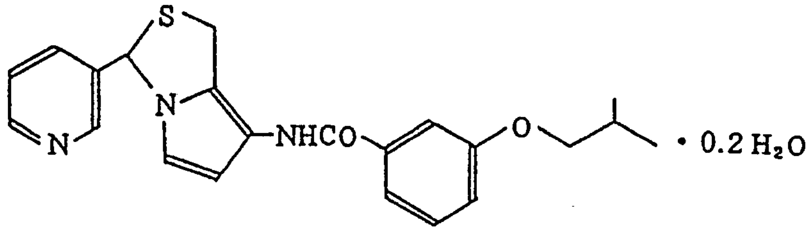

1-Bromo-2-methylpropane (0.15 ml) was added to a mixture of 0.4 g of m-hydroxy-N-[3-(3-pyridyl)-1H,3H-pyrrolo[1,2-c]thiazol-7-yl]benzamide, 0.2 g of potassium carbonate and 3 ml of dimethylformamide and the resultant mixture was stirred overnight at 90 °C. Ethyl acetate-benzene (2:1) was added to the reaction mixture and the whole mixture was washed with water and saturated aqueous sodium chloride solution and then dried over anhydrous sodium sulfate, and the solvent was distilled off. The residue was subjected to silica gel column chromatography. Elution with ethyl acetate-methylene chloride (1:2) gave m-(3-methylpropyloxy)-N-[3-(3-pyridyl)-1H,3H-pyrrolo[1,2-c]thiazol-7-yl]benzamide 0.2 hydrate.

| Elemental analysis (for $C_{22}H_{23}N_3O_2S \cdot 0.2H_2O$) | | | | |
|---|---|---|---|---|
| | C (%) | H (%) | N (%) | S (%) |
| Calcd: | 66.54 | 5.94 | 10.58 | 8.07 |
| Found: | 66.54 | 5.94 | 10.33 | 8.01 |

Mass analysis, m/z: 393 ($M^+$)

Example 91

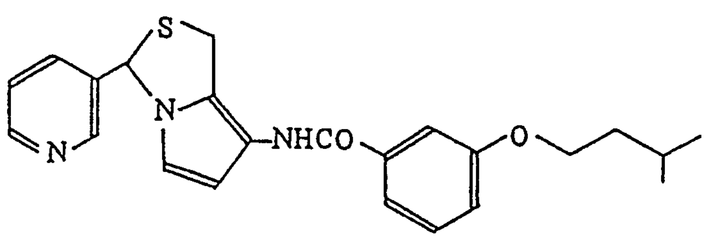

The procedure of Example 90 was followed using m-hydroxy-N-[3-(3-pyridyl)-1H,3H-pyrrolo[1,2-c]-thiazol-7-yl]benzamide and 1-iodo-3-methylbutane to give m-(3-methylbutyloxy)-N-[3-(3-pyridyl)-1H,3H-pyrrolo[1,2-c]thiazol-7-yl]benzamide.
Melting point: 132-134 °C

| Elemental analysis (for $C_{23}H_{25}N_3O_2S$) | | | | |
|---|---|---|---|---|
| | C (%) | H (%) | N (%) | S (%) |
| Calcd: | 67.79 | 6.18 | 10.31 | 7.87 |
| Found: | 67.87 | 6.13 | 10.21 | 7.90 |

Example 92

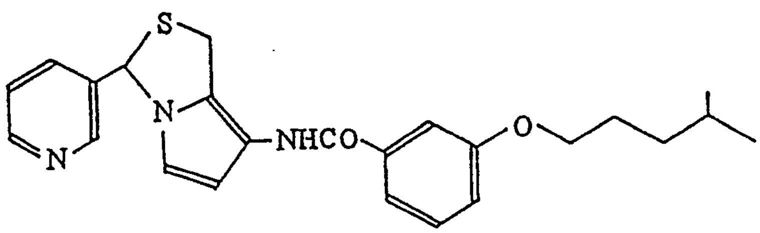

The procedure of Example 90 was followed using m-hydroxy-N-[3-(3-pyridyl)-1H,3H-pyrrolo[1,2-c]-thiazol-7-yl]benzamide and 1-bromo-4-methylpentane to give m-(4-methylpentyloxy)-N-[3-(3-pyridyl)-1H,3H-pyrrolo[1,2-c]thiazol-7-yl]benzamide.
Melting point: 128-130°C

| Elemental analysis (for $C_{24}H_{27}N_3O_2S$) | | | | |
|---|---|---|---|---|
| | C (%) | H (%) | N (%) | S (%) |
| Calcd: | 68.38 | 6.46 | 9.97 | 7.61 |
| Found: | 68.42 | 6.49 | 9.92 | 7.61 |

Example 93

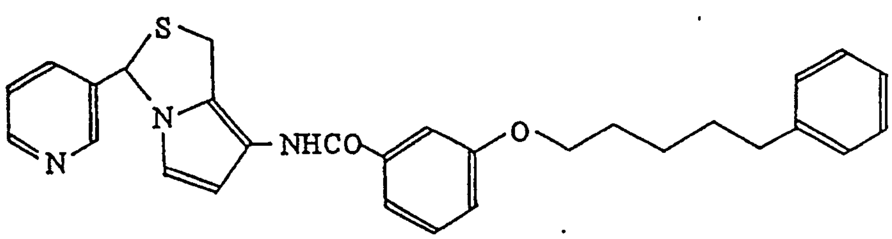

The procedure of Example 90 was followed using m-hydroxy-N-[3-(3-pyridyl)-1H,3H-pyrrolo[1,2-c]-thiazol-7-yl]benzamide and 5-phenylpentyl bromide to give m-(5-phenylpentyloxy)-N-[3-(3-pyridyl)-1H,3H-pyrrolo- [1,2-c]thiazol-7-yl]benzamide.
Melting point: 89-91°C

| Elemental analysis (for $C_{29}H_{29}N_3O_2S$) | | | | |
|---|---|---|---|---|
| | C (%) | H (%) | N (%) | S (%) |
| Calcd: | 72.02 | 6.04 | 8.69 | 6.63 |
| Found: | 71.91 | 5.99 | 8.50 | 6.55 |

Example 94

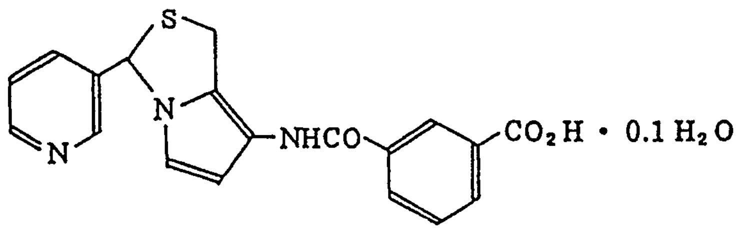

To a solution of 0.68 g of m-methoxycarbonyl-N-[3-(3-pyridyl)-1H,3H-pyrrolo[1,2-c]thiazol-7-yl]-benzamide in 6 ml of methanol was added 2.2 ml of 1 N aqueous sodium hydroxide. The mixture was stirred at 60°C for 1 hour. Water (50 ml) and 2.2 ml of 1 N hydrochloric acid were added to the reaction mixture and the resultant crystals were collected by filtration, washed with water and 2-propanol and dried under reduced pressure to give 0.48 g of m-[N-[3-(3-pyridyl}-1H,3H-pyrrolo[1,2-c]thiazol-7-yl]carbamoyl]-benzoic acid 0.1 hydrate.
Melting point: 208-211°C

| Elemental analysis (for $C_{19}H_{15}N_3O_3S \cdot 0.1H_2O$) | | | | |
|---|---|---|---|---|
| | C (%) | H (%) | N (%) | S (%) |
| Calcd: | 62.15 | 4.17 | 11.44 | 8.73 |
| Found: | 62.12 | 4.12 | 11.31 | 8.72 |

Example 95

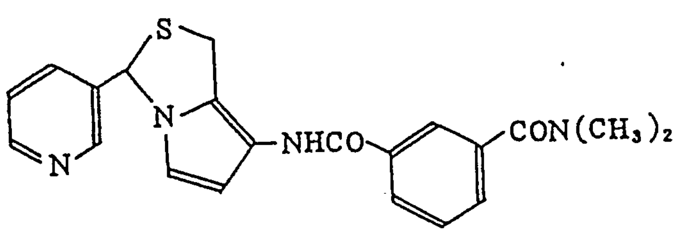

Diphenylphosphoryl azide (0.2 ml) and 0.3 ml of triethylamine were added to a mixture of 0.27 g of m-[N-[3-(3-pyridyl)-1H,3H-pyrrolo[1,2-c]thiazol-7-yl]carbamoyl]benzoic acid, 0.08 g,of dimethylamine hydrochloride and 1 ml of N,N-dimethylformamide and the resultant mixture was stirred overnight at room temperature. Ethyl acetate-benzene (2:1) (60 ml) was added to the reaction mixture and the resultant mixture was washed with water, saturated aqueous sodium hydrogen carbonate solution, water and saturated aqueous sodium chloride solution in that order. The organic layer was dried over anhydrous sodium sulfate and the solvent was distilled off under reduced pressure. The residue was subjected to silica gel column chromatography. Elution with ethyl acetate-methylene chloride (1:1) gave 0.14 g of m-(N,N-dimethylcarbamoyl)-N-[3-(3-pyridyl)-1H,3H-pyrrolo[1,2-c]thiazol-7-yl]benzamide.
Mass analysis, m/z: 392 ($M^+$)
NMR spectrum ($CDCl_3$, internal standard: TMS)
δ: 2.97 (3H, s), 3.10 (3H, s), 4.30 (1H, d), 4.43 (1H, d), 6.23 (1H, d), 6.27 (1H, d), 6.34 (1H, s), 7.1-8.0 (6H, m), 8.2-8.5 (2H, m), 8.64 (1H, s)

Example 96

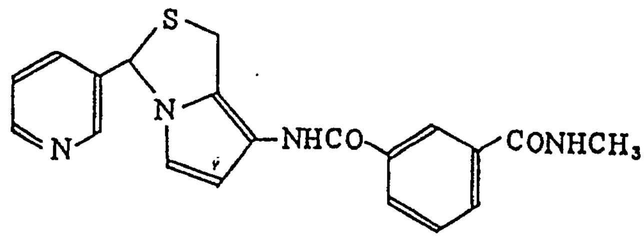

The procedure of Example 95 was followed using m-[N-[3-(3-pyridyl)-1H,3H-pyrrolo[1,2-c]]thiazol-7-yl]-carbamoyl]benzoic acid and methylamine to give m-(N-methylcarbamoyl)-N-[3-(3-pyridyl)-1H,3H-pyrrolo-[1,2-c]thiazol-7-yl]benzamide.
Melting point: 200-205° C
NMR spectrum (DMSO-$d_6$, internal standard: TMS)
δ: 2.80 (3H, s), 4.26 (1H, d), 4.36 (1H, d), 6.35 (1H, d), 6.49 (1H, d), 6.63 (1H, s), 7.3-7.7 (3H, m), 7.9-8.1 (2H, m), 8.4 (1H, s), 8.4-8.7 (2H, m), 10.10 (1H, s)

Example 97

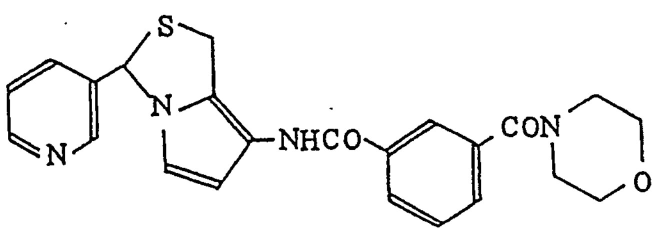

The procedure of Example 95 was followed using m-[N-[3-(3-pyridyl)-1H,3H-pyrrolo[1,2-c]thiazol-7-yl]-carbamoyl]benzoic acid and morpholine to give m-(morpholinocarbonyl)-N-[3-(3-pyridyl)-1H,3H-pyrrolo[1,2-c]thiazol-7-yl]benzamide.
Mass analysis, m/z: 434 ($M^+$)
NMR spectrum ($CDCl_3$, internal standard: TMS)
δ: 3.70 (8H, broad s), 4.38 (2H, s), 6.26 (2H, s), 6.36 (1H, s), 7.26-7.60 (4H, m), 7.88-7.97 (2H, m), 8.57-8.64 (2H, m)

Example 98

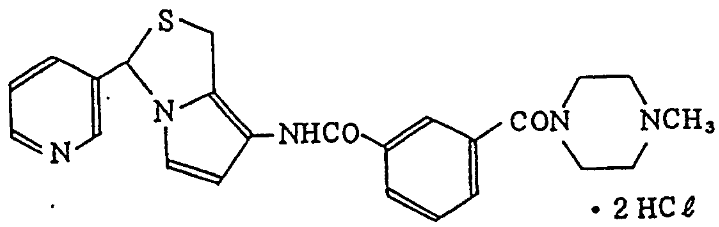

The procedure of Example 95 was followed using m-[N-[3-(3-pyridyl)-1H,3H-pyrrolo[1,2-c]thiazol-7-yl]-carbamoyl]benzoic acid and 1-methylpiperazine to give m-[(4-methyl-1-piperazinyl)carbonyl]-N-[3-(3-pyridyl)1H,3H-pyrrolo[1,2-c]thiazol-7-yl]benzamide dihydrochloride.
Mass analysis, m/z: 447 $(M-2HCl)^+$
NMR spectrum ($DMSO-d_6$, internal standard: TMS)
δ: 2.77 (3H, s), 3.0-3.20 (2H, m), 3.20-3.60 (2H, m), 4.38 (2H, dd), 6.38 (1H, d), 6.61 (1H, d), 6.79 (1H, s), 7.50-7.70 (2H, m), 7.90 (1H, dd), 8.0-8.10 (3H, m), 8.65 (1H, d), 8.82 (1H, dd)

Example 99

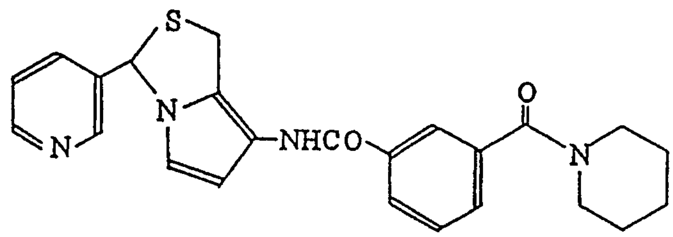

The procedure of Example 95 was followed using m-[N-[3-(3-pyridyl)-1H,3H-pyrrolo[1,2-c]thiazol-7-yl]-carbamoyl]benzoic acid and piperidine to give m-(piperidinocarbonyl)-N-[3-(3-pyridyl)-1H,3H-pyrrolo[1,2-c]-thiazol-7-yl]benzamide.
Mass analysis, m/z: 432 $(M^+)$
NMR spectrum ($CDCl_3$, internal standard: TMS)
δ: 1.50 (2H, s), 1.86 (4H, s), 3.31 (2H, s), 3.70 (2H, s), 4.40 (2H, dd), 6.25 (1H, d), 6.30 (1H, d), 6.35 (1H, s), 7.20-8.0 (6H, m), 8.50-8.70 (2H, m)

Example 100

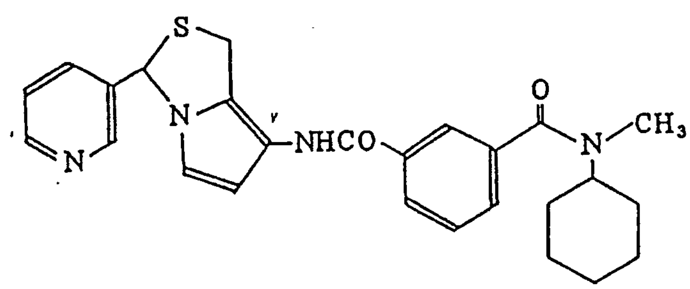

The procedure of Example 95 was followed using m-[N-[3-(3-pyridyl)-1H,3H-pyrrolo[1,2-c]thiazol-7-yl]-carbamoyl]benzoic acid and N-methylcyclohexylamine to give m-(N-cyclohexyl-N-methylcarbamoyl)-N-[3-(3-pyridyl)-1H,3H-pyrrolo[1,2-c]thiazol-7-yl]benzamide.
Mass analysis, m/z: 460 $(M^+)$
NMR spectrum ($CDCl_3$, internal standard: TMS)
δ: 0.9-1.2 (2H, m), 1.2-2.0 (8H, m), 2.6-3.1 (3H, m), 4.40 (2H, dd), 6.26 (1H, d), 6.30 (1H, d), 6.36 (1H, s), 7.0-8.0 (6H, m), 8.5-8.7 (2H, m)

Example 101

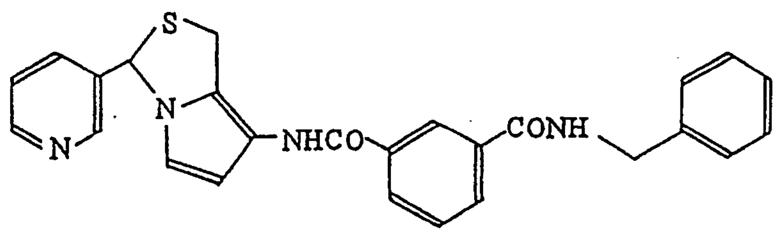

The procedure of Example 95 was followed using m-[N-[3-(3-pyridyl)-1H,3H-pyrrolo[1,2-c]thiazol-7-yl]-carbamoyl]benzoic acid and benzylamine to give m-(N-benzylcarbamoyl)-N-[3-(3-pyridyl)-1H,3H-pyrrolo[1,2-c]thiazol-7-yl]benzamide.
Melting point: 155-158°C

| Elemental analysis (for $C_{26}H_{22}N_4O_2S$) | |
|---|---|
| | S (%) |
| Calcd.: | 7.05 |
| Found : | 7.06 |

Example 102

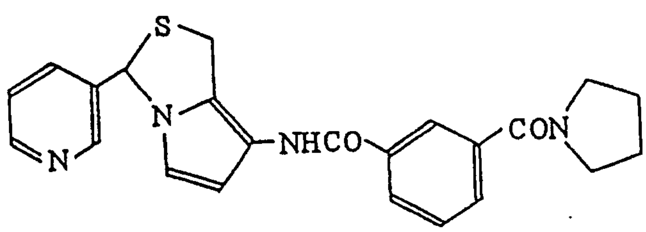

The procedure of Example 95 was followed using m-[N-[3-(3-pyridyl)-1H,3H-pyrrolo[1,2-c]thiazol-7-yl]-carbamoyl]benzoic acid and pyrrolidine to give m-(pyrrolidinocarbonyl)-N-[3-(3-pyridyl)-1H,3H-pyrrolo[1,2-c]-thiazol-7-yl]benzamide.
Mass analysis, m/z: 418 ($M^+$)
NMR spectrum ($CDCl_3$, internal standard: TMS)
δ: 1.8-2.1 (4H, m), 3.42 (2H, t), 3.63 (2H, t), 4.37 (2H, s), 6.24 (2H, s), 6.34 (1H, s), 7.2-8.2 (6H, m), 8.5-8.7 (2H, m)

Example 103

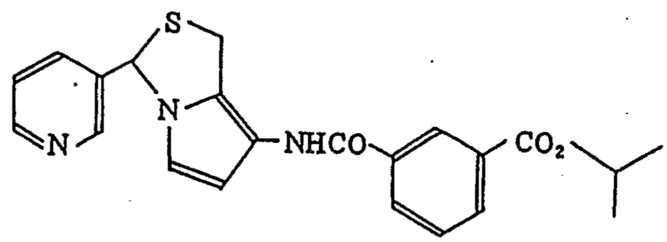

A mixture of 0.26 g of m-[N-[3-(3-pyridyl)-1H,3H-pyrrolo[1,2-c]thiazol-7-yl]carbamoyl]benzoic acid, 0.1 ml of isopropyl iodide, 0.1 g of potassium hydrogen carbonate and 1 ml of N,N-dimethylformamide was stirred at 60° C for 4 hours. Ethyl acetate-benzene (2:1) (60 ml) was added to the reaction mixture and the whole mixture was washed in sequence with three 20 ml portions of water and one 20 ml portion of saturated aqueous sodium chloride solution. The organic layer was dried over anhydrous sodium sulfate and the solvent was then distilled off under reduced pressure. The residue was subjected to silica gel column chromatography. Elution with methylene chloride-ethyl acetate (3:1) followed by recrystallization from ethyl acetate-n-hexane gave 0.11 g of m-isopropyloxycarbonyl-N-[3-(3-pyridyl)-1H,3H-pyrrolo[1,2-c]-thiazol-7-yl]benzamide.
Melting point: 126-129° C

| Elemental analysis (for $C_{22}H_{21}N_3O_2S$) | | | | |
|---|---|---|---|---|
| | C (%) | H (%) | N (%) | S (%) |
| Calcd.: | 64.85 | 5.19 | 10.31 | 7.87 |
| Found. | 64.79 | 5.15 | 10.23 | 7.77 |

Example 104

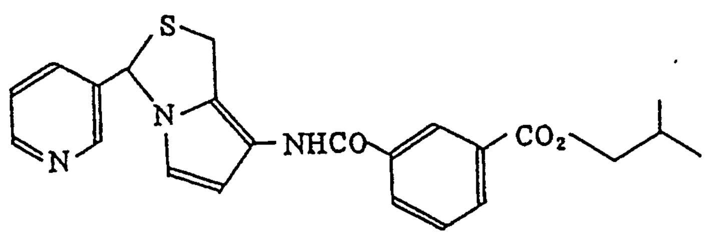

The procedure of Example 103 was followed using m-[N-[3-(3-pyridyl)-1H,3H-pyrrolo[1,2-c]thiazol-7-yl]-carbamoyl]benzoic acid and 1-bromo-2-methlpropane to give m-(2-methylpropyloxycarbonyl)-N-[3-(3-pyridyl)-1H,3H-pyrrolo[1,2-c]thiazol-7-yl]benzamide.
Melting point: 123-126° C

| Elemental analysis (for $C_{23}H_{23}N_3O_3S$) | |
|---|---|
| | S (%) |
| Calcd.: | 7.60 |
| Found : | 7.55 |

Example 105

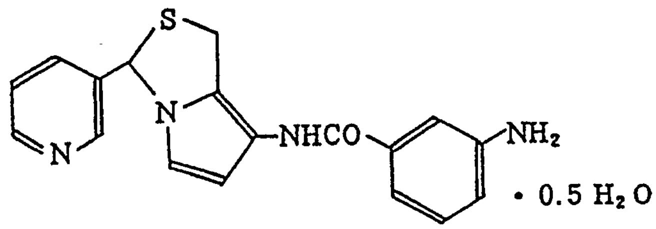

Trifluoroacetic acid was added to 0.7 g of m-tert-butoxycarbonylamino-N-[3-(3-pyridyl)-1H,3H-pyrrolo-[1,2-c]thiazol-7-yl]benzamide and the mixture was stirred at room temperature for 1 hour. The reaction mixture was poured into saturated aqueous sodium hydrogen carbonate solution. The resulting crystals were collected by filtration, washed with water and 2-propanol and dried under reduced pressure to give 0.36 g of m-amino-N-[3-(3-pyridyl)-1H,3H-pyrrolo[1,2-c]thiazol-7-yl]benzamide hemihydrate.
Melting point: 203-207 °C

| Elemental analysis (for $C_{18}H_{16}N_4OS \cdot 0.5H_2O$) | | | | |
|---|---|---|---|---|
| | C (%) | H (%) | N (%) | S (%) |
| Calcd.: | 62.59 | 4.96 | 16.22 | 9.28 |
| Found : | 62.55 | 4.73 | 16.05 | 9.29 |

Example 106

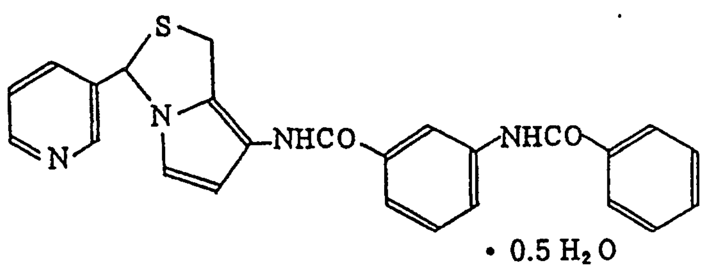

Benzoyl chloride (80 μl) and 0.1 ml of triethylamine were added to a solution of 0.15 g of m-amino-N-[3-(3-pyridyl)-1H,3H-pyrrolo[1,2-c]thiazol-7-yl]benzamide in 1 ml of methylene chloride with ice cooling and the mixture was stirred at room temperature for 4 hours. Saturated aqueous sodium hydrogen carbonate solution (30 ml) was added to the reaction mixture, followed by extraction with methylene chloride. The extract was dried over anhydrous sodium sulfate and the solvent was distilled off. The residue was subjected to silica gel column chromatography. Elution with methylene chloride-ethyl acetate (2:1) followed by recrystallization from 2-propanol-n-hexane to give 0.1 g of m-benzoylamino-N-[3-(3-pyridyl)-1H,3H-pyrrolo[1,2-c]thiazol-7-yl]benzamide hemihydrate.
Melting point: 138-141 °C

| Elemental analysis (for $C_{25}H_{20}N_4O_2S\cdot 0.5H_2O$) | |
|---|---|
| | S (%) |
| Calcd.:<br>Found : | 7.13<br>7.17 |

Example 107

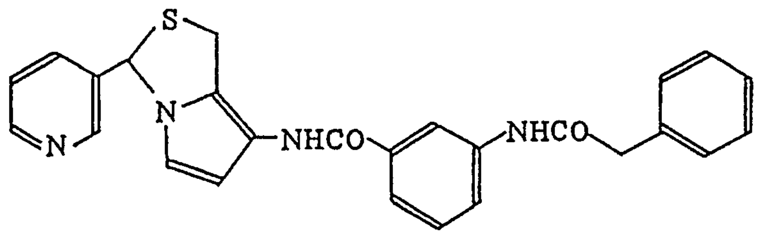

The procedure of Example 106 was followed using m-amino-N-[3-(3-pyridyl)-1H,3H-pyrrolo[1,2-c]thiazol-7-yl]benzamide and phenylacetyl chloride to give m-phenylacetamido-N-[3-(3-pyridyl)-1H,3H-pyrrolo[1,2-c]-thiazol-7-yl]benzamide.
Melting point: 137-140 °C

| Elemental analysis (for $C_{26}H_{22}N_4O_2S$) | |
|---|---|
| | S (%) |
| Calcd.:<br>Found : | 7.05<br>6.90 |

Example 108

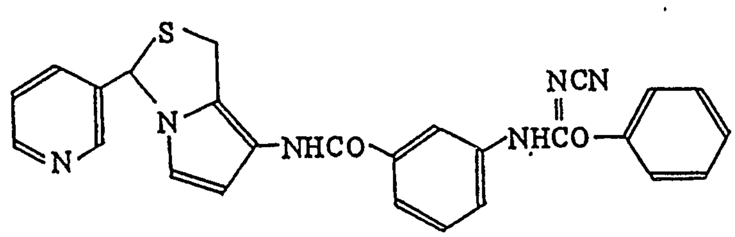

A mixture of 1.1 g of m-amino-N-[3-(3-pyridyl)-1H,3H-pyrrolo[1,2-c]thiazol-7-yl]benzamide, 0.79 g of diphenyl N-cyanocarbonimidate and 7 ml of 2-propanol was stirred overnight with heating under reflux. The

solvent was distilled off from the reaction mixture under reduced pressure and the residue was subjected to silica gel column chromatography. Elution with chloroform-methanol (50:1) followed by recrystallization from 2-propanol gave 0.8 g of m-(N-cyano-o-phenylureido)-N-[3-(3-pyridyl)-1H,3H-pyrrolo[1,2-c]thiazol-7-yl]-benzamide.
Melting point: 145-150 °C

| Elemental analysis (for $C_{26}H_{20}N_6O_2S$) | |
|---|---|
| | S (%) |
| Calcd.: | 6.67 |
| Found : | 6.58 |

Example 109

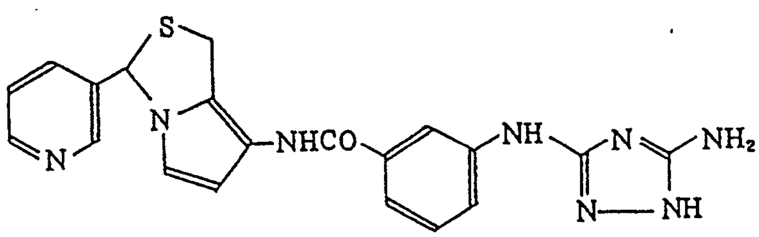

Hydrazine hydrate (0.03 ml) was added to a solution of 0.3 g of m-(N-cyano-o-phenylisoureido)-N-[3-(3-pyridyl)-1H,3H-pyrrolo[1,2-c]thiazol-7-yl]benazmide in 2 ml of methanol and the mixture was stirred at room temperature for 3 hours. The resultant crystals were collected by filtration and washed with methanol to give 0.21 g of m-[(5-amino-1,2,4-triazol-3-yl)amino]-N-[3-(3-pyridyl)-1H,3H-pyrrolo[1,2-c]thiazol-7-yl]benazmide.
Melting point: 225-230 °C

| Elemental analysis (for $C_{20}H_{18}N_8OS$) | | | | |
|---|---|---|---|---|
| | C (%) | H (%) | N (%) | S (%) |
| Calcd.: | 57.40 | 4.34 | 26.78 | 7.66 |
| Found: | 57.36 | 4.43 | 25.50 | 7.48 |

Example 110

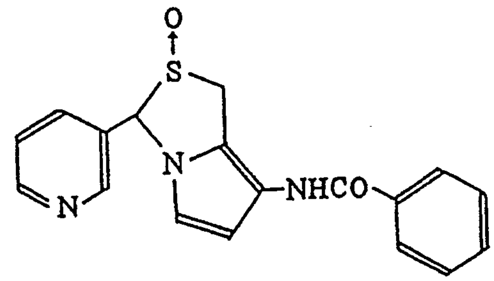

m-Chloroperbenzoic acid (200 mg) was added to a solution of 320 mg of N-[3-(3-pyridyl)-1H,3H-pyrrolo-[1,2-c]thiazol-7-yl]benzamide in 20 ml of methylene chloride with ice cooling and the mixture was stirred for 1 hour. m-Chloroperbenzoic acid (20 mg) was further added and stirring was continued for 1 hour. Methylene chloride (50 ml) was added to the reaction mixture and the whole mixture was washed in sequence with saturated aqueous sodium hydrogen carbonate solution, water and dried over anhydrous magnesium sulfate. The solvent was distilled off under reduced pressure and the residue was subjected to silica gel column chromatography. Elution with ethyl acetate gave 120 mg of 7-benzamido-3-(3-pyridyl)-1H,3H-pyrrolo[1,2-c]thiazole 2-oxide.
Mass analysis, m/z: 337 $(M+1)^+$
NMR spectrum ($CDCl_3$, internal standard: TMS)
δ: 4.2-4.87 (2H, m), 6.01-6.57 (3H, m), 7.2-7.98 (7H, m), 8.49 (1H, S), 8.65 (1H, t)

## Example 111

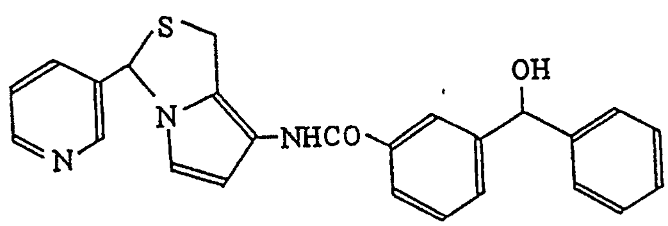

Sodium borohydride (120 mg) was added to a mixture of 260 mg of m-benzoyl-N-[3-(3-pyridyl)-1H,3H-pyrrolo[1,2-c]thiazol-7-yl]benzamide, 5 ml of ethanol and 5 ml of methylene chloride with ice cooling and the resultant mixture was stirred overnight at room temperature. The solvent was distilled off from the reaction mixture under reduced pressure. Ethyl acetate (50 ml) was added to the residue, the mixture was washed with water and dried over anhydrous magnesium sulfate, the solvent was distilled off under reduced pressure, and the residue was subjected to silica gel column chromatography. Elution with ethyl acetate gave 160 mg of m-(1-hydroxy-1-phenyl)methyl-N-[3-(3-pyridyl)-1H,3H-pyrrolo[1,2-c]thiazol-7-yl]benzamide.
Mass analysis, m/z: 427 $(M^+)$
NMR spectrum ($CDCl_3$, internal standard: TMS)
δ: 4.38 (2H, dd), 5.92 (1H, s), 6.21 (1H, d), 6.25 (1H, d), 6.35 (1H, s), 7.1-8.0 (1H, m), 8.54 (1H, d), 8.60 (1H, dd)

## Example 112

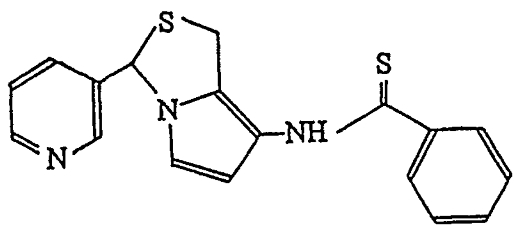

A mixture of N-[3-(3-pyridyl)-1H,3H-pyrrolo[1,2-c]thiazol-7-yl]benzamide (322 mg), phosphorus pentasulfide (270 mg), sodium hydrogen carbonate (110 mg) and 1,2-dimethoxyethane (10 ml) was stirred at 65°C for 1 hour and then at 85°C for 2 hours. After cooling, ice, water and potassium carbonate were added to the reaction mixture for dissolving the insoluble matter. The whole mixture was extracted with methylene chloride, the extract was dried over anhydrous sodium sulfate, and the solvent was distilled off under reduced pressure. The residue obtained was crystallized by addition of diethyl ether. Collection of the crystals by filtration gave N-[3-(3-pyridyl)-1H,3H-pyrrolo[1,2-c]thiazol-7-yl]thiobenzamide (157 mg).
Melting point: 131°C

| Elemental analysis (for $C_{18}H_{15}N_3S_2$) | | | | |
|---|---|---|---|---|
| | C (%) | H (%) | N (%) | S (%) |
| Calcd.: | 64.06 | 4.48 | 12.45 | 19.00 |
| Found : | 63.82 | 4.56 | 12.29 | 18.90 |

Example 113

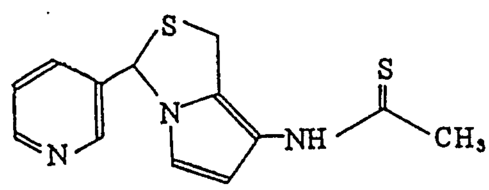

N-[3-(3-Pyridyl)-1H,3H-pyrrolo[1,2-c]thiazol-7-yl]thioacetamide was synthesized from N-[3-(3-pyridyl)-1H,3H-pyrrolo[1,2-c]thiazol-7-yl]acetamide by following the procedure of Example 112.
Melting point: 148°C

| Elemental analysis (for $C_{13}H_{13}N_3S_2$) | | | | |
|---|---|---|---|---|
| | C (%) | H (%) | N (%) | S (%) |
| Calcd.: | 56.70 | 4.76 | 15.26 | 23.29 |
| Found : | 56.70 | 4.79 | 15.08 | 23.23 |

Example 114

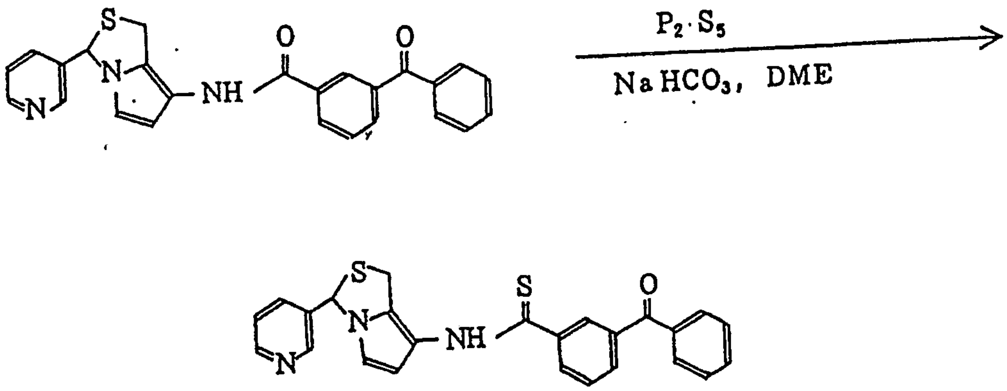


The procedure of Example 112 was followed starting with m-benzoyl-N-[3-(3-pyridyl)-1H,3H-pyrrolo[1,2-c]thiazol-7-yl]benzamide to give m-benzoyl-N-[3-(3-pyridyl)-1H,3H-pyrrolo[1,2-c]thiazol-7-yl]thiobenzamide.
Mass analysis, m/z: 441 (M+)
NMR spectrum ($CDCl_3$, internal standard: TMS)
δ: 4.28 (2H, s), 6.33 (1H, d), 6.39-6.43 (2H, m), 7.31-7.84 (9H, m), 8.17-8.22 (2H, m), 8.53-8.60 (2H, m)

Example 115

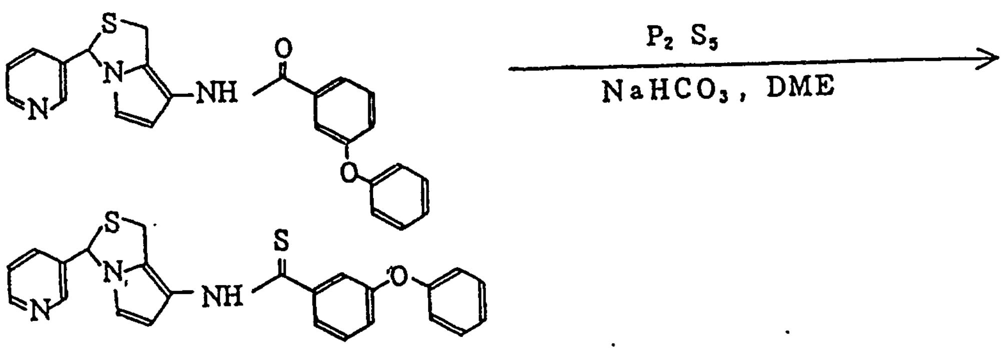


The procedure of Example 112 was followed starting with m-phenoxy-N-[3-(3-pyridyl)-1H,3H-pyrrolo-[1,2-c]thiazol-7-yl]benzamide to give m-phenoxy-N-[3-(3-pyridyl)-1H,3H-pyrrolo[1,2-c]thiazol-7-yl]-thiobenzamide.
Mass analysis, m/z: 429 ($M^+$)
NMR spectrum ($CDCl_3$, internal standard: TMS)
δ: 4.26 (2H, S), 6.33 (1H, d), 6.38 (1H, d), 6.41 (1H, S), 7.04-7.64 (11H, m), 8.57-8.64 (2H, m)

Example 116

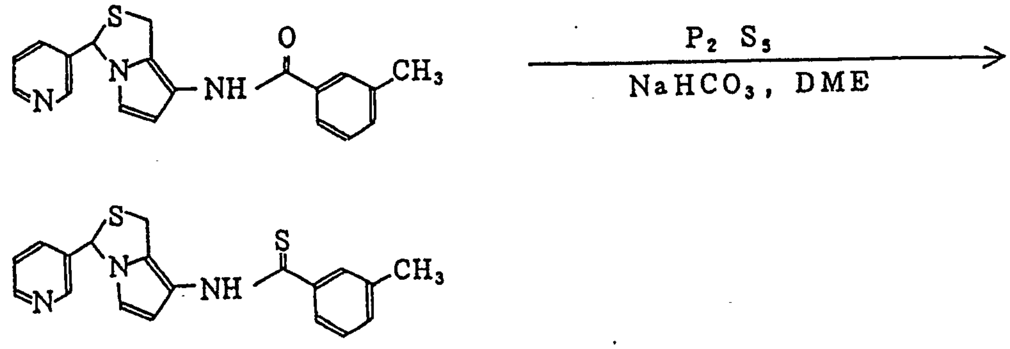


The procedure of Example 112 was followed starting with m-methyl-N-[3-(3-pyridyl)-1H,3H-pyrrolo[1,2-c]thiazol 7-yl]benzamide to give m-methyl-N-[3-(3-pyridyl)-1H,3H-pyrrolo[1,2-c]thiazol-7-yl]thiobenzamide.
Mass analysis, m/z: 351 ($M^+$)
NMR spectrum ($CDCl_3$, internal standard: TMS)
δ: 2.43 (3H, S), 4.28 (2H, S), 6.34 (1H, d), 6.39 (1H, d), 6.42 (1H, S), 7.32-7.38 (3H, m), 7.61-7.69 (2H, m), 7.72-7.75 (1H, m), 7.58-7.64 (2H, m)

## Example 117

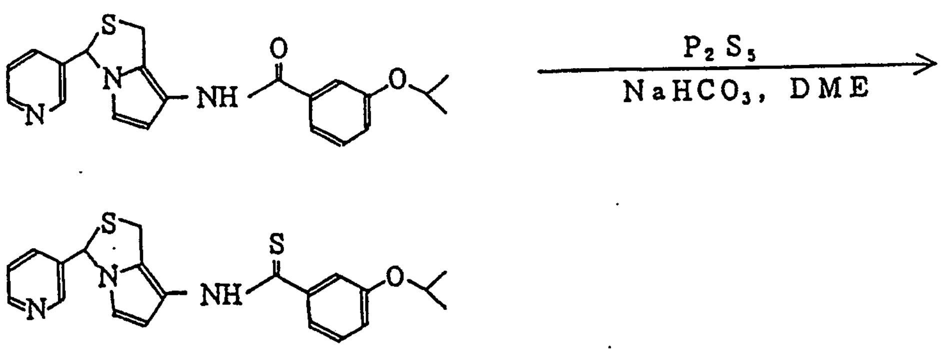


The procedure of Example 112 was followed starting with m-(1-methylethyl)-N-[3-(3-pyridyl)-1H,3H-pyrrolo[1,2-c]thiazol-7-yl]benzamide to give m-(1-methylethyl)-N-[3-(3-pyridyl)-1H,3H-pyrrolo[1,2-c]thiazol-7-yl]thiobenzamide.
Mass analysis, m/z: 395 ($M^+$)
NMR spectrum ($CDCl_3$, internal standard: TMS)
δ: 1.35 (6H, d), 4.28 (2H, S), 4.58-4.72 (1H, m), 6.34 (1H, d), 6.38-6.41 (2H, m), 7.00-7.05 (1H, m), 7.27-7.90 (m, 3H), 7.44-7.48 (1H, m), 7.60-7.66 (1H, m), 7.56-7.63 (2H, m)

## Example 118

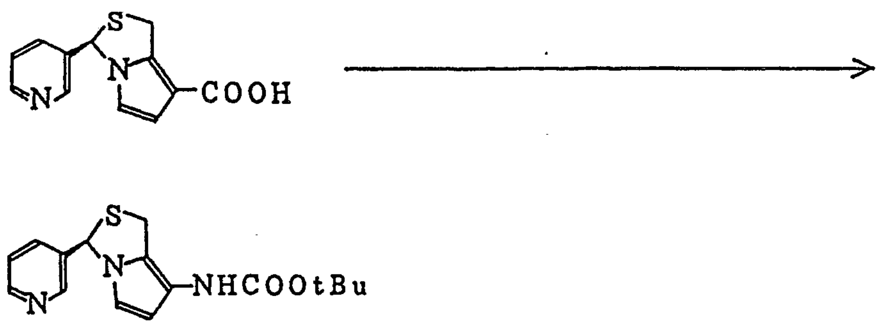

The procedure of Example 1 was followed starting with (R)-3-(3-pyridyl)-1H,3H-pyrrolo[1,2-c]-7-thiazolecarboxylic acid (7.39 g) to give (R)-7-tert-butoxycarbonylamino-3-(3-pyridyl)-1H,3H-pyrrolo[1,2-c]-thiazole (4.8 g).
Melting point: 81-84 °C
$[\alpha]_D^{20}$ : +154.94° (c=1.00, DMF)

Example 119

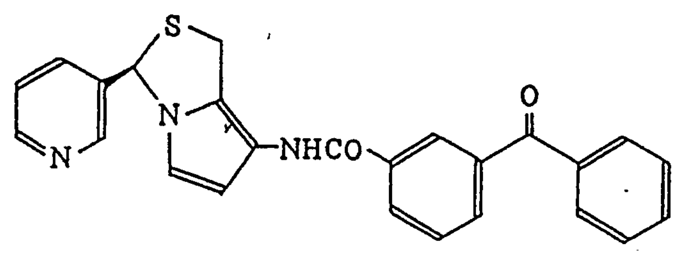

The procedure of Example 49 was followed starting with (R)-7-tert-butoxycarbonylamino-3-(3-pyridyl)-1H,3H-pyrrolo[1,2-c]thiazole and m-benzoylbenzoyl chloride (prepared from m-benzoylbenzoic acid) to give (R)-m-benzoyl-N-[3-(3-pyridyl)-1H,3H-pyrrolo[1,2-c]thiazol-7-yl]benzamide.
Melting point: 97-99 °C
$[\alpha]_D^{20}$ : +82.62° (c=0.99, DMF)

| Elemental analysis (for $C_{25}H_{19}N_3O_2S$) | |
|---|---|
| | S (%) |
| Calcd.: | 7.54 |
| Found : | 7.26 |

The compounds of Examples 120 to 122 were produced in the same manner as mentioned above.

Example 120

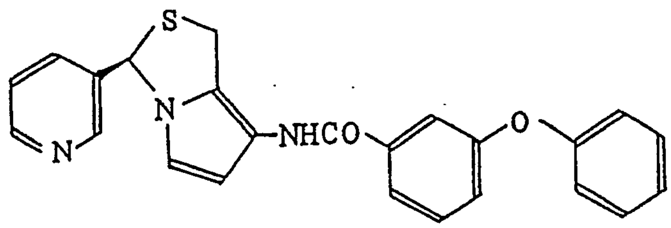

(R)-m-Phenoxy-N-[3-(3-pyridyl)-1H,3H-pyrrolo[1,2-c]thiazol-7-yl]benzamide
Melting point: 136-138 °C
$[\alpha]_D^{20}$ : +83.49 ° (c=1.00, DMF)

| Elemental analysis (for $C_{24}H_{19}N_3O_2S$) | | | | |
|---|---|---|---|---|
| | C (%) | H (%) | N (%) | S (%) |
| Calcd.: | 69.71 | 4.63 | 10.16 | 7.75 |
| Found : | 69.70 | 4.58 | 10.06 | 7.73 |

Example 121

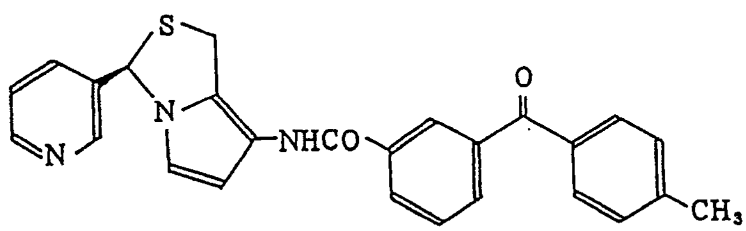

(R)-N-[3-(3-Pyridyl)-1H,3H-pyrrolo[1,2-c]thiazol-7-yl]-m-(p-toluoyl)benzamide
Melting point: 94-97 °C
$[\alpha]_D^{20}$ : +75.0 ° (c=1.00, DMF)

| Elemental analysis (for $C_{25}H_{21}N_3O_2S \cdot 0.5C_3H_8O$) | | | | |
|---|---|---|---|---|
| | C (%) | H (%) | N (%) | S (%) |
| Calcd.: | 70.34 | 5.37 | 8.95 | 6.83 |
| Found : | 70.27 | 5.18 | 8.94 | 7.00 |

Example 122

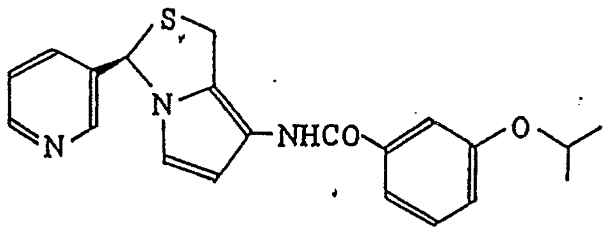

(R)-m-Isopropoxy-N-[3-(3-pyridyl)-1H,3H-pyrrolo[1,2-c]thiazol-7-yl]benzamide
Mass analysis, m/z: 379 ($M^+$)
NMR spectrum ($CDCl_3$)
δ: 1.27 (6H, d), 4.20 (1H, d), 4.39 (1H, d), 4.5-4.9 (1H, m), 6.29 (1H, d), 6.47 (1H, d), 6.61 (1H, S), 6.9-7.7 (6H, m), 8.3-8.6 (2H, m), 9.9 (1H, S)

## Example 123

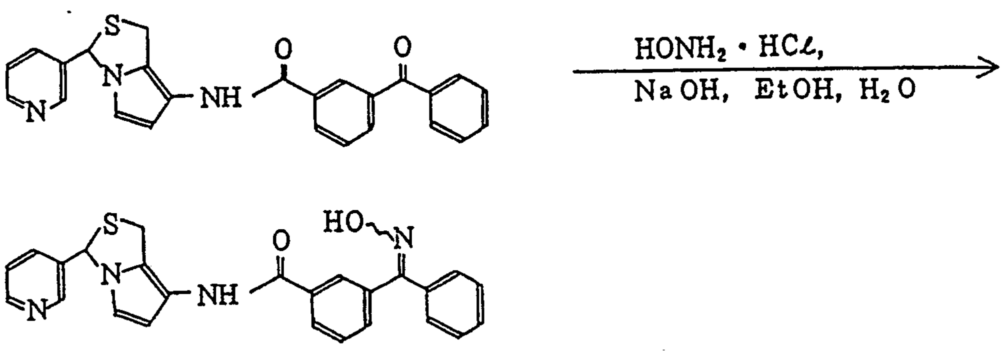


Finely divided sodium hydroxide (180 mg) was added to a mixture of m-benzoyl-N-[3-(3-pyridyl)-1H,3H-pyrrolo[1,2-c]thiazol-7-yl]benzamide (383 mg), hydroxylamine hydrochloride (98 mg), ethanol (4 ml) and water (1 ml) at room temperature. The reaction mixture was heated under reflux with stirring for 5 minute, then cooled and neutralized with 1 N hydrochloric acid. Methylene chloride was added and the resultant crystals were collected by filtration, washed in sequence with water and diethyl ether and recrystallized from 2-propanol to give m-(hydroxyiminophenylmethyl)-N-[3-(3-pyridyl)-1H,3H-pyrrolo[1,2-c]thiazol-7-yl]-benzamide 0.3 hydrate (85 mg).
Melting point: 144 °C

| Elemental analysis (for $C_{25}H_{20}N_4O_2S \cdot 3H_2O$) | | | | |
|---|---|---|---|---|
| | C (%) | H (%) | N (%) | S (%) |
| Calcd.: | 67.34 | 4.66 | 12.56 | 7.19 |
| Found : | 67.31 | 4.68 | 12.17 | 6.99 |

## Example 124

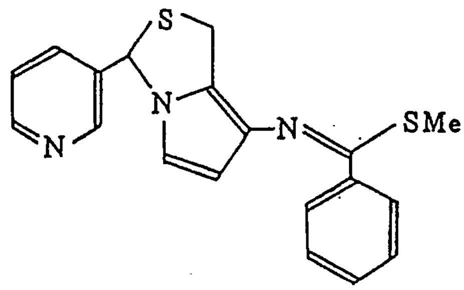

A mixture of N-[3-(3-pyridyl)-1H,3H-pyrrolo[1,2-c]thiazol-7-yl]thiobenzamide (100 mg, 0.30 m mol), potassium carbonate (49 mg) and ethanol (50 ml) was stirred at 50 °C for 3 hours. After cooling to 0 °C, methyl iodide (21 µl) was added dropwise to the mixture at that temperature and the resultant mixture was stirred at room temperature for 3 hours. The solvent was distilled off under reduced pressure, water was added to the residue, the mixture was extracted with methylene chloride, and the extract was dried over anhydrous sodium sulfate. The solvent was distilled off and the residue obtained was purified by silica gel column chromatography to give 7-[N-(methylthiobenzylidene)amino]-3-(3-pyridyl)-1H,3H-pyrrolo[1,2-c]-thiazole (55 mg, 0.16 m mol, 52%).

Mass analysis, m/z: 351 ($M^+$)

NMR spectrum ($CDCl_3$, internal standard: TMS)

δ: 2.15, 2.50 (each s, together 3H), 3.94, 4.29 (d and g, respectively, together 2H), 5.15, 6.00 (each d, together 1H), 6.21, 6.37 (each s, together 1H), 6.32, 6.55 (each d, together 1H), 7.20-7.65 (m, 7H), 8.41-8.58 (m, 2H)

While the invention has been described in detail and with reference to specific embodiments thereof, it will be apparent to one skilled in the art that various changes and modifications can be made therein without departing from the spirit and scope thereof.

## Claims

1. A heterocyclic compound of the formula (I) , or a pharmacologically acceptable salt thereof:

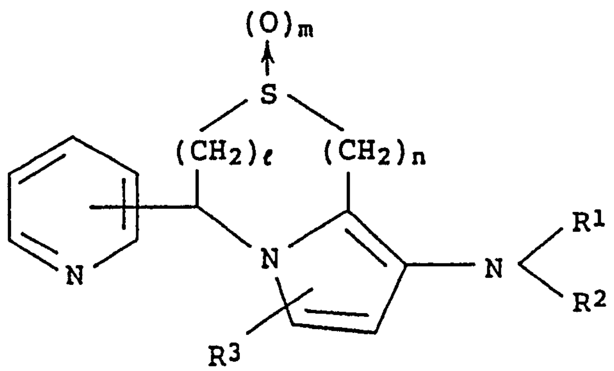

(I)

wherein

ℓ represents 0 or 1;

m represents 0, 1 or 2;

n represents 1 or 2, provided that the sum of ℓ and n is 1 or 2;

$R^1$ represents a hydrogen atom, a $C_1$-$C_6$ alkyl group, a group of the formula

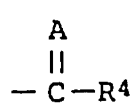

in which A is an oxygen atom or a sulfur atom, or a group of the formula $-SO_2R^4$;

$R^2$ represents a hydrogen atom or a $C_1$-$C_6$ alkyl group;
$R^3$ represents a hydrogen atom or a $C_1$-$C_6$ alkyl group;
$R^4$ represents a substituted or unsubstituted aryl group which may have one or more substituents selected from a group of the formula -O-$R^7$, a nitro group, a halogen atom, a cyano group, a trihalogeno-$C_1$-$C_6$ alkyl group, a group of the formula

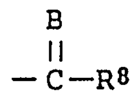

in which B is O or N-OH, an amino group of the formula

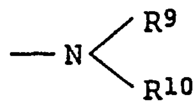 ,

a group of the formula

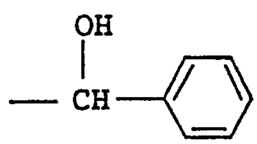 ,

a $C_1$-$C_6$ alkyl group and an aralkyl group;
a $C_1$-$C_6$ alkyl group, an aralkyl group, a $C_1$-$C_6$ alkoxy group, an aryloxy group, an aralkyloxy group, a group of the formula

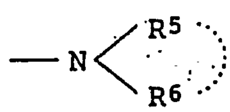 ,

a cycloalkyl group or a group of the formula

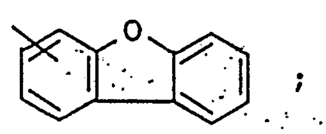 ;

$R^5$ and $R^6$, which may be the same or different, each represents a hydrogen atom, a $C_1$-$C_6$ alkyl group, an aralkyl group or an aryl group, or $R^5$ and $R^6$, together with the adjacent nitrogen atom, combinedly form a piperidine ring, a morpholine ring, or a piperazine ring which may optionally be substituted in position 4 of the piperazine ring by a $C_1$-$C_6$ alkyl group or an aralkyl group;
$R^7$ represents a hydrogen atom, a $C_1$-$C_6$ alkyl group, an aralkyl group, an aryl group which may have one or more substituents selected from a $C_1$-$C_6$ alkyl group and a halogen atom; or an acyl group;
$R^8$ represents an aralkyl group, an aryl group which may have one or more substituents selected from a $C_1$-$C_6$ alkoxy group, a $C_1$-$C_6$ alkyl group and a halogen atom; a group of the formula

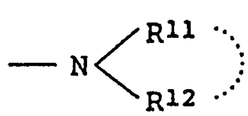 ,

a hydroxyl group or a $C_1$-$C_6$ alkoxy group;
$R^9$ and $R^{10}$ may be the same or different, and one of them is a hydrogen atom or a $C_1$-$C_6$ alkyl group and the other is a hydrogen atom, a $C_1$-$C_6$ alkyl group, a carboxyl group, a $C_1$-$C_6$ alkoxycarbonyl group, an

arylcarbonyl group, an aralkylcarbonyl group, a group of the formula

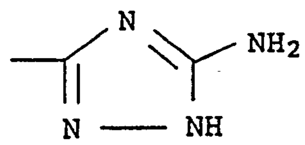

or a group of the formula

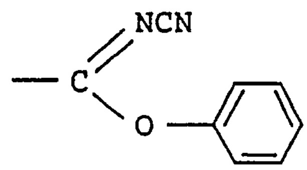

;

and

$R^{11}$ and $R^{12}$, which may be the same or different, each represents a hydrogen atom, a $C_1$-$C_6$ alkyl group, an aralkyl group, an aryl group or a cycloalkyl group, or $R^{11}$ and $R^{12}$, together with the adjacent nitrogen atom, combinedly form a pyrrolidine ring, a piperidine ring, a morpholine ring, or a piperazine ring which may optionally be substituted in position 4 of the piperazine ring by a $C_1$-$C_6$ alkyl group or aralkyl group.

2. A heterocyclic compound of the formula:

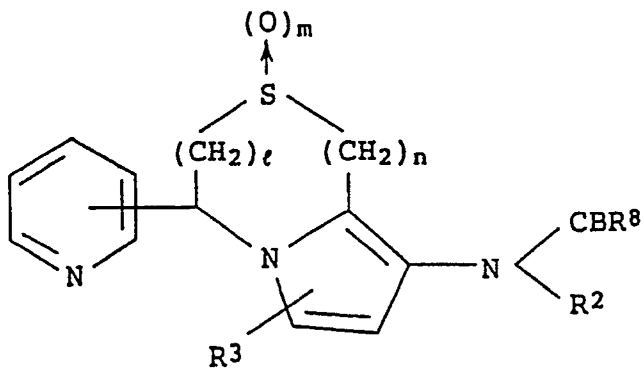

wherein ℓ, m, n, $R^2$, $R^3$, B and $R^8$ are as defined in Claim 1, or a pharmacologically acceptable salt thereof.

3. A heterocyclic compound of the formula:

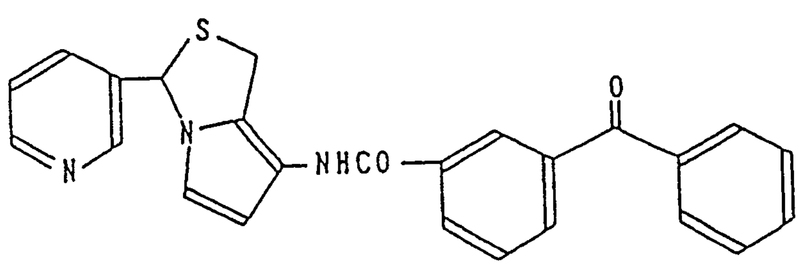

or a pharmacologically acceptable salt thereof.

4. A heterocyclic compound of the formula:

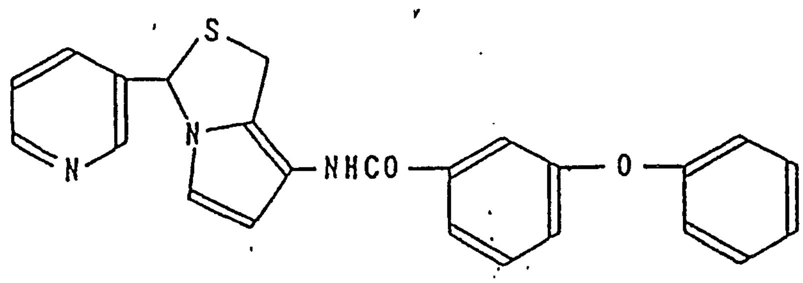

or a pharmacologically acceptable salt thereof.

5. A heterocyclic compound of the formula:

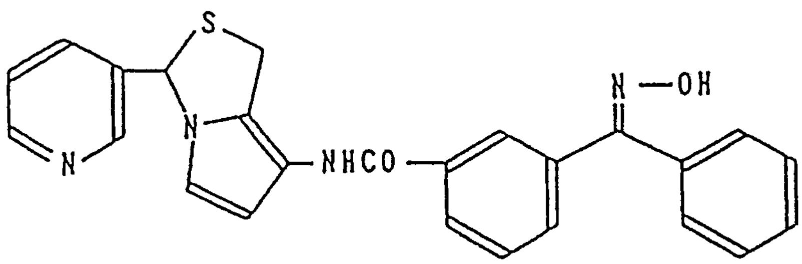

or a pharmacologically acceptable salt thereof.

6. A pharmaceutical composition containing an effective amount of a heterocyclic compound of Claim 1 or a pharmacologically acceptable salt thereof and a pharmaceutically acceptable carrier.

7. A pharmaceutical composition containing a heterocyclic compound of Claim 3, 4 or 5 or a pharmacologically acceptable salt thereof and a pharmaceutically acceptable carrier.

8. A process for producing heterocyclic compound of the formula (I):

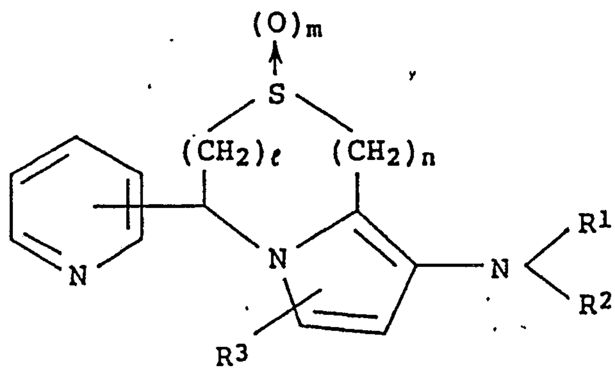

(I)

wherein

$\ell$ represents 0 or 1;

m represents 0, 1 or 2;

n represents 1 or 2, provided that the sum of $\ell$ and n is 1 or 2;

$R^1$ represents a hydrogen atom, a $C_1$-$C_6$ alkyl group, a group of the formula

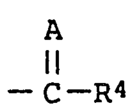

in which A is an oxygen atom or a sulfur atom, or a group of the formula $-SO_2R^4$;

$R^2$ represents a hydrogen atom or a $C_1$-$C_6$ alkyl group;

$R^3$ represents a hydrogen atom or a $C_1$-$C_6$ alkyl group;

$R^4$ represents a substituted or unsubstituted aryl group which may have one or more substituents selected

from a group of the formula -O-$R^7$, a nitro group, a halogen atom, a cyano group, a trihalogeno-$C_1$-$C_6$ alkyl group, a group of the formula

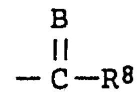

-$R^8$ in which B is O or N-OH, an amino group of the formula

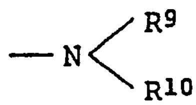 ,

a group of the formula

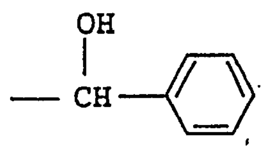 ,

a $C_1$-$C_6$ alkyl group and an aralkyl group;

a $C_1$-$C_6$ alkyl group, an aralkyl group, a $C_1$-$C_6$ alkoxy group, an aryloxy group, an aralkyloxy group, a group of the formula

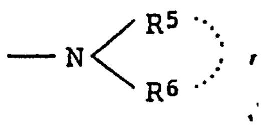 ,

a cycloalkyl group or a group of the formula

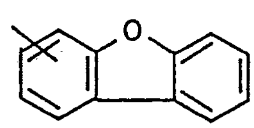 ;

$R^5$ and $R^6$, which may be the same or different, each represents a hydrogen atom, a $C_1$-$C_6$ alkyl group, an aralkyl group or an aryl group, or $R^5$ and $R^6$, together with the adjacent nitrogen atom, combinedly form a piperidine ring, a morpholine ring, or a piperazine ring which may optionally be substituted in position 4 of the piperazine group by a $C_1$-$C_6$ alkyl group or an aralkyl group;

$R^7$ represents a hydrogen atom, a $C_1$-$C_6$ alkyl group, an aralkyl group, an aryl group which may have one or more substituents selected from a $C_1$-$C_6$ alkyl group and a halogen atom; or an acyl group;

$R^8$ represents an aralkyl group, an aryl group which may have one or more substituents selected from a $C_1$-$C_6$ alkoxy group, a $C_1$-$C_6$ alkyl group and a halogen atom; a group of the formula

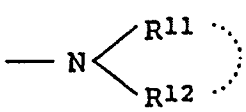 ,

a hydroxyl group or a $C_1$-$C_6$ alkoxy group;

$R^9$ and $R^{10}$ may be the same or different, and one of them is a hydrogen atom or a $C_1$-$C_6$ alkyl group and the other is a hydrogen atom, a $C_1$-$C_6$ alkyl group, a carboxyl group, a $C_1$-$C_6$ alkoxycarbonyl group, an arylcarbonyl group, an aralkylcarbonyl group, a group of the formula

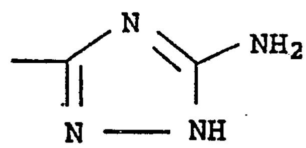

or a group of the formula

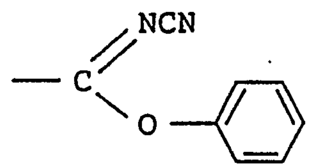

;

and

$R^{11}$ and $R^{12}$, which may be the same or different, each represents a hydrogen atom, a $C_1$-$C_6$ alkyl group, an aralkyl group, an aryl group or a cycloalkyl group, or $R^{11}$ and $R^{12}$, together with the adjacent nitrogen atom, combinedly form a pyrrolidine ring, a piperidine ring, a morpholine ring, or a piperazine ring which may optionally be substituted in position 4 of the piperazine ring by a $C_1$-$C_6$ alkyl group or aralkyl group: and salts thereof which comprises any of the following.

(1) the rearrangement reaction for a amine synthesis represented by the following reaction equation:

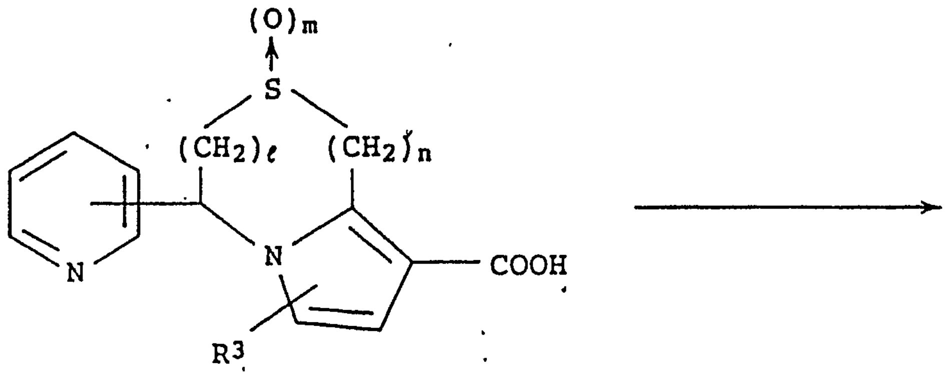

(II) or a derivative thereof

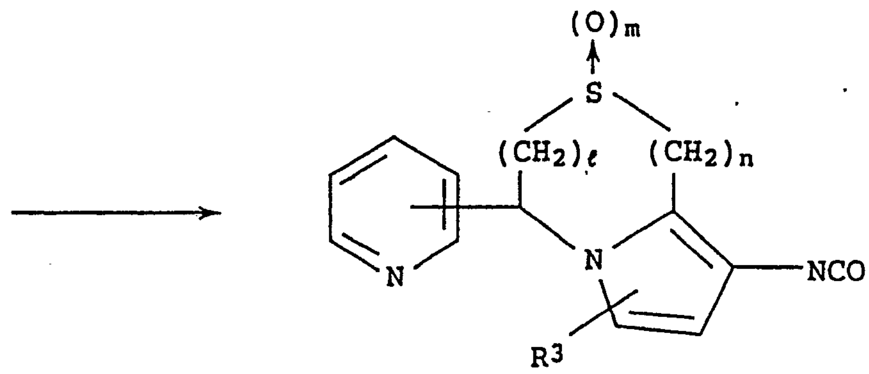

(III)

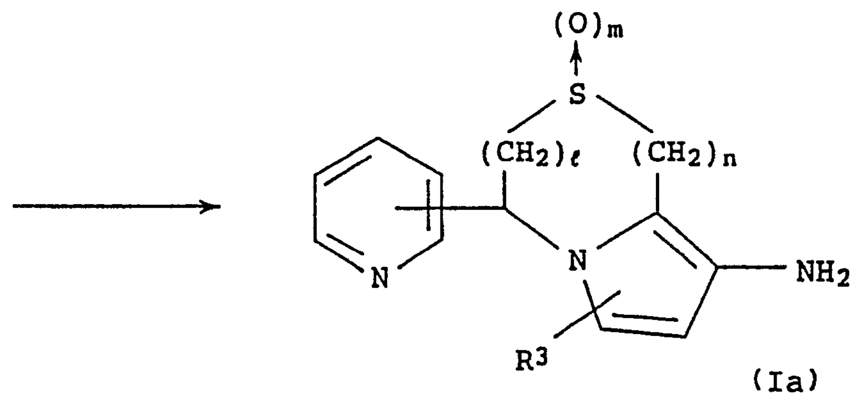

(Ia)

wherein $R^3$, ℓ, m and n are as defined above;
(2) the reaction represented by the following reaction equation:

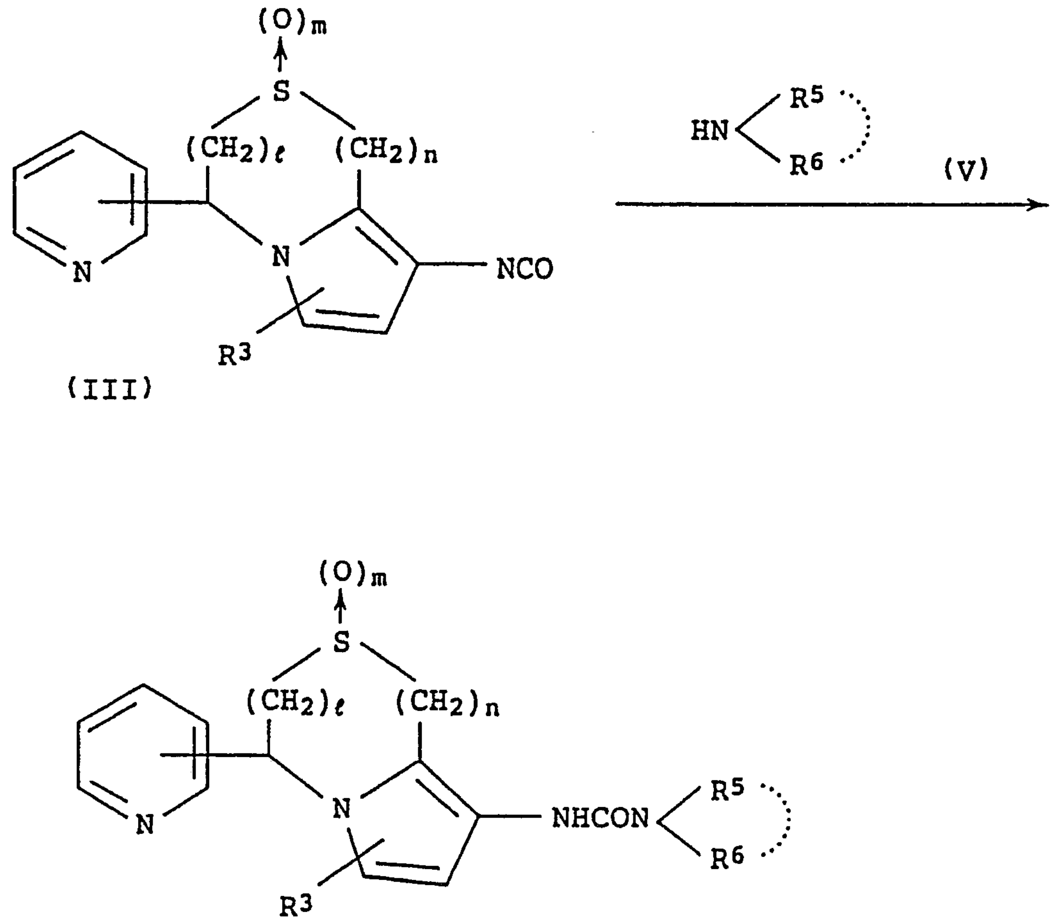

(III) (V) (Ic)

wherein $R^3$, $R^5$, $R^6$, $\ell$, m and n are as defined above;
(3) the reaction represented by the following reaction equation:

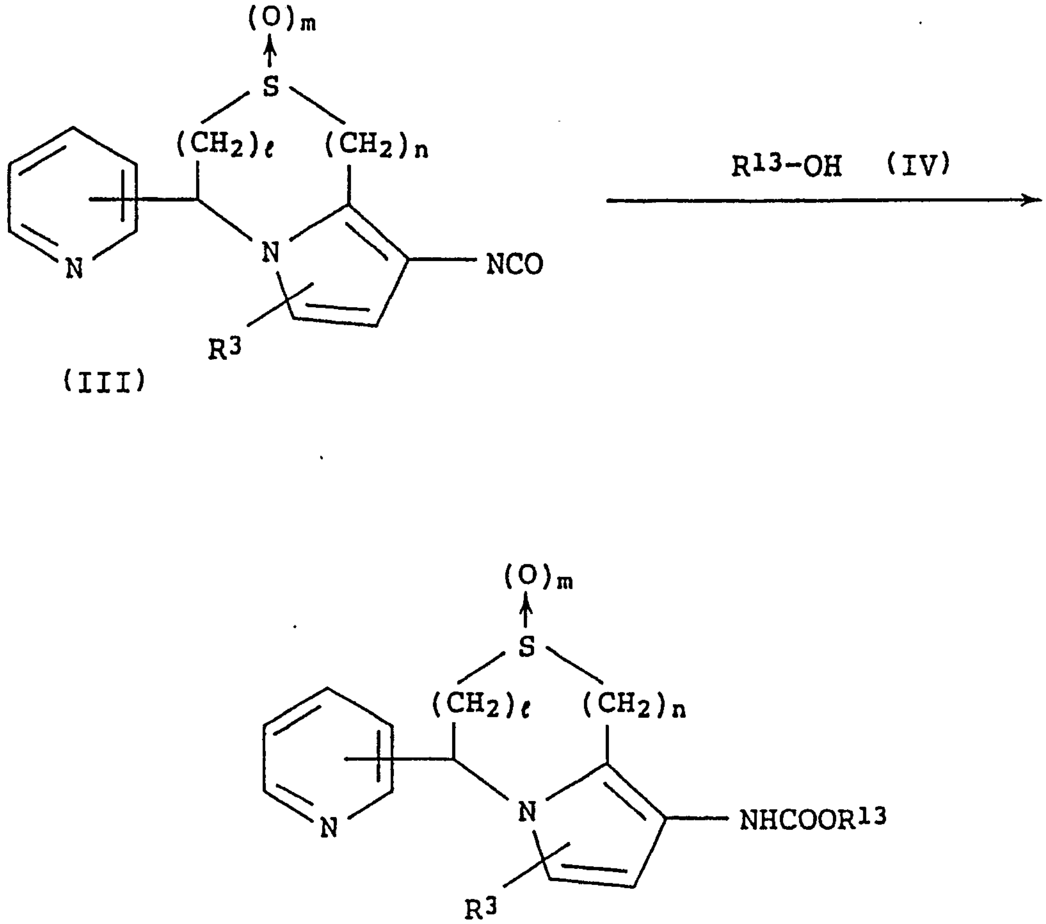


(III)

(Id)

wherein $R^3$, $\ell$, m and n are as defined above and $R^{13}$ represents a $C_1$-$C_6$ alkyl, aralkyl or aryl group;
(4) the reaction represented by the following reaction equation:

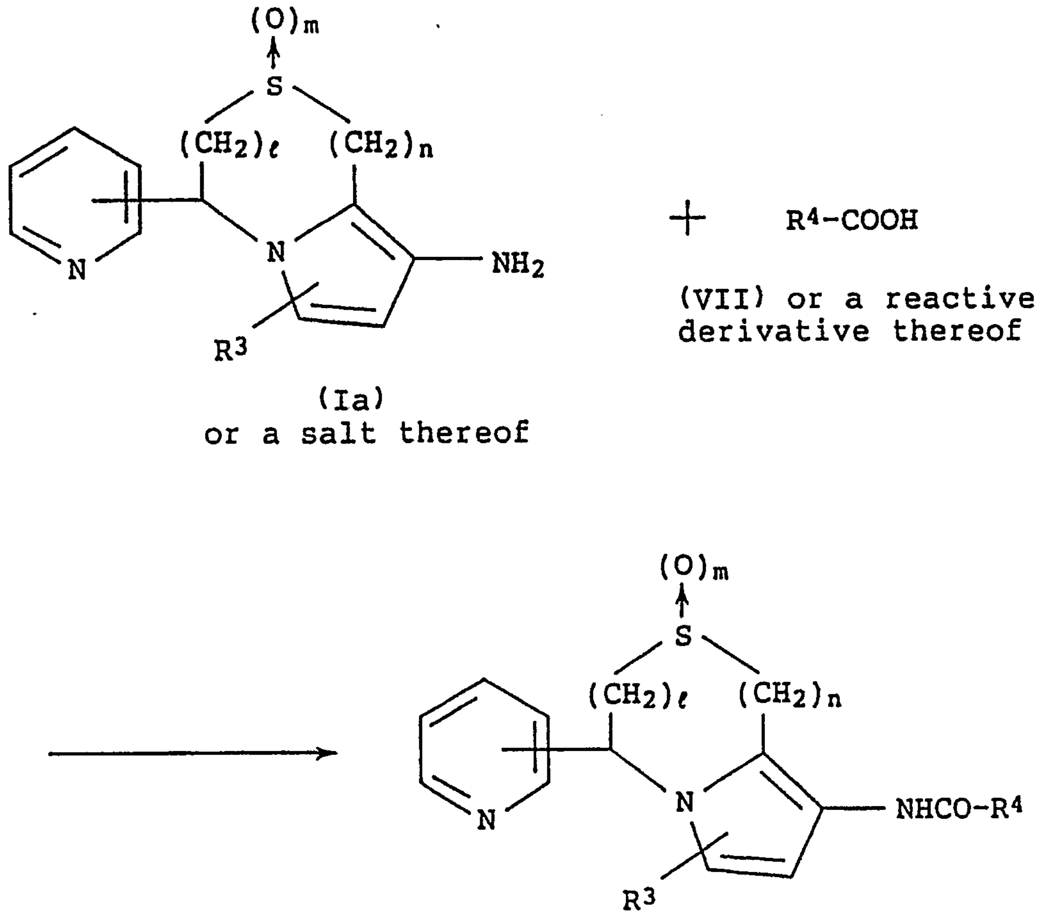

(Ia)
or a salt thereof

(VII) or a reactive derivative thereof

(Ie)

wherein $R^3$, $R^4$, ℓ, m and n are as defined above;
(5) the reaction represented by the following reaction equation:

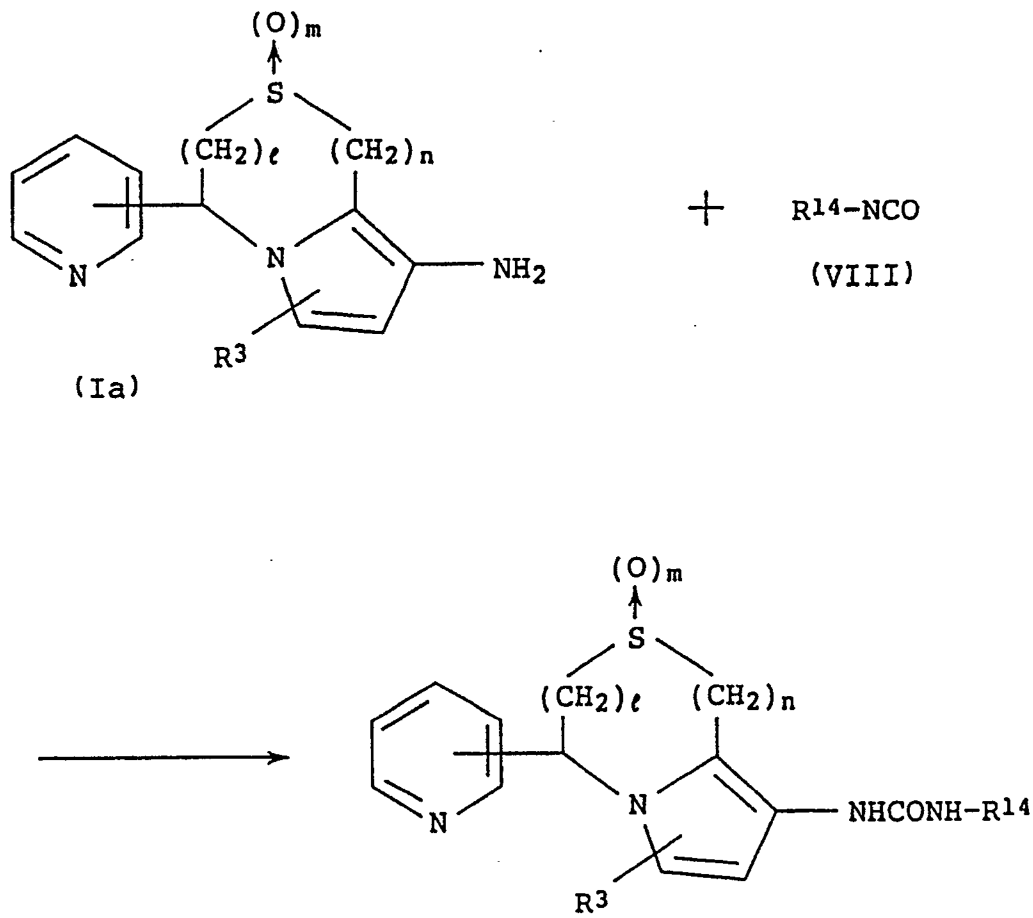

(Ia) (VIII) (If)

wherein $R^3$, $\ell$, m and n are as defined above and $R^{14}$ represents a $C_1$-$C_6$ alkyl, aryl, or aralkyl group;

(6) the reaction represented by the following reaction equation:

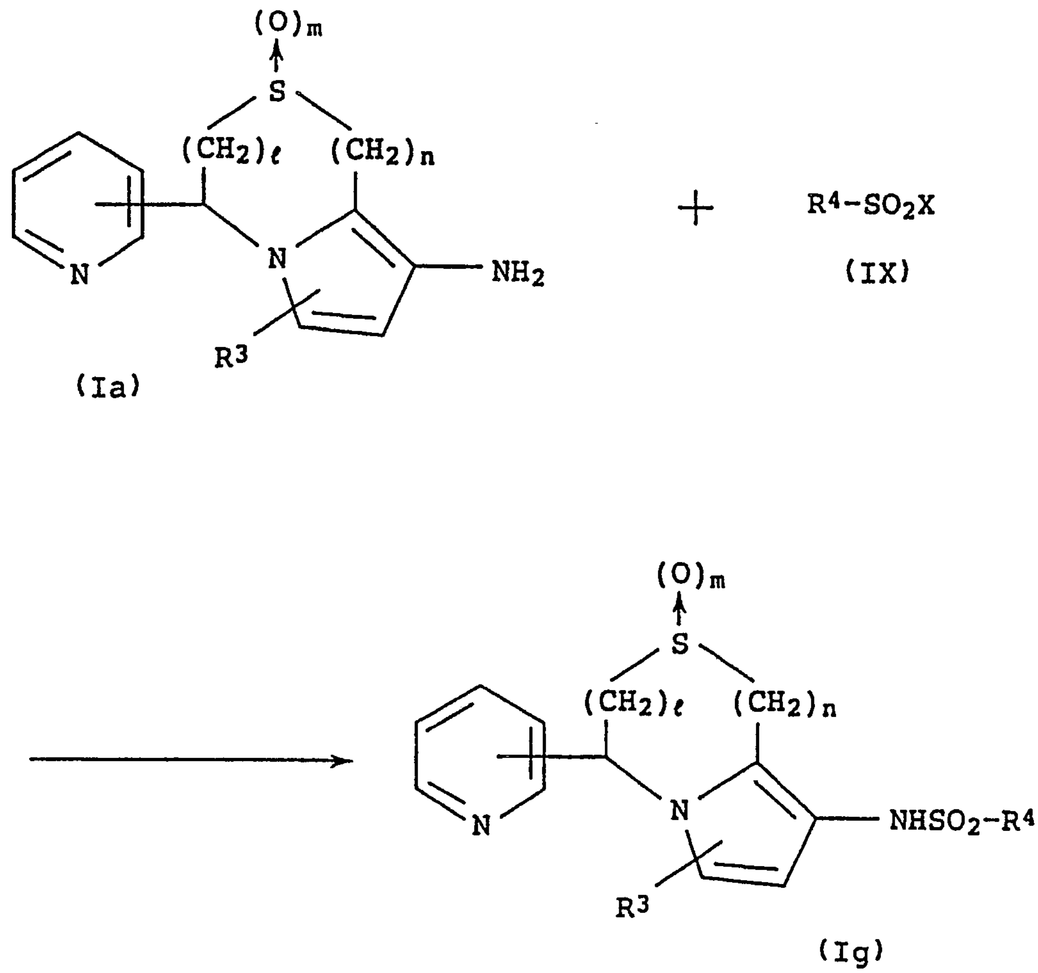

(Ia) (IX) (Ig)

wherein $R^3$, $R^4$, $\ell$, m and n, are as defined above and X represents a halogen atom;

(7) the reaction represented by the following reaction equation:

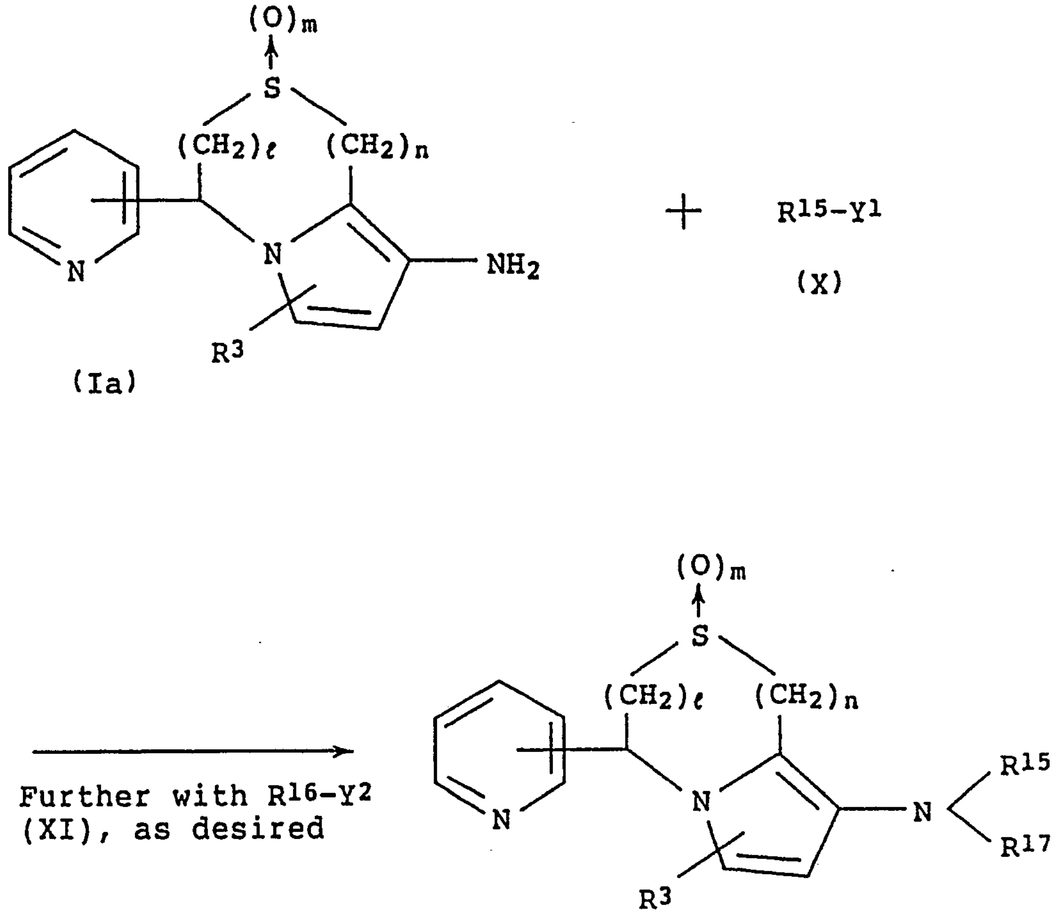


(Ia)

(X)

(Ih)

wherein $R^3$, ℓ, m and n are as defined above, $R^{15}$ represents a $C_1$-$C_6$ alkyl group, $R^{16}$ represents a $C_1$-$C_6$ alkyl group differing from $R^{15}$, $R^{17}$ represents a hydrogen atom, the same group as $R^{15}$ or the same group as $R^{16}$, and $Y^1$ and $Y^2$ are the same or different and each represents a halogen atom or an organic sulfonic acid residue;

(8) the alkylation reaction represented by the following reaction equation:

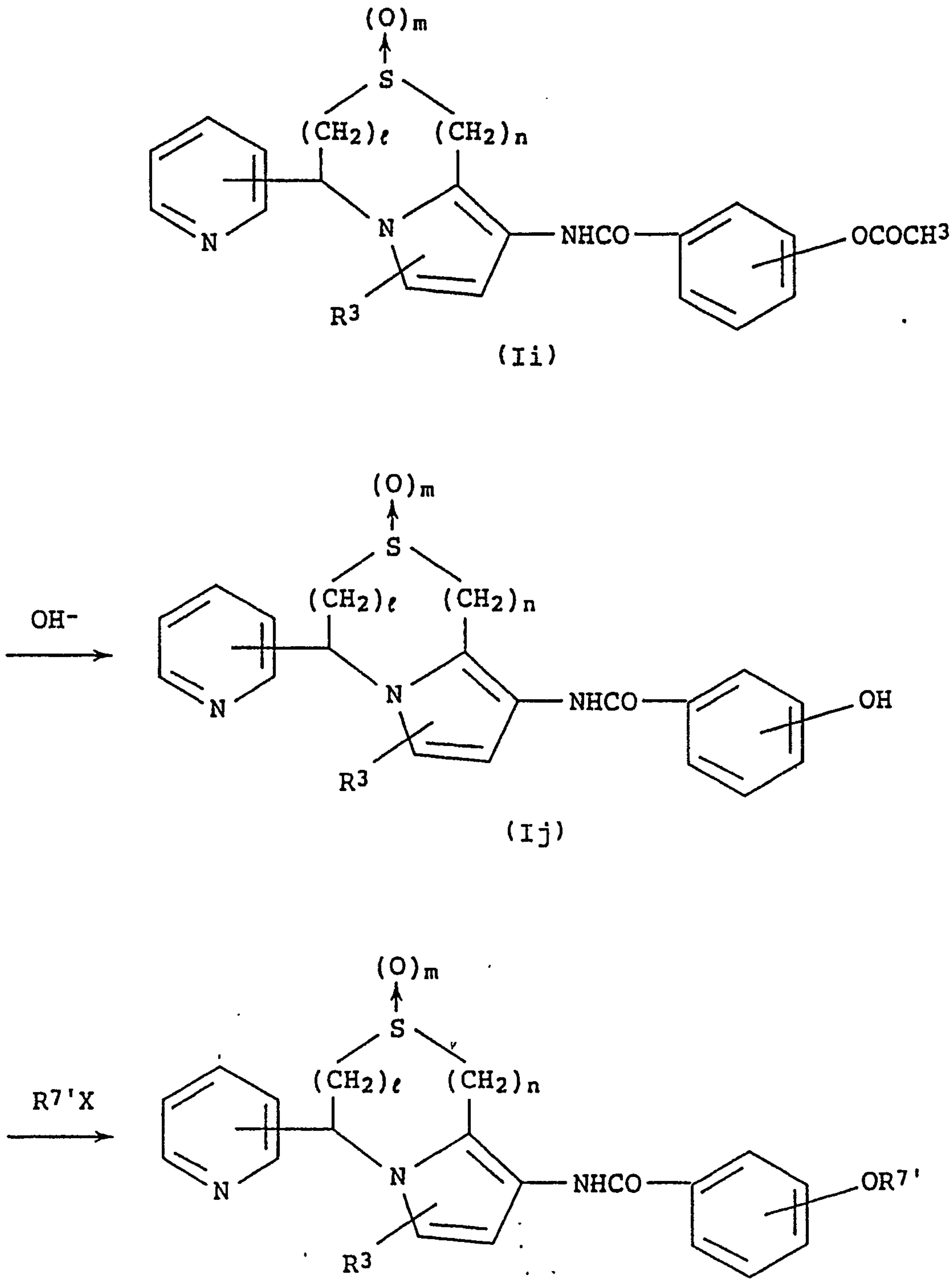


(Ii)

(Ij)

(Ik)

wherein $R^3$, ℓ, X, m and n are as defined above, and $R^{7'}$ represents a $C_1$-$C_6$ alkyl group, aralkyl group, an acyl group or an aryl group which may optionally have one or more substituents each independently selected from the group consisting of a $C_1$-$C_6$ alkyl group and a halogen atom;

(9) the coupling reaction represented by the following reaction equation:

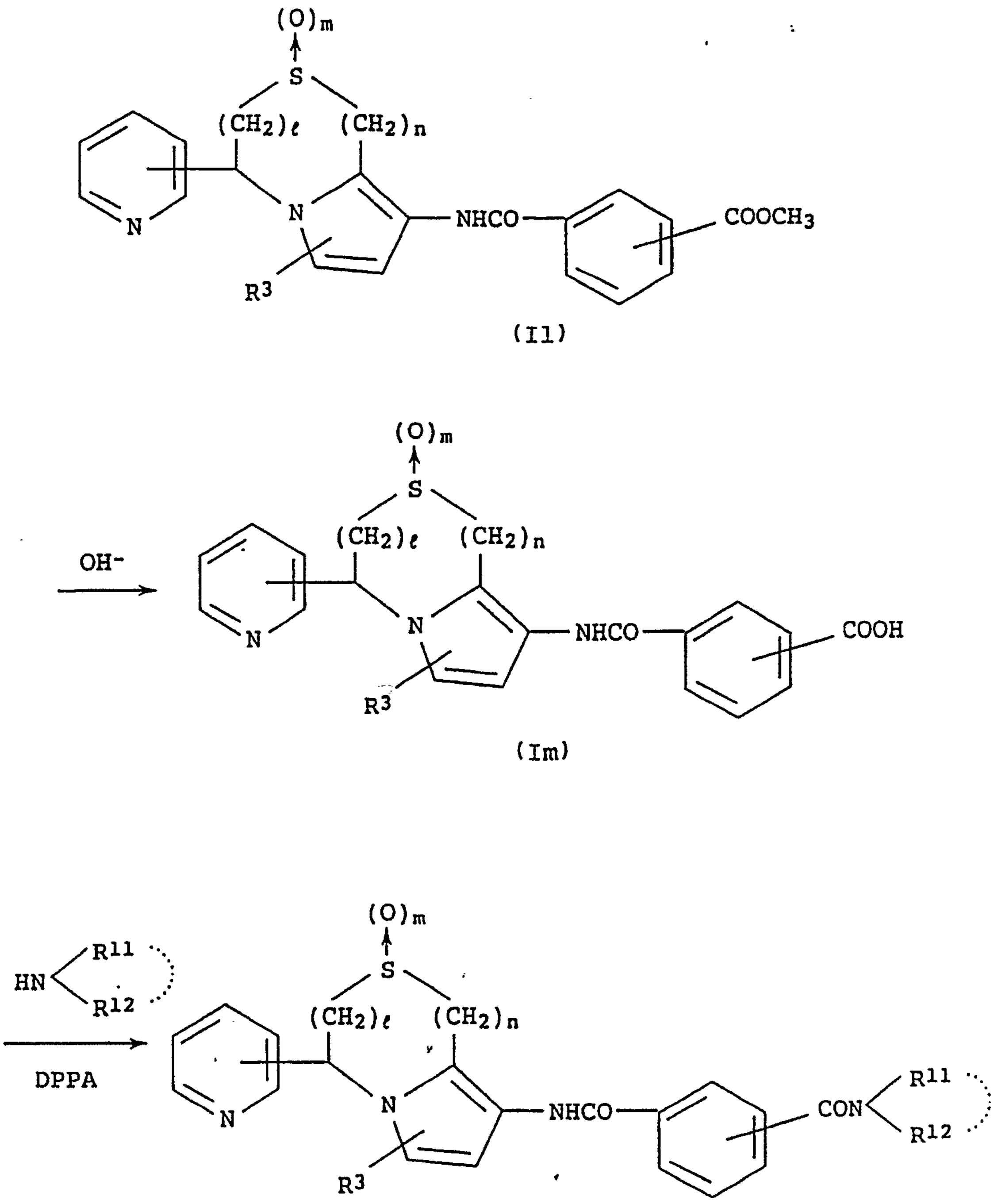


(Il)

(Im)

(In)

wherein $R^3$, $R^{11}$, $R^{12}$, ℓ, m, n are as defined above;

(10) the alkylation reaction represented by the following reaction equation:

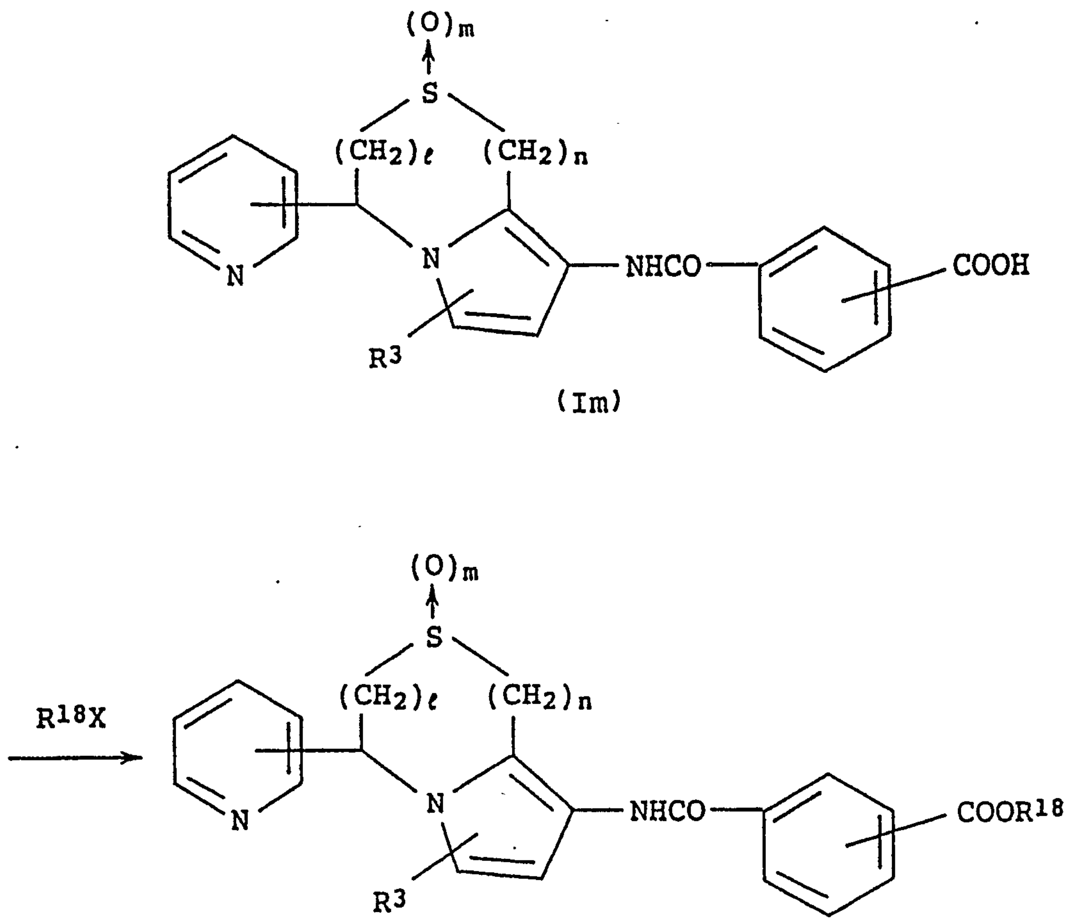


(Im)

(Io)

wherein $R^3$, ℓ, m, n and X are as defined in above and $R^{18}$ represents a $C_1$-$C_6$ alkyl group;
(11) the reaction represented by the following reaction equation:

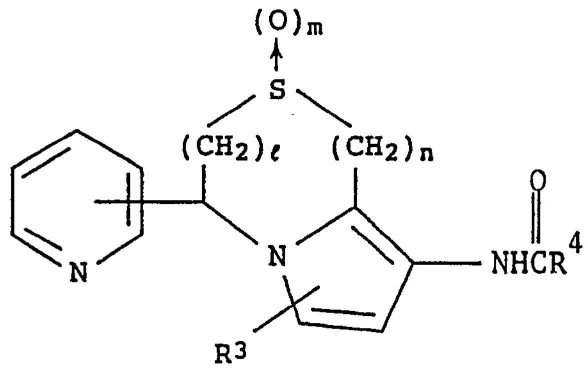

(Ip)

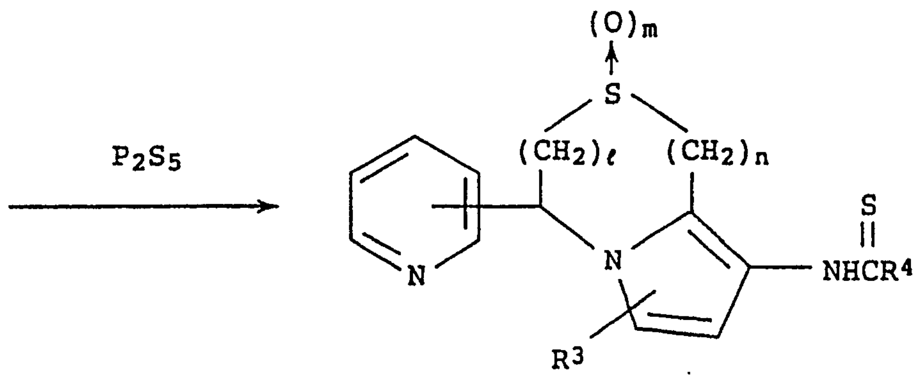


(Iq)

wherein $R^3$, $R^4$, ℓ, m and n are as defined above; and

(12) the reaction represented by the following reaction equation:

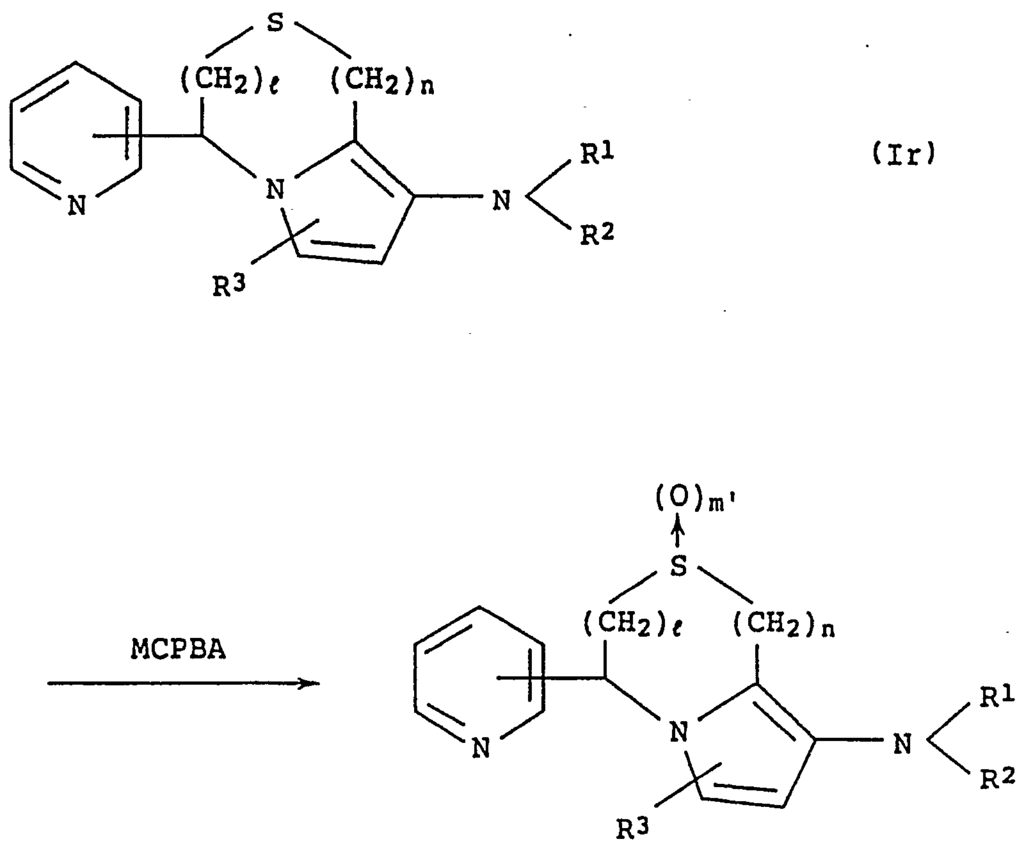


(Ir)

(Is)

wherein $R^1$, $R^2$, $R^3$, ℓ and n are as defined above and m′ represents 1 or 2.

European Patent Office

# EUROPEAN SEARCH REPORT

Application Number

**EP 90 31 1144**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 118 321 (RHONE-POULENC)<br>* Claim 1; page 16, lines 12-19 *<br>- - - | 1,6 | C 07<br>D 513/04<br>A 61 K 31/425<br>A 61 K 31/54 //<br>(C 07 D 513/04<br>C 07 D 277:00<br>C 07 D 209:00 )<br>(C 07 D 513/04<br>C 07<br>D 279:00<br>C 07 D 209:00 ) |
| D,A | EP-A-0 252 823 (RHONE-POULENC)<br>* Claim 1; page 7, lines 10-25 *<br>- - - | 1,6 | |
| D,A | EP-A-0 253 711 (RHONE-POULENC)<br>* Claim 1; page 7, line 25 - page 8, line 5 *<br>- - - - - - | 1,6 | |
| | | | **TECHNICAL FIELDS SEARCHED (Int. Cl.5)** |
| | | | C 07 D 513/00<br>A 61 K 31/00 |

The present search report has been drawn up for all claims

| Place of search | Date of completion of search | Examiner |
|---|---|---|
| The Hague | 14 January 91 | VOYIAZOGLOU D. |

**CATEGORY OF CITED DOCUMENTS**

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document